# EUROPEAN PATENT SPECIFICATION

(11) **EP 3 113 800 B1**
(45) Date of publication and mention of the grant of the patent: **19.06.2024**
(21) Application number: 15758620.7
(22) Date of filing: 02.03.2015
(51) Int. Cl.: A61K 45/00, C07H 21/04, C12N 15/00

(54) **ENHANCED PRODUCTION OF RECOMBINANT CRM197 IN E. COLI**
VERBESSERTE HERSTELLUNG REKOMBINANTER CRM197 IN E. COLI
PRODUCTION ACCRUE DE CRM197 RECOMBINANTE CHEZ E. COLI

(30) Priority: 03.03.2014 US 201461947234 P
(43) Date of publication of application: 11.01.2017
(73) Proprietor: Scarab Genomics, LLC, Madison, WI 53713 (US)
(72) Inventor: BLATTNER, Cristopher, R., Madison, Wisconsin 53711 (US); FRISCH, David, A., Fitchburg, WI 53711 (US); NOVY, Robert, E., Verona, WI 53593 (US); HENKER, Terrance, M., Madison, WI 53711 (US); STEFFEN, Eric, A., Mount Horeb, WI 53572 (US); BLATTNER, Frederick, R., Madison, Wisconsin 53715 (US); CHOI, Hyunsic, Madison, WI 53719 (US); POSFAI, Gyorgy, H-6727 Szeged (HU); LANDRY, Charles, F., Fitchburg, WI 53711 (US)
(74) Representative: Mathys & Squire
(86) International application number: PCT/US2015/018338
(87) International publication number: WO 2015/134402

(56) References cited:
- WO-A1-2013/059595
- WO-A2-2007/024756
- WO-A2-2011/042516
- US-A1- 2005 260 720
- US-A1- 2009 104 667
- US-A1- 2012 128 727
- US-A1- 2012 289 688
- US-A1- 2013 011 874
- BLATTNER F R ET AL: "THE COMPLETE GENOME SEQUENCE OF ESCHERICHIA COLI K-12", SCIENCE, AMERICAN ASSOCIATION FOR THE ADVANCEMENT OF SCIENCE, vol. 277, 5 September 1997 (1997-09-05), pages 1453-1462, XP002069950, ISSN: 0036-8075, DOI: 10.1126/SCIENCE.277.5331.1453
- HORLER ET AL.: 'Furanose-specific Sugar Transport: Characterization of a Bacterial Galactofuranose -binding Protein.' THE JOUMAL OF BIOLOGICAL CHEMISTRY vol. 284, no. 45, 06 November 2009, ISSN 0021-9258 pages 31156 - 31163, XP055222496
- POSFAI ET AL.: 'Reduced Genome Escherichia coli: A platform for genomic and metabolic engineering.' METABOLOMICS AND ENVIRONMENTAL BIOTECHNOLOGY EC - US TASK FORCE ON BIOTECHNOLOGY RESEARCH June 2008, page 44, XP055358043

## Description

### FIELD OF THE INVENTION

The present invention is directed to the production of recombinant CRM197 in *E. coli,* preferably in reduced genome *E*. *coli* K12 strains.

### BACKGROUND OF THE INVENTION

Diphtheria toxin (DTx) is a two-component exotoxin of *Corynebacterium diphtheriae* synthesized as a single polypeptide chain of 535 amino acids containing an A (active) domain and a B (binding) domain linked together by a disulfide bridge. The toxin binds to a cell receptor (HB-EGF receptor) and enters the cell by endocytosis where the A domain is released from the B domain by proteolytic cleavage. The A domain then exits the endosome through pores made by the B domain and enters the cytoplasm where it inhibits protein synthesis ultimately resulting in cell death.

CRM197 is a mutated form of Dtx containing a single amino acid substitution of glutamic acid for glycine (G52E) that renders the protein enzymatically inactive and non-toxic. CRM197 has been found to be an ideal carrier for conjugate vaccines against encapsulated bacteria. Conjugate vaccines comprise CRM197 covalently linked to poorly immunogenic and T-cell independent capsular polysaccharides, thus creating conjugate antigens that are highly immunogenic and result in long-lasting immunity against the antigen(s).

Vaccines containing CRM197 as a carrier protein have been successfully used to immunize millions of children and include Menveo^{®}, a tetravalent conjugate vaccine against serogroups A-C-W135-Y of *Neisseria meningitidis*, Menjugate^{®} and Meningitec^{®} (against serotype C of *N. meningitidis*), Vaxem-Hib^{®} and HibTITER^{®} (against *Haemophilus influenzae* type B, Hib), and the multivalent pneumococcal conjugate Prevnar^{™}.

In contrast to tetanus and diphtheria toxins, CRM197 does not require chemical detoxification and can therefore be purified to homogeneity and used directly for conjugation. CRM197 is currently manufactured by the fermentation of either *Corynebacterium diphtheriae C7,* where it is expressed from multiple lysogens of the β phage, or from a plasmid system in *Pseudomonas flurorescens.* The yield of CRM197 (which is released into the media during C. *diphtheriae* fermentation) is low ranging from tens of mg/L to -200 mg/L and requires biosafety level 2 facilities, resulting in a retail price of about $500 US per milligram of CRM197. A single dose of vaccine typically contains about 10 and 60 µg of CRM197 and over 150 million doses are used each year. Current demand for conjugate CRM197 vaccines has outpaced supply and has resulted in delays in initiating vaccination programs in developing countries placing the health of millions of children at risk.

Moreover, a possible therapeutic use for CRM197 in treating cancers such as ovarian cancer has recently been reported, based on CRM197's ability to bind the soluble form of heparin-binding epidermal growth factor (pro-HB-EGF), which is highly expressed in some cancers. The research and development of this therapeutic potential places even more of a strain on current production methods.

The single greatest factor contributing to the high price and short supply of CRM197 is the historical inability to generate high amounts of CRM197 in the production workhorse *E*. *coli.* Although an insoluble form of CRM197 can be fermented in *E. coli* to relatively moderate yields, only a fraction of the insoluble product can be converted to the soluble form (Stefan et al., 2011). Producing high amounts of soluble CRM197 in *E. coli* has been even more challenging. A method for reliably and inexpensively producing high amounts of CRM197 for therapeutic use would constitute a significant advance in the art.

WO 2011/042516 describes production of the the bacterial toxin CRM 197 in *E. coli.* WO 2013/059595 describes reduced genome bacteria with improved genetic stability.

### SUMMARY OF THE INVENTION

The present invention is defined by the claims.

The present invention relates to a method for producing a recombinant CRM197 protein in a reduced genome *E. coli* host comprising incubating a reduced genome *E. coli* comprising an expression vector comprising a nucleotide sequence encoding a CRM197 protein fused to an ompA, ompF or ytfQ signal sequence that directs transfer of the CRM197 protein to the periplasm, wherein said nucleotide sequence is operably linked to an expression control sequence, under conditions suitable for the expression of the recombinant CRM197 protein, wherein the native parent *E*. *coli* strain is a K-12 strain comprising a native -2 frameshift mutation wherein the native -2 frameshift mutation comprises a deletion of nucleotides GT at positions 983 and 984 of *ilvG* relative to the intact *ilvG,* preferably K12 MG1655, and wherein the reduced genome *E. coli* host comprises the following modifications: (i) deletion of at least the following DNA segments: b0245-b0301, b0303-b0310, b1336-b1411, b4426-b4427, b2441-b2450, b2622-b2654, b2657-b2660, b4462, b1994-b2008, b4435, b3322-b3338, b2349-b2363, b1539-b1579, b4269-b4320, b2968-b2972, b2975-b2977, b2979-b2987, b4466-4468, b1137-b1172, b0537-b0565, b0016-b0022, b4412-b4413, b0577-b0582, b4415, b2389-b2390, b2392-b2395, b0358-b0368, b0370-b0380, b2856-b2863, b3042-b3048, b0656, b1325-b1333, b2030-b2062, b2190-b2192, b3215-b3219, b3504-b3505, b1070-b1083, b1878-b1894, b1917-b1950, b4324-b4342, b4345-b4358, b4486, b0497-b0502, b0700-b0706, b1456-b1462, b3481-b3484, b3592-b3596, b0981-b0988, b1021-b1029, b2080-b2096, b4438, b3440-b3445, b4451, b3556-b3558, b4455, b1786, b0150-b0153 and b2945 of the *E. coli* K-12 strain MG1655 (ii) deletion of the *rph* gene to enhance orotate phosphoribosyltransferase activity, (iii) correction of the native -2 frameshift mutation in the *ilvG* gene in order to restore the active acetohydroxy acid synthase II production, preferably wherein said correction is an insertion of two nucleotides between positions 982 and 983, and (iv) deletion of the *iclR and ankyrin-like regulator protein* (b4017) genes; whereby a yield of at least 1 gram per liter, preferably at least 2 grams per liter of soluble CRM197 is obtained.

In one aspect, the nucleic acid sequence encoding a CRM197 protein is fused to a nucleic acid sequence encoding a signal sequence as claimed that directs transfer of the CRM197 protein to the periplasm of the *E. coli* host cell as claimed, whereby a yield of about 1 gram per liter to about 10 grams per liter of soluble CRM197 is achieved.

According to this aspect of the invention, the reduced genome *E. coli* host comprises an expression vector comprising a nucleic acid sequence comprising a 5' signal sequence portion encoding a polypeptide having an amino acid sequence capable of directing transport of CRM197 to the *E. coli* periplasm and a 3' CRM197 portion encoding the CRM197 protein lacking its native signal sequence. Preferably the expression of CRM197 is inducible and the method comprises the steps of (a) growing the reduced genome *E. coli* and (b) inducing expression of CRM197. Preferably, the method is carried out in a fermentor.

In related aspects, the reduced genome *E. coli* host cell is transformed with an expression vector comprising an inducible promoter (e.g. a lac derivative promoter) operatively linked to the protein coding sequence and expression of CRM197 is induced by the addition of a suitable amount of inducer (e.g. Isopropyl β-D-1-thiogalactopyranoside (IPTG)). Preferably, under shake flask conditions, induction occurs at an optical density (OD) at 600 nm (at which wavelength 1 OD unit corresponds to about 0.8 x 10**'** cells/ml) of about 0.1 to about 1.5 (more preferably about 0.2 to about 0.9, even more preferably about 0.3 to about 0.6). Under fermentation conditions, induction preferably occurs at an OD600 of about 100 to 400, more preferably about 150 to 300, most preferably between 200 to 275 (e.g. 230 and 250). In other related aspects, the pH of the culture during growth and/or induction is from about 6.5 to about 7.5, the growth and/or induction temperature is from about 20 °C to about 30 °C (preferably about 25 °C) and the growth media is free of serum, yeast extract and animal-derived byproducts. In particularly preferred embodiments, growing the (e.g. reduced genome) *E. coli* comprises a relatively short initial incubation at 37 °C (e.g. 1 to 3 hours) followed by growth at 20 °C to 30 °C (preferably at about 25 °C) prior to and subsequent to induction or comprises continuous growth at 20 °C to 30 °C (preferably at about 25 °C) prior to and subsequent to induction.

In related embodiments of the disclosure, the yield of soluble CRM197 obtained is at least about 0.5 g/L or at least about 0.7 g/L. In related embodiments of the invention, the yield of soluble CRM197 obtained is at least about 1.0 g/L, at least about 1.3 g/L, at least about 1.5 g/L, at least about 1.7 g/L, at least about 2.0 g/L, at least about 2.3 g/L, at least about 2.5 g/L, at least about 2.7 g/L, at least about 3.0 g/L, at least about 3.5 g/L, at least about 3.7 g/L, at least about 4.0 g/L, at least about 4.5 g/L, at least about 5 g/L, at least about 5.5 g/L, at least about 6.0 g/L, at least about 7.0 g/L, at least about 8.0 g/L, at least about 9.0 g/L or at least about 10.0 g/L. In other embodiments, the yield of soluble CRM197 obtained is from about 1.0 g/L to about 10.0 g/L, from about 1.0 g/L to about 9.0 g/L, from about 1.0 g/L to about 8.0 g/L, from about 1.0 g/L to about 7.0 g/L, from about 1.0 g/L to about 6.0 g/L, from about 1.0 g/L to about 5.0 g/L, from about 1.0 g/L to about 4.0 g/L, from about 1.0 g/L to about 3.0 g/L or from about 1.0 g/L to about 2.0 g/L. In other embodiments, the yield of soluble CRM197 obtained is from about 2.0 g/L to about 10.0 g/L, from about 2.0 g/L to about 9.0 g/L, from about 2.0 g/L to about 8.0 g/L, from about 2.0 g/L to about 7.0 g/L, from about 2.0 g/L to about 6.0 g/L, from about 2.0 g/L to about 5.0 g/L, from about 2.0 g/L to about 4.0 g/L, from about 2.0 g/L to about 4.0 g/L or from about 2.0 g/L to about 3.0 g/L. In other embodiments, the yield of soluble CRM197 obtained is from about 3.0 g/L to about 10.0 g/L, from about 3.0 g/L to about 9.0 g/L, from about 3.0 g/L to about 8.0 g/L, from about 3.0 g/L to about 7.0 g/L, from about 3.0 g/L to about 6.0 g/L, from about 3.0 g/L to about 5.0 g/L, or from about 3.0 g/L to about 4.0 g/L.

A signal sequence is defined in the claims. A 5' signal sequence portion described herein may encode a signal recognition particle (SRP) dependent signal sequence such as the DsbA, TolB and TorT secretion signals, a Sec-dependent signal sequence such as the OmpF, OmpT, OmpA, PhoA, MalE, LamB, LivK and PelB secretion signals, or a twin arginine translocation (TAT) signal sequence such as the TorA and Sufl secretion signals. In some embodiments of the invention, the 5' signal sequence portion encodes a Sec-dependent signal sequence, preferably the OmpA or OmpF secretion signal. In a particularly preferred embodiment, the 5' signal sequence portion encodes the ompF secretion signal.

A 5' signal sequence portion described herein may encode a signal sequence selected from an MglB, MalE, OppA, RbsB, Agp, FkpA, YtfQ, HdeA, HdeB, OmpC and GlnH secretion signal. In a particularly preferred embodiment according to the invention, the 5' signal sequence portion encodes the YtfQ secretion signal.

In another related aspect, the *E. coli* host cell additionally comprises one or more nucleic acids comprising a sequence encoding one or more proteins for assisting the translocation and/or folding of CRM197 in the periplasm, operably linked to an expression control sequence. The nucleic acid(s) comprising a sequence encoding one or more proteins for assisting the translocation and/or folding of CRM197 in the periplasm may be part of the same expression vector comprising the nucleotide sequence encoding CRM197 or may be located on a different expression vector. Proteins for assisting the translocation and/or folding of CRM197 include, without limitation, chaperones such as Skp, DnaK, DnaJ, CaflM, and CaflA; disulfide bond formation proteins such as DsbA, DsbB, DsbC and DsbD; peptidyl-prolyl cis-trans isomerases such as PpiA, PpiD, FkpA and SurA; soluble partner proteins such as MBP, GST, and thioredoxin; secretion pathway proteins such as YebF, MalE, HlyA, Hirudin, OmpF, and Spy; protease inhibitors such as YccA; and proteins that relieve export saturation such as PspA.

In several aspects, the present invention relates to a method as claimed for producing a recombinant CRM197 protein wherein the yield of soluble CRM197 is about 1 to 10 g/L, preferably about 2 to 10 g/L, and further comprising harvesting the *E. coli* cells from the culture and lysing the harvested cells by a mechanical method in the absence of detergent. Optionally, the method further comprises obtaining a soluble fraction of the resulting lysate (e.g. by centrifugation to separate a soluble and insoluble fraction) and subjecting the soluble fraction (comprising soluble CRM197 produced by the *E. coli* host) to one or more purification steps. In one embodiment the soluble CRM197 is subjected to hydrophobic interaction chromatography and/or anion exchange chromatography. The *E. coli* host cell is a reduced genome *E. coli* host cell.

In other aspects, the invention relates to a reduced genome *E. coli* host cell comprising an expression vector, the expression vector comprising a nucleotide sequence encoding a CRM197 protein fused to an ompA, ompF or ytfQ signal sequence that directs transfer of the CRM197 protein to the periplasm, wherein said nucleotide sequence is operably linked to an expression control sequence, wherein the native parent *E. coli* strain is a K-12 strain, comprising a native -2 frameshift mutation wherein the native -2 frameshift mutation comprises a deletion of nucleotides GT at positions 983 and 984 of *ilvG* relative to the intact *ilvG,* preferably K12 MG1655, and wherein the reduced genome *E. coli* host comprises the following modifications: (i) deletion of at least the following DNA segments: b0245-b0301, b0303-b0310, b1336-b1411, b4426-b4427, b2441-b2450, b2622-b2654, b2657-b2660, b4462, b1994-b2008, b4435, b3322-b3338, b2349-b2363, b1539-b1579, b4269-b4320, b2968-b2972, b2975-b2977, b2979-b2987, b4466-4468, b1137-b1172, b0537-b0565, b0016-b0022, b4412-b4413, b0577-b0582, b4415, b2389-b2390, b2392-b2395, b0358-b0368, b0370-b0380, b2856-b2863, b3042-b3048, b0656, b1325-b1333, b2030-b2062, b2190-b2192, b3215-b3219, b3504-b3505, b1070-b1083, b1878-b1894, b1917-b1950, b4324-b4342, b4345-b4358, b4486, b0497-b0502, b0700-b0706, b1456-b1462, b3481-b3484, b3592-b3596, b0981-b0988, b1021-b1029, b2080-b2096, b4438, b3440-b3445, b4451, b3556-b3558, b4455, b1786, b0150-b0153 and b2945 of the *E*. *coli* K-12 strain MG1655, (ii) deletion of the *rph* gene to enhance orotate phosphoribosyltransferase activity (iii) correction of the native -2 frameshift mutation in the *ilvG* gene in order to restore the active acetohydroxy acid synthase II production, preferably wherein said correction is an insertion of two nucleotides between positions 982 and 983 and (iv) deletion of the *iclR and ankyrin-like regulator protein* (b4017) genes.

These and other embodiments of the present invention are described in more detail herein below.

### DESCRIPTION OF THE DRAWINGS

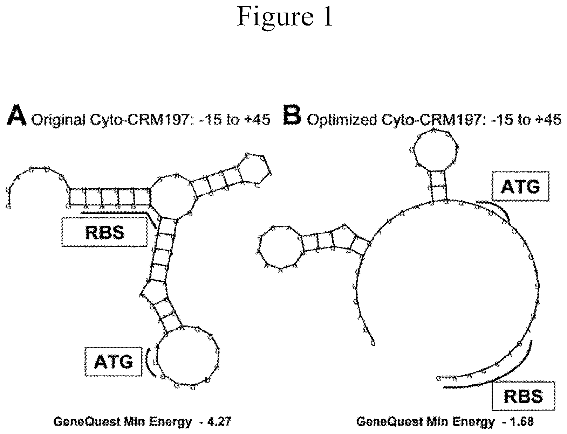

Figure 1 depicts the DNA sequence changes that result in a release of hairpin structures in the CRM197 sequence used in experiments aimed at examining the insoluble form of CRM197. The optimized sequence (B) generates a higher minimal energy (-1.68 for the optimized sequence compared to -4.27 for the original sequence) and relaxes secondary structure enhancing recognition of both the start site (ATG) and ribosomal binding site (RBS) relative to the original sequence.

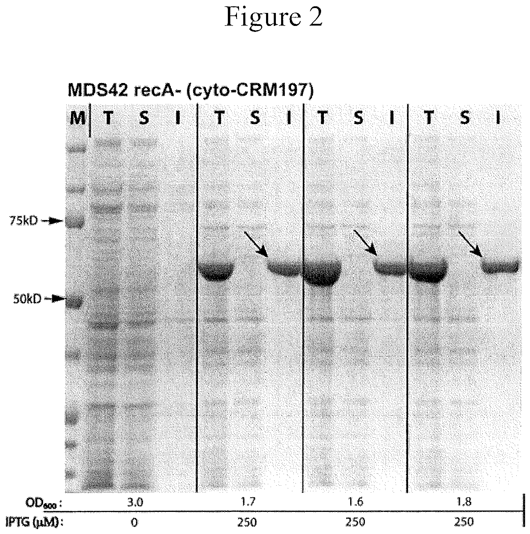

Figure 2 depicts cytosolic expression of CRM197 in reduced genome *E. coli* strain MDS42 *recA* (MDS42 strain with a *recA* deletion). Shake flask cultures were grown in minimal media to an optical density (OD) of 0.5 and then induced with either 0 or 250 µM IPTG as indicated. Electrophoresis was with a 4-12% gradient acrylamide Bis-Tris gel followed by protein staining using GelCode stain reagent. 0.04 OD of material was loaded per lane. M = marker lane; T = total cellular protein; S = soluble fraction; I = insoluble fraction. High amounts of cytosolic CRM197 were present in the insoluble fraction (arrows) validating the robust nature of the construct and strain in the production of insoluble CRM197.

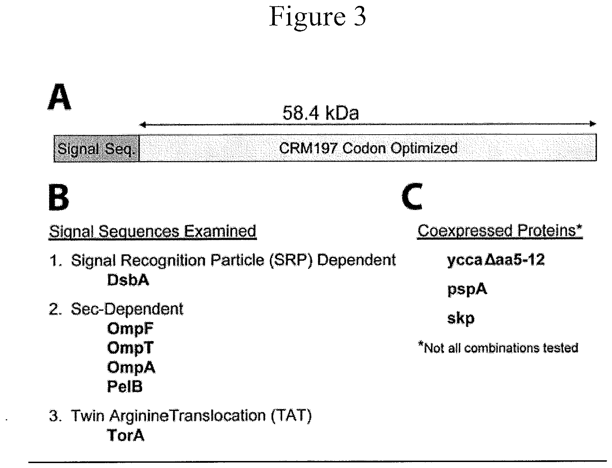

Figure 3 depicts signal sequences examined in relation to periplasmic delivery of CRM197 in reduced genome strain MDS42recA. The top panel (A) is an illustration of the heterologous signal sequence fused to the 5' end of the codon optimized CRM197 sequence. The lower left panel (B) illustrates signal sequences representative of the three *E. coli* secretion pathways. The lower right panel (C) illustrates proteins that were co-expressed with CRM197 to test their ability to assist translocation and/or folding of CRM197 in the periplasm.

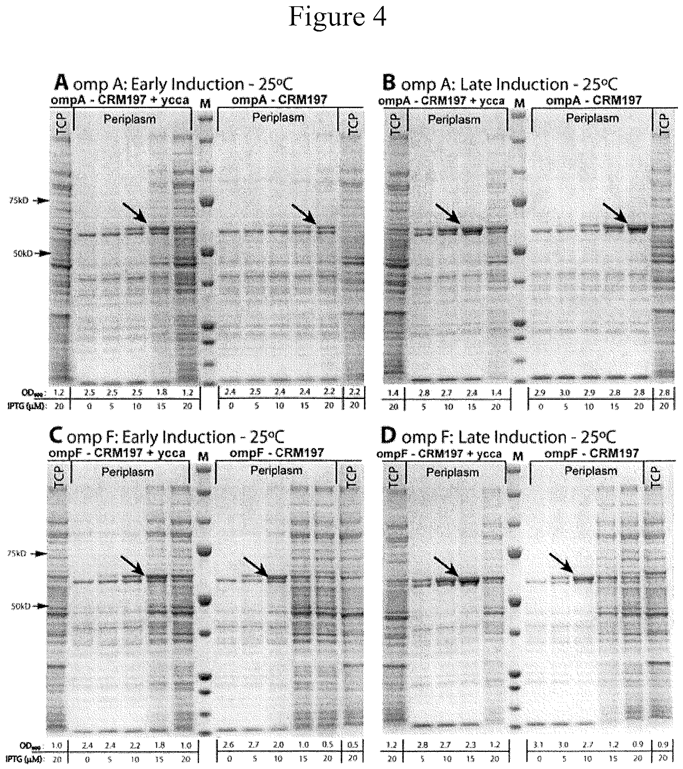

Figure 4 depicts protein gel analysis of CRM197 expression in MDS42recA using OmpA and OmpF signal sequences. Early (Panels A and C; inducer added at OD₆₀₀ of 0.01) and late (Panels B and D; inducer added at OD₆₀₀ of about 0.4) induction at 25 °C using either Δaa1-5 YccA (OmpA + CRM197 +/- YccA) or Δaa1-5 YccA (OmpF +CRM197 +/- YccA) as chaperones. Arrows indicate the highest level of CRM197 in each inducer series. Note the endogenous *E*. *coli* protein that migrates directly below CRM197. The gel method was as described for Figure 2. The periplasmic samples were prepared with the aid of Periplasting Buffer (Epicentre, Madison, WI). A 2 OD sample was harvested by centrifugation at 7,500 x g for 10 minutes in a 1.5 ml Eppendorf tube. The supernatant was removed and the cell pellet gently resuspended in 50 µl of Periplasting Buffer (200 mM Tris-HCl [pH 7.5], 20% sucrose, 1 mM EDTA, and 30 U/µl Ready-Lyse Lysozyme). After 5 minutes at room temperature, 50 µl of ice cold water was rapidly added to the resuspended pellet. The mixture was incubated on ice for 5 minutes prior to fractionating the periplasmic fraction from the spheroplasts by centrifuging at 4,000 x g for 15 minutes. The supernatant representing the periplasmic fraction was prepared for SDS-PAGE analysis. An amount equivalent to 0.12 OD units was loaded.

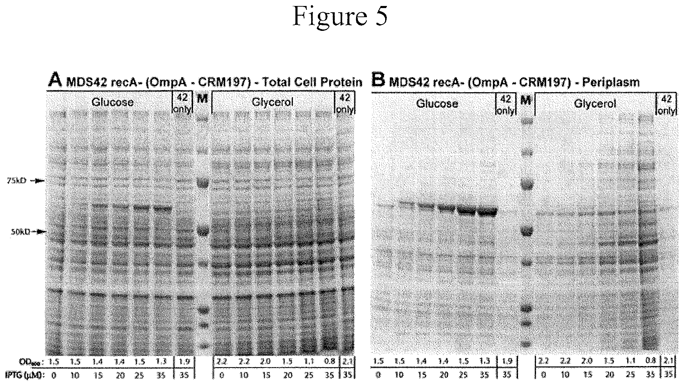

Figure 5 compares the effects of using glucose or glycerol as a carbon source on CRM197 expression in MDS42recA host cells. Panel A depicts total cellular protein (0.04 OD loaded per lane). Panel A depicts isolated periplasmic proteins (0.12 OD loaded per lane). Note that higher levels of CRM197 were generated in glucose-supplemented media. Lanes labeled "42 only" are control lanes with MDS42recA without the expression vector (i.e. not expressing CRM197).

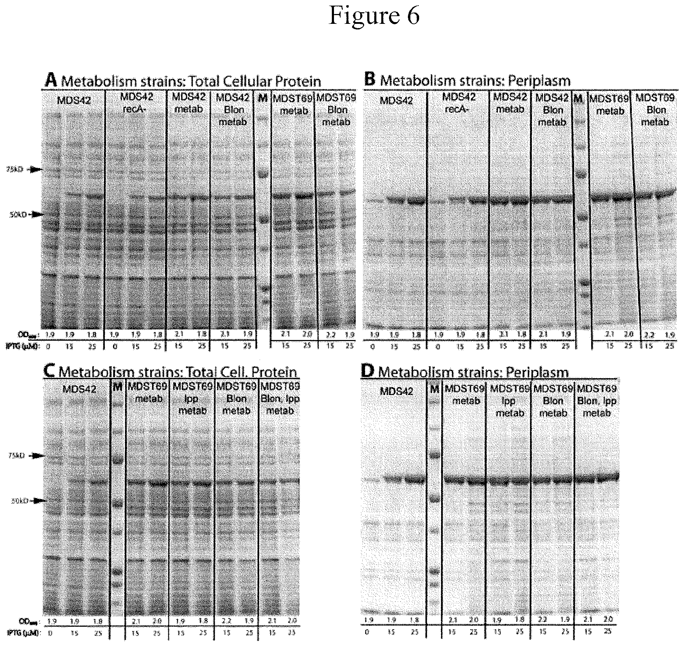

Figure 6 depicts protein gel analysis of CRM197 expression in eight reduced genome *E. coli* strains carrying an expression vector coding for CRM197 fused to an ompA signal sequence including four strains built onto the MDS42 platform and four strains built onto the MDS69 platform. MDS42 strains tested are: (i) MDS42, (ii) MDS42recA, (iii) MDS42 metab (MDS42 strain comprising corrections of the *rph* and *ilvG* frameshift mutations and deletions of the *iclR* and *arpA* genes) and (iv) MDS42 Blon metab (MDS42 metab further comprising a modification of the Lon protease (b0439) promoter region to mimic the sequence of the Ion promoter region of B strain *E*. *coli,* in which an IS insertion separates the -35 region from the -10 region of the ancestral *E*. *coli lon* promoter.). MDS69 strains tested are: (i) MDS69 metab (MDS69 strain comprising corrections of the *rph* and *ilvG* frameshift mutations and deletions of the *iclR* and *arpA* genes) (ii) MDS69 Blon metab (MDS69 metab further comprising the Lon protease promoter modification described above) (iii) MDS69 Ipp metab (MDS69 metab further comprising a deletion of the *Ipp* gene (nucleotides 1755260-1755687 of MG1655) and (iv) MDS69 Blon, Ipp metab. Panels A and C depict total cellular protein isolated after induction at the IPTG concentration indicated. Panels B and D indicate periplasmic isolation done in parallel. The gel method was as described in Figure 2.

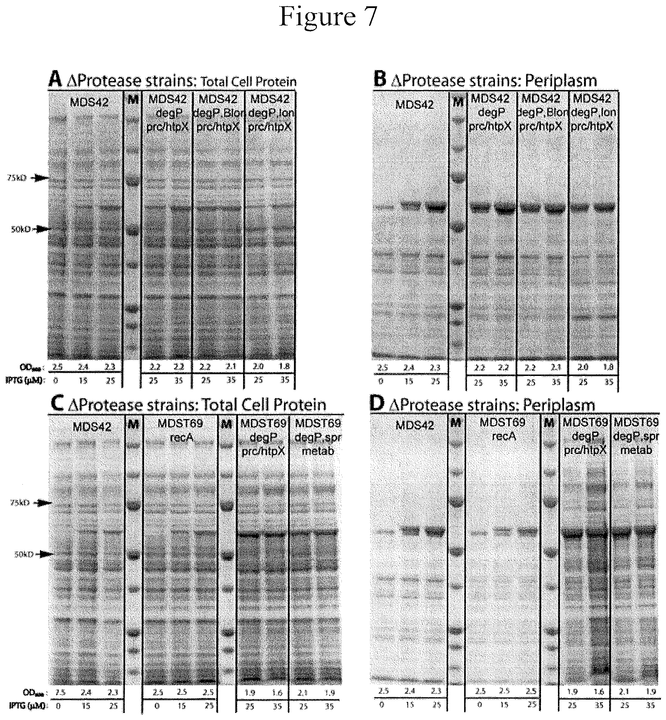

Figure 7 depicts protein gel analysis of CRM197 expression in MDS42 and MDS69 protease strains. Panels A and C depict total cellular protein isolated after induction at the IPTG concentration indicated. Panels B and D depict periplasmic isolation done in parallel. The gel method was as described in Figure 2.

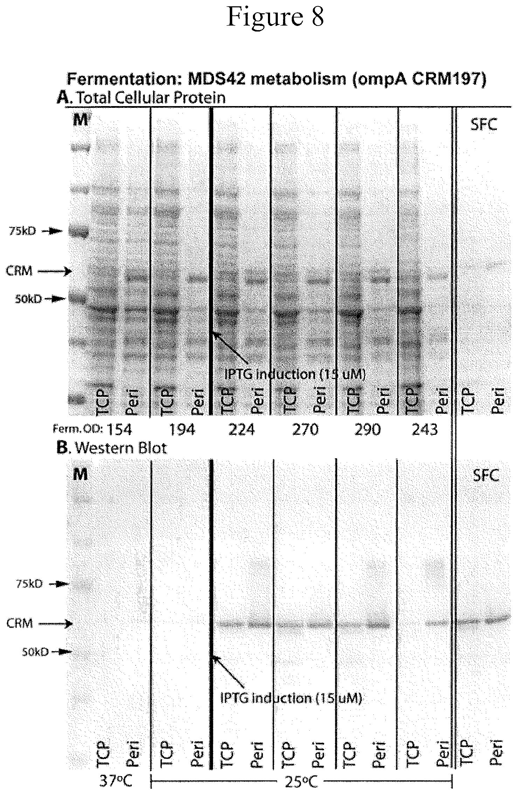

Figure 8 depicts protein gel and Western blot of fermentation samples following fed-batch fermentation of reduced genome *E*. *coli* strain MDS42 metab carrying an expression vector coding for CRM197 fused to an OmpA signal sequence in defined minimal media at the 10 liter scale. Panel A: total cell protein (TCP) and periplasmic (Peri) preparations were collected at the fermentation ODs indicated. IPTG was added at OD = 200. Panel B: Western blotting with anti-diphtheria toxin antibody was used to definitively identify CRM197. Note that CRM197 was not expressed prior to induction. SFC = shake flask control. 0.04 and 0.12 OD was loaded per lane for TCP and Peri samples, respectively. The gel method was as described in Figure 2.

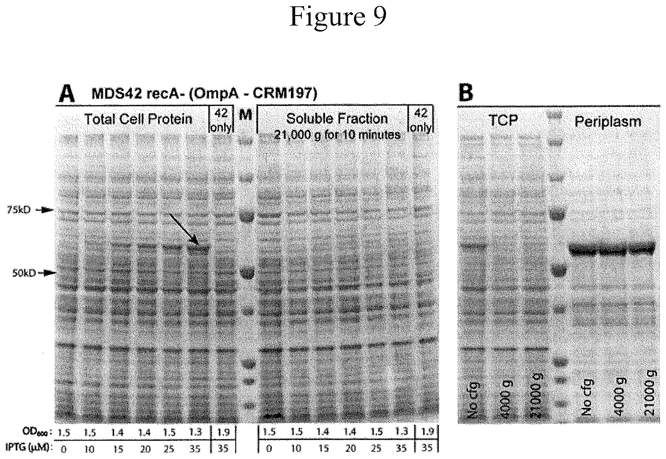

Figure 9 depicts protein gels of (i) total cell protein (TCP) isolated using a conventional detergent-based buffer (Panel A and left three lanes of Panel B) and (ii) periplasmic protein preparations (right three lanes of Panel B), following expression of OmpA-CRM197 in MDS42recA strain. Panel A: samples of total cell protein (left) were centrifuged for 10 minutes at high speed (21k g) and then reanalyzed (right). CRM197 is not found in the soluble fraction. Panel B: high speed centrifugation of total cell protein (TCP) and periplasmic fractions showing that CRM197 is insoluble in the cytoplasmic fractions exposed to detergent (left) and soluble in the periplasmic fractions (right) that were not exposed to detergent. The gel method was as described in Figure 2. The arrow indicates CRM197 in TCP that does not appear in the soluble fraction.

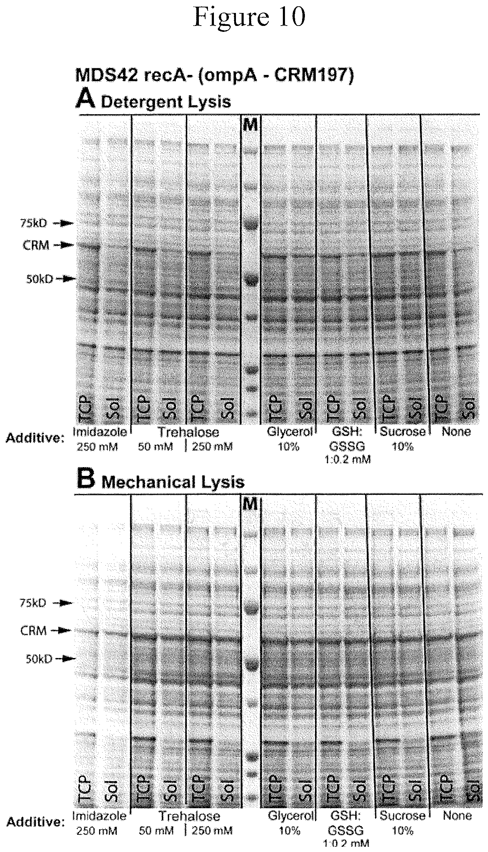

Figure 10 depicts protein gels of detergent and mechanical lysis and CRM197 solubility. MDS42recA cells carrying an expression vector encoding ompA fused to CRM197 were subjected to fed-batch fermentation as described for Figure 8. Cells were lysed using either (A) detergent (Bugbuster^{®} (Novagen), a proprietary mixture of non-ionic detergents that disrupt the cell membrane) or (B) mechanical (sonication) lysis in the presence of solubilization agents. Note that lysis in the absence of detergent resulted in high levels of soluble CRM197. GSH:GSSG = reduced to oxidized ratio of glutathione; M = marker; Sol = soluble fraction; TCP = total cell protein. Inducer (IPTG) = 35 µM.

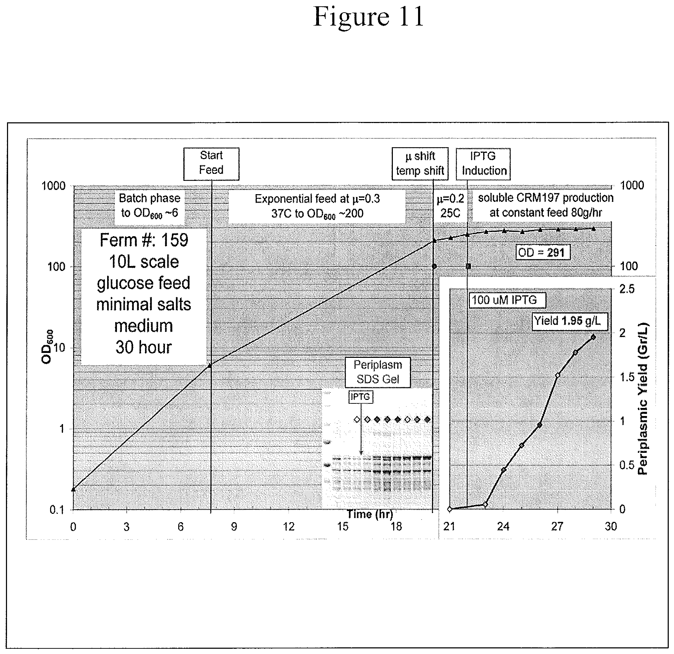

Figure 11 is a graph depicting fermentation of MDS69 metab host cells carrying an expression vector encoding OmpA-CRM197. CRM197 was found to increase in fed-batch fermentation up to the 30 hour time point where a maximum yield of 1.95 g/L was achieved.

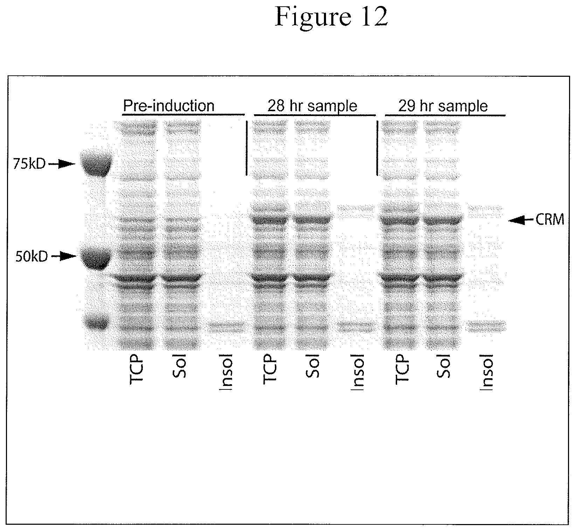

Figure 12 depicts protein gels from the fermentation of Figure 11 showing a high amount of soluble CRM197 in fermentation samples. Strain MDS69 metab was subjected to fed-batch fermentation in minimal media with glucose as the carbon source. Samples collected prior to the addition of inducer (22 hrs) or at either 28 or 29 hrs were homogenized by sonication and different cellular fractions isolated. CRM197 was found exclusively in the soluble (Sol) fraction. TCP = total cellular protein fraction; Insol = insoluble fraction.

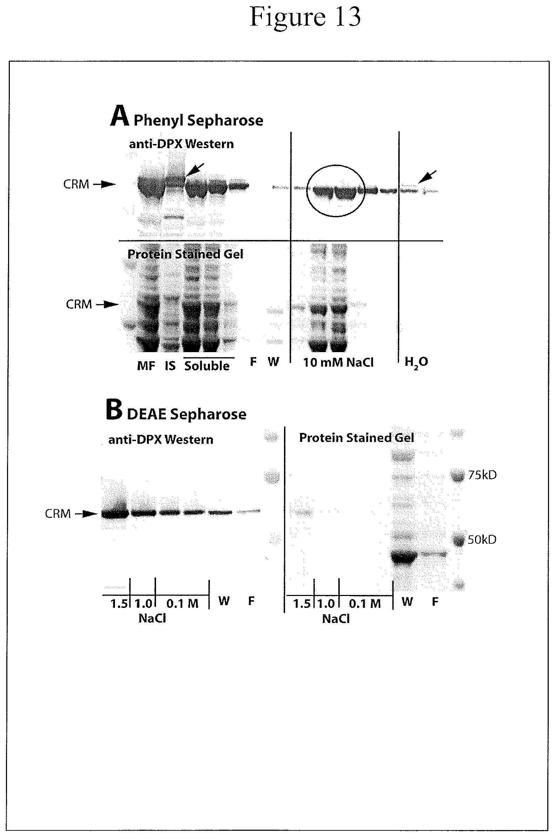

Figure 13 depicts the results of a two column purification of CRM197. Panel A: 50 OD of the 28 hr fermentation sample shown in Fig 12 was subjected to microfluidizer (MF) homogenization. Soluble and insoluble (IS) fractions were isolated and soluble material (25 OD equivalents) was loaded onto a phenyl sepharose column. The three lanes labeled "Soluble" are 0.1, 0.07 and 0.04 OD of pre-loaded soluble samples, respectively. CRM197-containing material was eluted with 10 mM NaCl, 10 mM phosphate buffer, pH 7.5. Five consecutive 2.5 ml samples were collected (left to right) and fractions indicated by a circle were pooled. Note the small amount of unprocessed CRM197 (arrows) that was purified away from the main eluted sample and found following elution using distilled water only. Panel B: Pooled samples from Panel A were loaded onto a DEAE sepharose column and eluted using different salt concentrations (0.1 M NaCl followed by 1 M NaCl and finally, 1.5 M NaCl). CRM197 of highest purity was eluted at 1 M NaCl. Anti-diphtheria toxin antibody (1:1000 dilution) Western blots are shown alongside protein-stained gels run in parallel. F, flow through; W, column wash; MF = total homogenate following microfluidization; IS = insoluble fraction following centrifugation of microfluidization homogenate; S = soluble fraction following centrifugation of microfluidization homogenate.

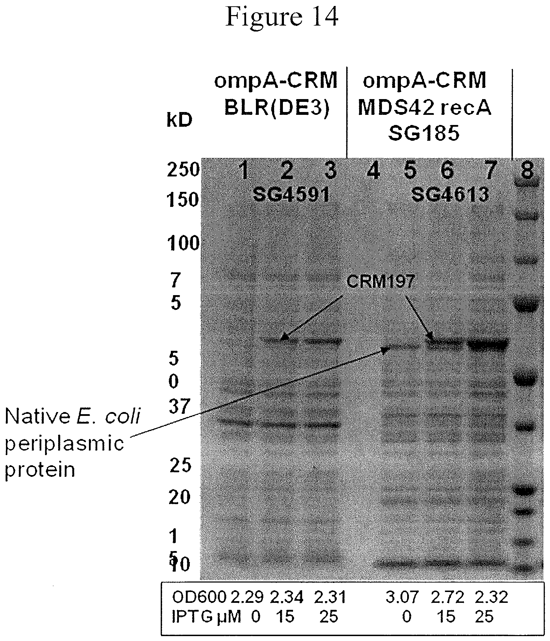

Figure 14 protein gel analysis of CRM197 expression in MDS42recA compared to wild type B strain BLR(DE3). Cells were transformed with an expression vector encoding an ompA-CRM197 fusion directing the protein to the periplasm and grown at 25 °C for 19 hours in shaken flasks. Expression of CRM197 was induced at OD = 0.3 (late stage) by addition of IPTG at the indicated concentrations. Periplasmic proteins were isolated and analyzed. Note the endogenous *E. coli* protein that migrates directly below CRM197.

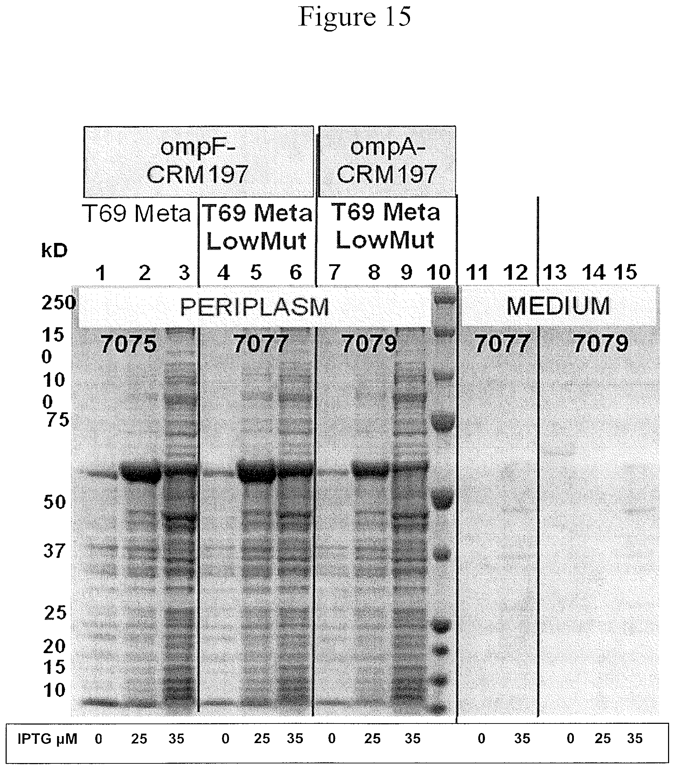

Figure 15 protein gel analysis of the periplasmic fractions generated from pSX2-OmpA CRM197 and pSX2-OmpF CRM197 expression in MDS69 metab and MDS69 metab low mutation rate hosts. The OmpF-CRM197 clone produces more soluble periplasmic CRM197 in the MDS69 meta low mut host (lanes 5-6) than the ompA-CRM197 clone (lanes 8-9) in the same host and using the same induction conditions. Lanes 11-15 represent samples of the medium supernatant after cell harvest and demonstrate that little to no CRM197 is released to the medium under these induction and growth conditions.

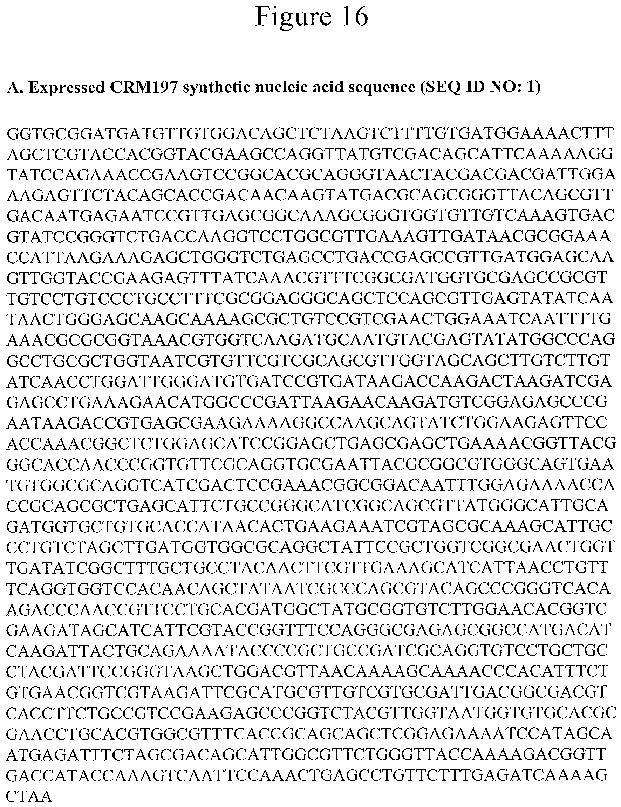

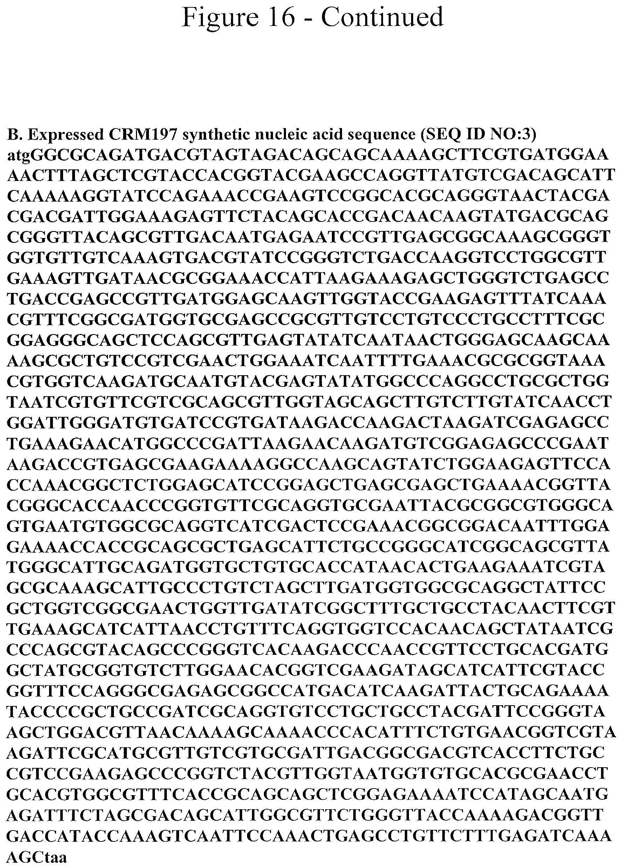

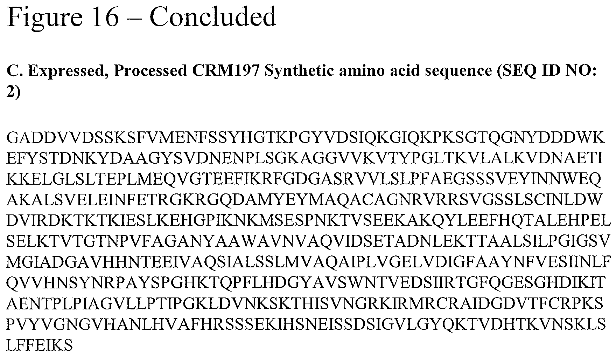

Figures 16A-16C depict CRM197 DNA and amino acid sequences. Figure 16A depicts the DNA sequence of the CRM197 ORF optimized for expression with a signal sequence to direct expression and processing of the mature CRM197 protein to the periplasmic space. Figure 16B depicts the DNA sequence of the CRM197 ORF optimized for expression in the cytoplasm. Figure 16C depicts the amino acid sequence of the mature CRM197 protein produced after signal sequence processing (Figure 16A) or N-terminal Methionine removal (Figure 16B).

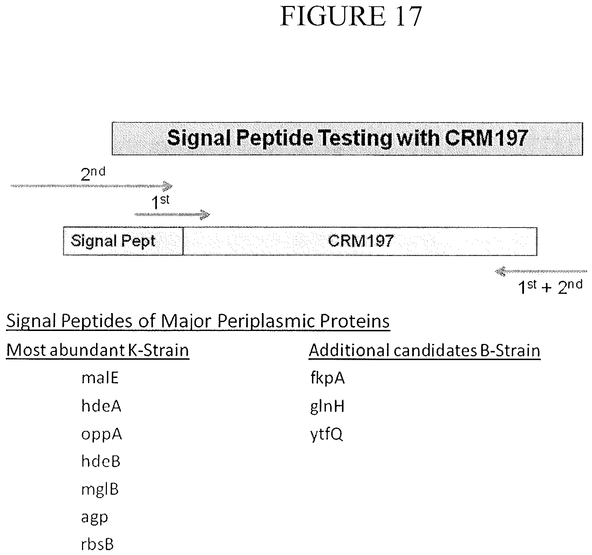

Figure 17 depicts signal sequences examined in relation to periplasmic delivery of CRM197 in reduced genome strain MDS69 metab, classified according to their relative abundance in *E. coli* K and/or B strains.

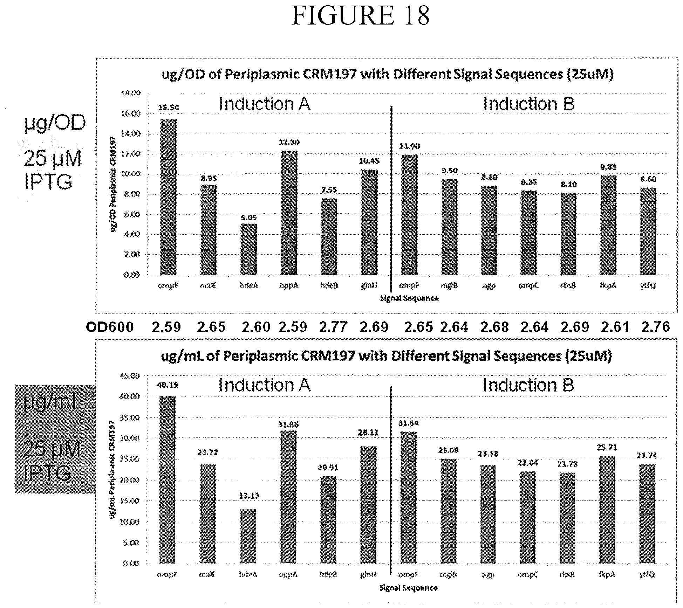

Figure 18 depicts the µg/OD (top panel) and µg/ml of periplasmic CRM197 (Caliper analysis) with the following signal sequences: OmpF, MalE, HdeA, OppA, HdeB and GlnH (Induction A) and OmpF, MgIB, Agp, OmpC, RbsB, FkpA, and YtfQ (Induction B) at 25 µM inducer (IPTG) added at OD ~ 0.3 in reduced genome *E. coli* strain MDS69 metab.

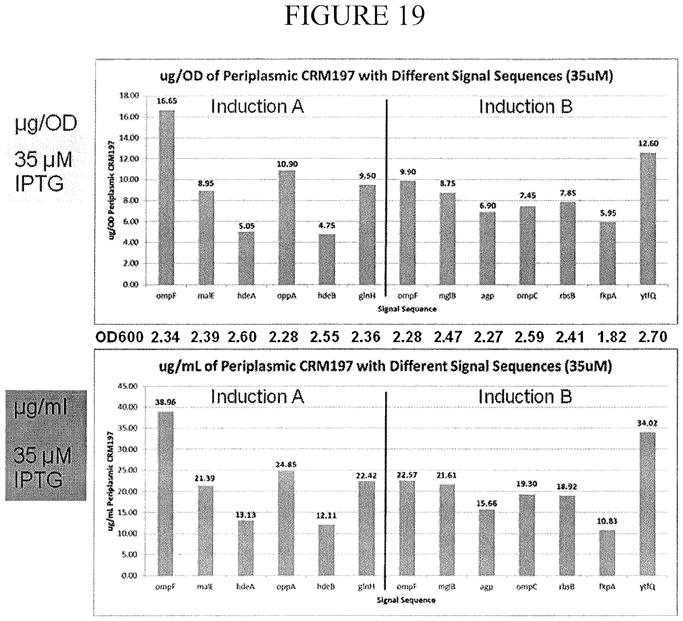

Figure 19 depicts the µg/OD (top panel) and µg/ml of periplasmic CRM197 (Caliper analysis) with the following signal sequences: OmpF, MalE, HdeA, OppA, HdeB and GlnH (Induction A) and OmpF, MglB, Agp, OmpC, RbsB, FkpA, and YtfQ (Induction B) at 35 µM inducer (IPTG) added at OD ~ 0.3 in reduced genome *E. coli* strain MDS69 metab.

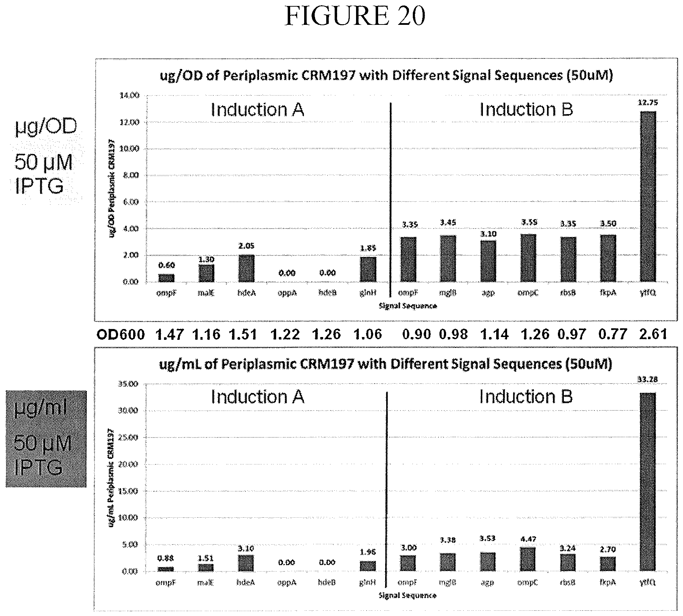

Figure 20 depicts the µg/OD (top panel) and µg/ml of periplasmic CRM197 (Caliper analysis) with the following signal sequences: OmpF, MalE, HdeA, OppA, HdeB and GlnH (Induction A) and OmpF, MglB, Agp, OmpC, RbsB, FkpA, and YtfQ (Induction B) at 50 µM inducer (IPTG) added at OD ~ 0.3 in reduced genome *E. coli* strain MDS69 metab.

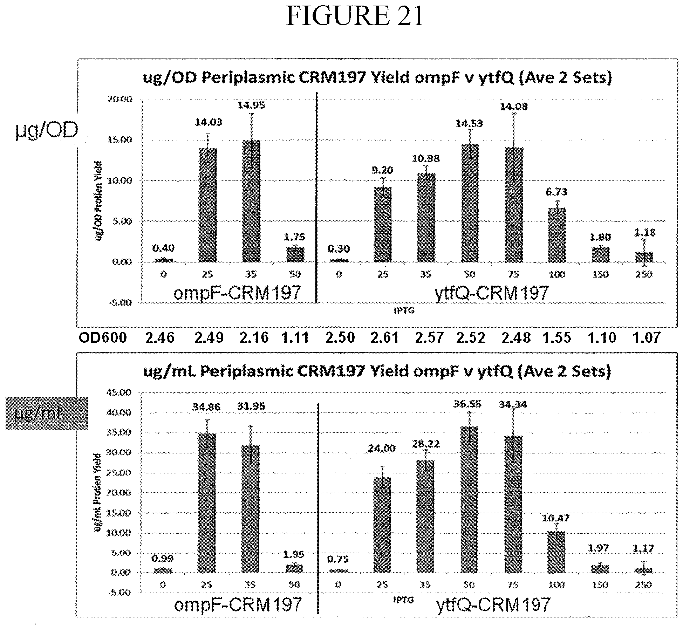

Figure 21 compares the µg/OD (top panel) and µg/ml of periplasmic CRM197 (Caliper analysis) obtained with the OmpF signal sequence at 0, 25 35 and 50 µM inducer and with the YtfQ signal sequence at 0, 25, 35, 50, 75, 100, 150 and 250 µM inducer added at OD ~ 0.3 in reduced genome *E. coli* strain MDS69 metab. The results are the average of two sets of experiments.

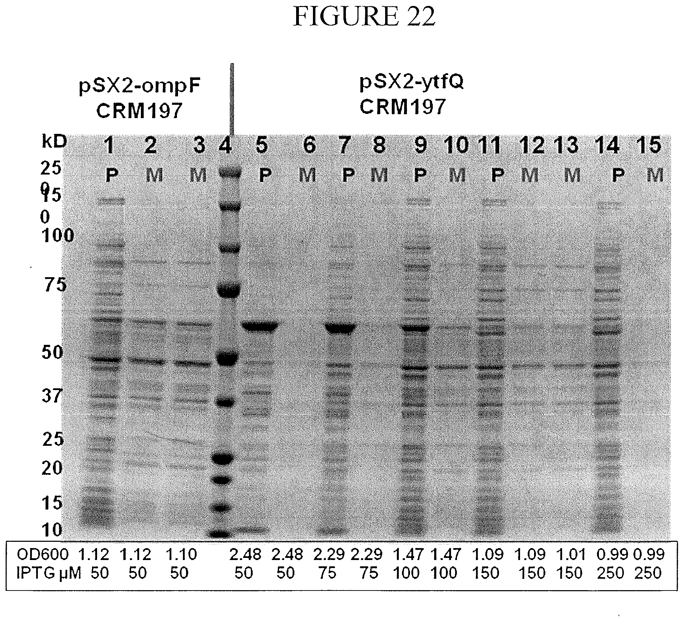

Figure 22 is a protein gel analysis comparing CRM197 yield in periplasm (P) and media (M) of MDS69 metab with OmpF or YtfQ signal sequence at inducer concentration of 50 µM (OmpF) or 50, 75, 100, 150,250 µM (YtfQ) added at OD = 0.3 (late stage). Samples were collected at the specified OD for analysis.

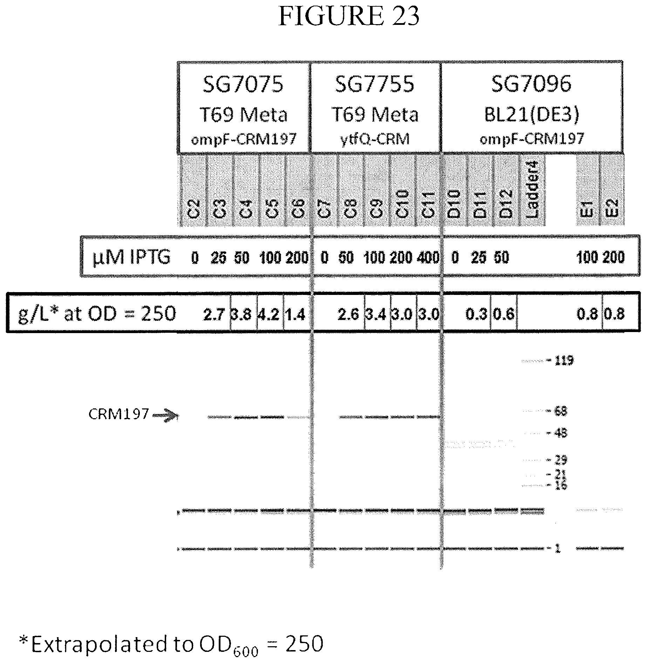

Figure 23 compares periplasmic expression of CRM197 in MDS metab cells with OmpF or YtfQ signal sequence and in BL21(DE3) cells with ompF signal sequence at the indicated inducer concentrations. Inducer was added at OD ~ 2 (very late induction) and samples were analyzed by Caliper. The g/L of (soluble) periplasmic CRM197 (extrapolated to OD600 = 250) is indicated at each concentration of inducer.

### DETAILED DESCRIPTION OF THE INVENTION

The present invention is defined by the claims. While the present invention is capable of being embodied in various forms, the description below of several embodiments is made with the understanding that the present disclosure is to be considered as an exemplification of the invention, and is not intended to limit the invention to the specific embodiments illustrated. Headings are provided for convenience only and are not to be construed to limit the invention in any manner. Embodiments illustrated under any heading may be combined with embodiments illustrated under any other heading.

The use of numerical values in the various ranges specified in this application, unless expressly indicated otherwise, are stated as approximations as though the minimum and maximum values within the stated ranges were both preceded by the word "about." In this manner, slight variations above and below the stated ranges can be used to achieve substantially the same results as values within the ranges. As used herein, the terms "about" and "approximately" when referring to a numerical value shall have their plain and ordinary meanings to one skilled in the pertinent art at issue. Also, the disclosure of ranges is intended as a continuous range including every value between the minimum and maximum values recited as well as any ranges that can be formed by such values. This includes ranges that can be formed that do or do not include a finite upper and/or lower boundary. This also includes ratios that are derivable by dividing a given disclosed numeral into another disclosed numeral. Accordingly, the skilled person will appreciate that many such ratios, ranges, and ranges of ratios can be unambiguously derived from the data and numbers presented herein and all represent various embodiments of the present invention.

A "reduced genome" bacterium as used herein means a bacterium having about 1% to about 75% of its genome (e.g. protein coding genes) deleted, for example about 5%, about 10%, about 20%, about 30% about 40%, about 50% or about 60% of the genome deleted. In one embodiment, the reduced genome bacteria used in the practice of the present invention have a genome that is preferably genetically engineered to be at least two percent (2%) and up to twenty percent (20%) (including any number therebetween) smaller than the genome of a native parent strain. Preferably, the genome is at least five percent (5%) and up to thirty percent (30%) smaller than the genome of a native parent strain. More preferably, the genome is eight percent (8%) to fourteen percent (14%) to twenty percent (20%) (including any number therebetween) or more smaller than the genome of the native parent strain. Alternatively, the genome may be engineered to be less than 20%, less than 30%, less than 40% or less than 50% smaller than the genome of a native parental strain. The term "native parental strain" means a bacterial strain found in a natural or native environment as commonly understood by the scientific community to represent the foundation of a strain line and on whose genome a series of deletions can be made to generate a bacterial strain with a smaller genome. Native parent strain also refers to a strain against which the engineered strain is compared and wherein the engineered strain has less than the full complement of the native parent strain. The percentage by which a genome has become smaller after a series of deletions is calculated by dividing "the total number of base pairs deleted after all of the deletions" by "the total number of base pairs in the genome before all of the deletions" and then multiplying by 100. Similarly, the percentage by which the genome is smaller than the native parent strain is calculated by dividing the total number of nucleotides in the strain with the smaller genome (regardless of the process by which it was produced) by the total number of nucleotides in a native parent strain and then multiplying by 100.

In one embodiment, a "reduced genome" bacterium means a bacteria for which removal of the above amounts of genome does not unacceptably affect the ability of the organism to grow on minimal medium. Whether removal of two or more genes "unacceptably affects" the ability of the organism to grow on minimal medium in the present context depends on the specific application. For example, a 30% reduction in proliferation rate may be acceptable for one application but not another. In addition, adverse effect of deleting a DNA sequence from the genome may be reduced by measures such as changing culture conditions. Such measures may turn an otherwise unacceptable adverse effect to an acceptable one. In one embodiment, the proliferation rate is approximately the same as the parental strain. However, proliferation rates ranging from about 5%, 10%, 15%, 20%, 30%, 40% to about 50% lower than that of the parental strain are within the scope of the invention. More particularly, doubling times of bacteria of the present invention may range from about fifteen minutes to about three hours. Non-limiting examples of suitable reduced genome bacteria, as well as methods for deleting DNA from a bacterium such as *E*. *coli,* are disclosed in U.S. Pat Nos. 6,989,265, 7,303,906, 8,119,365, 8,039,243 and 8,178,339.

The term "b number" used herein refers to the unique ID assigned to each gene of the K-12 MG1655 strain as described in Blattner et al., Science 277:1453-1474 (1997).

The term "CRM197" used herein refers to cross-reacting material 197 (CRM197), a diphtheria toxin variant having a single G → A transition leading to the substitution of glycine (at position 52 in the wild-type toxin) with glutamic acid in CRM197. This missense mutation is responsible for the loss of ADP-ribosyltransferase activity. See e.g. Giannini et al., Nucleic Acids Res. 12(10):4063-4069 (1984).

A method as claimed for producing a recombinant CRM197 protein in a reduced genome *E. coli* host cell is provided. It has been found that a surprisingly high yield of recombinant CRM197 can be produced in insoluble or soluble form using reduced genome *E. coli* host strains e.g. compared to wild type *E. coli* host strains. In one aspect, the method leads to increased production of insoluble CRM197 in the cytoplasm of the host cell. In other aspects, the method leads to increased production of soluble CRM197 in the periplasm of the host cell.

The native parent *E*. *coli* strain used to create the reduced genome *E. coli* host cell is defined in the claims, such as K-12 strain MG1655.

In some embodiments, K-12 MG1655 is used to produce recombinant CRM197 according to the methods herein described.

The nucleotide sequence of CRM197 for use according to the present invention may be prepared using recombinant DNA technology. For example, CRM197 can be chemically synthesized or can be prepared by site-directed mutagenesis based on the known nucleotide sequence of the wild type structural gene for diphtheria toxin carried by comyebacteriophage β (Greenfield et al., Proc Nat Acad Sci, 80:6953-6957 (1993)). Preferably, the nucleotide sequence of CRM197 is optimized for expression in *E*. *coli.*

A variety of sequence features of the heterologous nucleic acid can be optimized including, without limitation, modification of translation initiation regions, alteration of mRNA structural elements, and the use of different codon biases. Methods for optimizing nucleic acid sequence to improve expression in *E. coli* host cells are known in the art and described e.g. in U.S. Patent No. 7,561,972. Preferably, optimization of the nucleotide sequence of CRM197 for expression in *E. coli* comprises at least codon optimization. The presence of codons in the heterologous nucleic acid sequence that are rarely used in *E. coli* can delay translation of the encoded protein and result in a reduced expression in the *E. coli* host cell. Thus, in one aspect, the general codon usage in *E. coli* is used to optimize the expression of CRM197 in *E. coli.* Optimization of CRM197 for expression in *E. coli* also preferably includes minimization of interfering secondary structure. Interfering secondary structure can result in reduced expression of heterologous proteins in *E. coli* by impeding transcription and translation. For example, mRNA secondary structure at the initiation site has been inversely correlated to translational efficiency. An exemplary CRM197 nucleotide sequence, optimized for expression in the periplasm of *E. coli* when attached to an upstream region encoding a signal sequence is provided as SEQ ID NO: 1 (Figure 16A). An exemplary CRM197 nucleotide sequence, optimized for expression in the cytoplasm of *E. coli* when attached to an upstream ATG start codon is provided as SEQ ID NO: 3 (Figure 16B). It is to be understood that the methods of the present invention are not limited to the CRM197 nucleotide sequence set forth as SEQ ID NO: 1. Additional strategies for optimizing heterologous nucleotide sequences for expression in *E. coli* are known in the art and can be used in addition to or as an alternative to the strategies described above.

Processes for preparing recombinant heterologous proteins from genetically engineered bacterial host cells such as *E. coli* comprising expression systems are well known to those skilled in the art. Recombinant CRM197 can be expressed in reduced genome *E. coli* host cells by any of these methods. In one aspect, the present methods relate to reduced genome *E. coli* host cells comprising expression systems, the expression systems comprising nucleotide sequence encoding CRM197 operably linked to an inducible promoter such that CRM197 is expressed in the host cells when the promoter is induced. In a preferred aspect, the promoter is induced by addition of a suitable amount of IPTG. Introduction of a polynucleotide into the reduced genome *E. coli* host cell can be accomplished by any of several standard molecular biology techniques such as those described in Davis et al., Basic Methods in Molecular Biology (1986) and Sambrook et al., Molecular Cloning: A Laboratory Manual, 2nd Ed., Cold Spring Harbor Laboratory Press, Cold Spring Harbor, N.Y. (1989) including, without limitation, calcium phosphate transfection, microinjection, electroporation, conjugation, infection and the like. Similarly, any system or vector suitable to maintain, propagate or express polynucleotides and/or express a polypeptide in a host may be used to practice the present invention. For example, the appropriate DNA sequence may be inserted into a vector such as a plasmid by standard techniques.

One aspect of the invention relates to periplasmic expression of CRM197 in a reduced genome *E. coli* host cell. The expression of proteins in the periplasm has been used for industrial use and has been reviewed in Hanahan, J. Mol. Biol., 166:557-580 (1983); Hockney, Trends Biotechnol., 12:456-632 (1994); and Hannig et al., Trends Biotechnol., 16:54-60 (1998). Thus, methods are provided comprising growing a reduced genome *E. coli* comprising an expression vector comprising a nucleic acid sequence encoding a CRM197 protein fused to a signal sequence as claimed, operably linked to an expression control sequence under conditions suitable for the expression of the recombinant CRM197 protein, wherein the signal sequence directs transfer of the CRM197 protein to the periplasm of the *E. coli* host. According to these methods, a surprisingly high yield of intact soluble CRM197 is produced and substantially all of the soluble CRM197 can be recovered.

The presence of a signal sequence on a protein facilitates the transport of the newly translated protein across the inner membrane of *E. coli* into the periplasmic space. The signal sequence is then cleaved; accordingly replacement of the native C. *diphtheriae* signal sequence with a signal sequence that directs transfer of CRM197 to the periplasm of *E. coli* ultimately results in a mature protein having the same amino acid sequence.

The signal sequence is defined in the claims. Representative examples of signal sequences described herein capable of directing heterologous proteins to the *E. coli* periplasm are listed below. Preferably, the signal sequence results in direction of at least 70, 80, 90 or 100% of the polypeptide to the periplasm when expressed in *E. coli.*

| **Signal Sequence** | **Amino acid sequence** |
|---|---|
| PelB (pectate lyase B) | MKYLLPTAAAGLLLLAAQPAMA |
| OmpA (outer-membrane protein A) | MKKTAIAIAVALAGFATVAQA |
| StII (heat-stable enterotoxin 2) | MKKNIAFLLASMFVFSIATNAYA |
| Endoxylanase | MFKFKKKFLVGLTAAFMSISMFSATASA |
| PhoA (alkaline phosphatase) | MKQSTIALALLPLLFTPVTKA |
| OmpF (outer-membrane protein F) | MMKRNILAVIVPALLVAGTANA |
| PhoE (outer-membrane pore protein E) | MKKSTLALVVMGIVASASVQA |
| MalE (maltose-binding protein) | MKIKTGARILALSALTTMMFSASALA |
| OmpC (outer-membrane protein C) | MKVKVLSLLVPALLVAGAANA |
| Lpp (murein lipoprotein) | MKATKLVLGAVILGSTLLAG |
| LamB (λ receptor protein) | MMTTLRKLPLAVAVAAGVMSAQAMA |
| OmpT (protease VII) | MRAKLLGIVLTTPIAISSFA |
| LTB (heat-labile enterotoxin subunit B) | MNKVKCYVLFTALLSSLYAHG |
| MglB (methyl galactose transporter) | MNKKVLTLSAVMASMLFGAAAHA |
| OppA (oligopeptide transporter) | MTNITKRSLVAAGVLAALMAGNVALA |
| RbsB (subunit ribose transporter) | MNMKKLATLVSAVALSATVSANAMA |
| Agp (glucose-1 phosphatase, 3-phytase)) | MNKTLIAAAVAGIVLLASNAQA |
| FkpA (peptidyl-prolyl cis-trans isomerase) | MKSLFKVTLLATTMAVALHAPITFA |
| YtfQ (galactofuranose binding protein, subunit ABC transporter) | MWKRLLIVSAVSAAMSSMALA |
| HdeA (stress response induced by acidic conditions) | MKKVLGVILGGLLLLPVVSNA |
| HdeB (stress response induced by acidic conditions) | MNISSLRKAFIFMGAVAALSLVNAQSALA |
| GlnH (subunit of glutamine ABC transporter) | MKSVLKVSLAALTLAFAVSSHA |

Additional signal sequences for use according to the disclosure include, without limitation, CpdB (3'-nucleotidease/2',3'-cyclic nucleotide 2'-phosphodiesterase), YdeN (putative sulfatase), OsmY (induced by hyperosmotic stress), Art! (subunit Arginine ABC transporter), GltL (glutamate ABC transporter), and CybC (cytochrome b562).

The signal sequence is selected from the ytfQ, OmpA and OmpF signal sequences. In a particularly preferred embodiment, the signal sequence is the OmpF signal sequence. In another particularly preferred embodiment, the signal sequence is the YtfQ signal sequence.

A reduced genome *E. coli* strain as claimed used as a host cell according to the methods of the disclosure described herein. In one aspect, the reduced genome *E*. *coli* has a genome that is genetically engineered to be at least two percent (2%) and up to forty percent (40%) (including any number therebetween), such as between 5% and 30% or between 5% and 20%, smaller than the genome of its native parent strain. The percentage by which a genome has become smaller after a series of deletions is calculated by dividing "the total number of base pairs deleted after all of the deletions" by "the total number of base pairs in the genome of the parental strain before all of the deletions" and then multiplying by 100. In another aspect, the reduced genome bacterium has a genome that is between 4.41 Mb and 3.71 Mb, between 4.41 Mb and 3.25 Mb or between 4.41 Mb and 2.78 Mb. The reduced genome *E*. *coli* strain for use according to the methods described herein may be produced by cumulative genomic deletions of a parent *E*. *coli* strain by the methods described in International Patent Publication No. WO 2003/070880.

The parental *E*. *coli* strain is defined in the claims (e.g. MG1655 (ATCC No. 47076) or W3110 (ATCC No. 27325)). A particularly preferred parental *E. coli* strain is K-12 strain MG1655 (annotated version m56, NCBI accession no. U000961) with a genome having 4,639,674 base pairs.

A parental *E.coli* strain described herein may be a K-12 strain lacking one or more of the genes listed at Tables 2-20 of U.S. Patent No. 8,178,339.Table 2 of U.S. Patent No. 8,178,339 is reproduced below as Table 6:

**Table 6: reproduction of Table 2 of U.S. Patent No. 8,178,339**

| Strain | Type | Coordinates | Name | B | Function |
|---|---|---|---|---|---|
| MDS01 | CDS | complemeiit(262552...262893) | | b0245 | orf, hypothetical protein |
| MDS01 | CDS | complemant(262914...263231) | yafW | b0246 | orf, hypothetical protein |
| MDS01 | CDS | oomplemant(263480...263956) | ykfG | b0247 | putative DNA repair protein |
| MDS01 | CDS | comptement(263972...264430) | yafX | b0248 | orf, hypothetical protein |
| MDS01 | CDS | complemant(264528...264767) | ykfF | b0249 | orf, hypothetical protein |
| MDS01 | CDS | complement(254844...265311) | ykfb | b0250 | orf, hypothetical protein |
| MDS01 | CDS | complement(265334...266191) | yafY | b0251 | hypothetical transcriptinnal regulator |
| MDS01 | CDS | comptement(266408...267244) | yafZ | b0252 | orf, hypothetical protein |
| MDS01 | CDS | comptement(267321...268187) | ykfA | b0253 | putative OTP- binding protein |
| MDS01 | CDS | complement(268513...269406) | perR | b0254 | peroxide resistance protein |
| MDS01 | CDS | 269466...269870 | yi9la | b0255 | |
| MDS01 | CDS | 269827...270978 | tra8_1 | b0256 | IS30 transposase |
| MDS01 | CDS | 271054...271479 | | b0257 | putative transposase |
| MDS01 | CDS | 272086...273216 | ykfC | b0258 | orf, hypothetical protein |
| MDS01 | CDS | comptement(273325...274341) | tra5_1 | b0259 | IS5 transposase |
| MDS01 | CDS | 274525...275952 | ykfB | b0260 | orf, hypothetical protein |
| MDS01 | CDS | 275939...276871 | yafY | b0261 | hypothetical transcriptional regulator |
| MDS01 | CDS | complement(276980...278038) | afuC | b0262 | putative ATP-binding component of a transport system |
| MDS01 | CDS | complement(278038...278400) | | b0263 | |
| MDS01 | CDS | complement(218402...278905) | insB_2 | b0264 | IS1 protein InsB |
| MDS01 | CDS | complement(278824...279099) | insA_2 | b0265 | IS1 protein InaA |
| MDS01 | CDS | complement(279609...279986) | yagB | b0266 | orf, hypothetical protein |
| MDS01 | CDS | complement(280053...281207) | yagA | b0267 | orf, hypothetical protein |
| MDS01 | CDS | 281481...282410 | yagE | b0268 | putative lyase/synthase |
| MDS01 | CDS | 282425...284392 | yagF | b0269 | putative dehydratase |
| MDS01 | CDS | 284619...286001 | yagG | b0270 | putative permease |
| MDS01 | CDS | 286013...287623 | yagH | b0271 | putative bda-xyloaidase (EC 3.2.13T |
| MDS01 | CDS | complement(287628...288386) | yagI | b0272 | putative regulator |
| MDS01 | CDS | complement(288525...289529) | argF | b0273 | ornithine carbamoyltransferase 2, chain F |
| MDS01 | CDS | complement(289873...290376) | insB_3 | b0274 | IS1 protein InsB |
| MDS01 | CDS | comptement(290295...290570) | instA_3 | b0275 | IS1 protein InsA |
| MDS01 | CDS | 290724...291455 | yagJ | b0276 | orf, hypothetical protein |
| MDS01 | CDS | complement(291546...292172) | yagK | b0277 | orf, hypothetical protein |
| MDS01 | CDS | complement(292444...293142) | yegL | b0278 | DNA-hindingprotein |
| MDS01 | CDS | comptement(293169...294023) | yagM | b0279 | orf, hypothetical protein |
| MDS01 | CDS | complemant(294363...294803) | yagN | b0280 | orf, hypothetical protein |
| MDS01 | CDS | comptement(294920...296320) | intF | b0281 | putative phage integrase |
| MDS01 | CDS | complement:(296605...297015) | yagP | b0282 | putative transcriptional regulator LYSR-type |
| MDS01 | CDS | complement(296994...297950) | yagQ | b0283 | orf, hypothetical protein |
| MDS01 | CDS | oomplement:(297960...300158) | yagR | b0284 | orf, hypothetical protein |
| MDS01 | CDS | complement(300155...301111) | yagS | b0285 | orf, hypothetical protein |
| NDS01 | CDS | complement(301108...301797) | yagT | b0286 | putative xanthine dehydrogenase (EC 1.1.1.20 |
| MDS01 | CDS | 302215...302829 | yagU | b0287 | orf, hypothetical protein |
| MDS01 | CDS | complemant(303077...303406) | ykgl | b0288 | putative ferredoxin |
| MDS01 | CDS | complemet(303719...304474) | yagV | b0289 | orf, hypothetical protein |
| MDS01 | CDS | complement(304398...306041) | yagW | 60290 | putative receptor |
| MDS01 | CDS | complement(306031...308556) | yagX | 60291 | putative enzyme |
| MDS01 | CDS | complement(308582...309250) | yagY | 60292 | orf, hypothetical protein |
| MDS01 | CDS | complement(309308...309895) | yagZ | b0293 | orf, hypothetical protein |
| MDS01 | CDS | complement(309970...310560) | ykgK | 60294 | putative regulator |
| MDS01 | CDS | 311336...311563 | ytgL | b0295 | orf, hypothetical protein |
| MDS01 | CDS | complement(311738...312001) | ykgM | b0296 | putative ribosomal protein |
| MDS01 | CDS | 313581...314468 | eaeH | 60297 | attaching and effacing protein, pathogenesis factor |
| MDS01 | CDS | 314506...314814 | | b0298 | |
| MDS01 | CDS | 314811...315677 | tra5_5 | b0299 | |
| MDS01 | CDS | complement(315674...316393) | ykgA | b0300 | putative ARAC-type regulatory protein |
| MDS01 | CDS | complement(316950...317552) | ykgB | b0301 | orf, hypothetical protein |
| MDS01 | CDS | complement(317555...317806) | ykgI | b0303 | orf, hypothetical protein |
| MDS01 | CDS | complement(317900...319252) | ykgC | b0304 | putative oxidoreductase |
| MDS01 | CDS | 319451...320305 | ykgD | b0305 | putative ARAC-type regulatory protein |
| MDS01 | CDS | 320832...321551 | ykgB | b0306 | putative dehydrogenase subunit |
| MDS01 | CDS | 321562...322989 | ykgF | b0307 | orf, hypothetical protein |
| MDS01 | CDS | 322829...323677 | ykgG | b0308 | putative transporter |
| MDS01 | CDS | complement(323632...323844) | | b0309 | orf, hypothetical protein |
| MDS01 | CDS | complement(323920...324588) | ykgH | b0310 | orf, hypothetical protein |
| MDS02 | CDS | complement(1398271...1399803) | ydaH | 6133b | |
| MDS02 | CDS | complement(1399934...1401279) | | bl337 | |
| MDS02 | CDS | complement(1401279...1402604) | ydaJ | 61338 | |
| MDS02 | CDS | 1402765...1403673 | ydaK | b1339 | putative tranacriptianal regulator LYSR-type |
| MDS02 | CDS | 1404003...1404566 | ydaL | b1340 | orf, hypothetical protein |
| MDS02 | CDS | complement(1404587...1405879) | | b1341 | orf, hypothetical protein |
| MDS02 | CDS | 1406074...1407057 | | b1342 | orf, hypothetical protein |
| MDS02 | CDS | 1407535...1408908 | dbpA | bl343 | ATP-dependent RNA helicase |
| MDS02 | CDS | complement(1409037...1409972) | ydaO | bl344 | orf, hypothetical protein |
| MDS02 | CDS | complement(1410024...1411259) | | bl345 | |
| MDS02 | CDS | complement(1411261...1411500) | ydaQ | bl346 | putative lambdoid prophage Rac excisionase |
| MDS02 | CDS | complement(1411555...1411764) | ydaC | bl347 | orf, hypothetical protein |
| MDS02 | CDS | complement(1411757...1411951) | lar | bl348 | restriction alleviation and modification enhancement |
| MDS02 | CDS | complement(1412008...1412817) | recT | b1349 | recombinase, DNA renaturation |
| MDS02 | CDS | complement(1412810...1415410) | recE | b1350 | exonucleaseVIII, dt DNA exonuclease 5' --> 3' specific |
| MDS02 | CDS | complement(1415512...1415787) | racC | bl351 | RacC protein |
| MDS02 | CDS | complement(1416032...1416265) | kil | b1352 | Kil protein (killing function) of lambdoid praphage Rae |
| MDS02 | CDS | 1416572...1417183 | sieB | b1353 | phase superinfectian exclusion protein |
| MDS02 | CDS | 1417192...1417368 | | b1354 | orf, hypothetical protein |
| MDS02 | CDS | complement(1417346...1417525) | | b1355 | arf, hypothetical protein |
| MDS02 | CDS | complement(1417789...1418265) | ydaR | b13S6 | |
| MDS02 | CDS | 1418389...1418685 | ydaS | b1357 | orf, hypothetical protein |
| MDS02 | CDS | 1418708...1419130 | ydaT | b1358 | orf, hypothetical protein |
| MDS02 | CDS | 1419143...1420000 | ydaU | b1359 | orf, hypothetical protein |
| MDS02 | CDS | 1420007...1420753 | | b1300 | putative DNA replication fector |
| MDS02 | CDS | 1420725...1421336 | ydaW | b1361 | orf, hypothetical protein |
| MDS02 | CDS | 1421363...1421668 | | b1362 | putative Rac praphage endopeptidase |
| MDS02 | CDS | 1421806...1423263 | trkG | b1363 | |
| MDS02 | CDS | 1423202...1423483 | | b1364 | orf, hypothetical protein |
| MDS02 | CDS | 1423401...1423664 | | b1365 | orf, hypothetical protein |
| MDS02 | CDS | 1423645...1424004 | ydaY | b1366 | orf, hypothetical protein |
| MDS02 | CDS | 1424079...1424312 | | b1367 | orf, hypothetical protein |
| MDS02 | CDS | 1424478...1425506 | | b1368 | putative alpha helix protein |
| MDS02 | CDS | 1425482...1425637 | | b1369 | orf, hypothetical protein |
| MDS02 | CDS | complement(1425770...1426750) | trs5_5 | b1370 | |
| MDS02 | CDS | 1426547...1427008 | | b1371 | orf, hypothetical protein |
| MDS02 | CDS | 1427067...1430435 | | b1372 | |
| MDS02 | CDS | 1430435...1431010 | | b1373 | tail fiber assembly protein homolog from lambdoid praphage Rac |
| MDS02 | CDS | complement(1431108...1431698) | | b1374 | |
| MDS02 | CDS | complement(1432015...1432281) | ynaE | b1375 | erf, hypothetical protein |
| MDS02 | CDS | complement(1433209...1433715) | | b1376 | ynaF putative filament protein |
| MDS02 | CDS | complement(1433784...1434917) | | b1377 | |
| MDS02 | CDS | complement(1435284...1438808) | ydbK | b1378 | putative oxidoreductase, Fe-S subunit |
| MDS02 | CDS | complement(1439345...1439767) | hs1J | b1379 | heat shock protein HslJ |
| MDS02 | CDS | complement(1439878...1440867) | IdhA | b1380 | fermentative D-lactate dehydrogenase NAD-dependent |
| MDS02 | CDS | 1441075...1443714 | ydbH | b1381 | orf, hypothetical protein |
| MDS02 | CDS | 1443711...1443896 | ynbe | b1382 | orf, hypothetical protein |
| MDS02 | CDS | 1443898...1444230 | ydbL | b1383 | orf, hypothetical protein |
| MDS02 | CDS | complement(1444402...1445307) | feaR | b1384 | regulatory protein for 2-phenylethylamine catabolism |
| MDS02 | CDS | 1445540...1447042 | feaB | b1385 | phenylacetaldehyde dehydrogenase |
| MDS02 | CDS | complement(1447100...1449373) | tynA | b1386 | copper amine oxidase (tynminc oxidase) |
| MDS02 | CDS | complement(1449621...1451666) | maoC | b1387 | |
| MDS02 | CDS | 1451951...1452880 | ydbO | b1388 | |
| MDS02 | CDS | 1452892...1453179 | ynbF | b1389 | |
| MDS02 | CDS | 1453188...1453934 | ydbP | b1390 | |
| MDS02 | CDS | 1453943...1454446 | | b1391 | |
| MDS02 | CDS | 1454454...1455524 | | b1392 | |
| MDS02 | CDS | 1455521...1456288 | ydbS | b1393 | |
| MDS02 | CDS | 1456288...1457076 | | b1394 | |
| MDS02 | CDS | 1457078...1458505 | ydbU | b1395 | |
| MDS02 | CDS | 1458495...1458917 | | b1396 | |
| MDS02 | CDS | 1458917...1460122 | | b1397 | |
| MDS02 | CDS | 1460149...1461462 | | b1398 | |
| MDS02 | CDS | 1461563...1462513 | | b1399 | |
| MDS02 | CDS | 1462495...1463085 | | b1400 | |
| MDS02 | CDS | 1463416...1465974 | ydbA_1 | b1401 | |
| MDS02 | CDS | complement(14b5945...1466850) | yi22_2 | b1402 | IS2 hypothetical protein |
| MDS02 | CDS | complement(1466808...1467218) | yi21_2 | b1403 | IS2 hypothetical protein |
| MDS02 | CDS | 1467382...1468533 | tra8_2 | b1404 | IS30 transposase |
| MDS02 | CDS | 1468714...1472037 | ydbA_2 | b1405 | |
| MDS02 | CDS | 1472245...1473105 | ydbC | b1406 | putative dehydrogenase |
| MDS02 | CDS | 1473162...1475474 | ydbD | b1407 | orf, hypothetical protein |
| MDS02 | CDS | 1475639...1476250 | | b1408 | |
| MDS02 | CDS | 1476250...1477146 | | b1409 | putative phosphatidate cytidiltransferase |
| MDS02 | CDS | 1477162...1478919 | | b1410 | orf, hypothetical protein |
| MDS02 | CDS | 1478933...1480225 | ynbD | b1411 | putative enzymes |
| MDS02 | misc_RNA | complement(1403676...1403833) | IS061 | b4426 | |
| MDS02 | misc_RNA | 1435145...1435252 | tke8 | b4427 | |
| MDS03 | CDS | complement(2555340...2556743) | cutB | b2441 | ethanolamine ammonit-lyise, heavy chain |
| MDS03 | CDS | 2556793...2558088 | | b3442 | putative praphage integrase |
| MDS03 | CDS | 2558279...2558920 | | b2443 | orf, hypothetical protein |
| MDS03 | CDS | 2559390...2559635 | | b2444 | orf, hypothetical protein |
| MDS03 | CDS | 2559632...2560015 | | b2445 | orf, hypothetical protein |
| MDS03 | CDS | 2560133...2560549 | | b2446 | orf, hypothetical protein |
| MDS03 | CDS | 2560546...2561139 | | b2447 | orf, hypothetical protein |
| MDS03 | CDS | 2561599...2561991 | | b2448 | orf, hypothetical protein |
| MDS03 | CDS | 2562002...2562394 | | b2449 | orf, hypothetical protein |
| MDS03 | CDS | 2562515...2563354 | | b2450 | orf, hypothetical protein |
| MDS04 | CDS | 2754181... 2755422 | intA | b2622 | praphage CP4-57 integrase |
| MDS04 | CDS | complement(2755666...2756622) | yfjH | b2623 | putative histone |
| MDS04 | CDS | 2756666...2756878 | alpA | b2624 | prophage C14-57 regulatory protein alpA |
| MDS04 | CDS | 2757007...2758416 | yfjI | b2625 | orf, hypothetical protein |
| MDS04 | CDS | 2758569...2759195 | yfjJ | b2626 | orf, hypothetical protein |
| MDS04 | CDS | complement(2759373...2761562) | yfjK | b2627 | orf, hypothetical protein |
| MDS04 | CDS | complement(2761559...2763175) | yfjL | b2b28 | orf, hypothetical protein |
| MDS04 | CDS | complement(2763535...2763798) | yfjM | b2629 | orf, hypothetical protein |
| MDS04 | CDS | 2763940...2765013 | yfjN | b2630 | putative cell division protein |
| MDS04 | CDS | 2765057...2765377 | yfjO | b2631 | orf, hypothetical protein |
| MDS04 | CDS | 2765732...2766595 | yfjP | b2632 | putative GTP-binding protein |
| MDS04 | CDS | 2766687...2767508 | yfjQ | b2633 | orf, hypothetical protein |
| MDS04 | CDS | 2767725...2768426 | yfjR | b2634 | arf, hypothetical protein |
| MDS04 | CDS | 2768311...2768703 | | b2635 | orf, hypothetical protein |
| MDS04 | CDS | 2768454...2769146 | | b2636 | orf, hypothetical protein |
| MDS04 | CDS | 2769170...2769637 | yfjT | b2637 | orf, hypothetical protein |
| MDS04 | CDS | complement(2769862...2770176) | | b2638 | orf, hypothetical protein |
| MDS04 | CDS | complement(2770189...2770707) | | b2639 | putative pump protein |
| MDS04 | CDS | complement(2770858...2771058) | | b2640 | orf, hypothetical protein |
| MDS04 | CDS | complement(2770998...2771114) | | b2641 | orf, hypothetical protein |
| MDS04 | CDS | 2771340 ... 2773043 | yfjW | b2642 | orf, hypothetical protein |
| MDS04 | CDS | 2773941 ... 2774399 | yfjX | b2643 | orf, hypothetical protein |
| MDS04 | CDS | 2774408 ... 2774890 | yfjY | b2644 | putative DNA repair protein |
| MDS04 | CDS | 2775137 ... 2775454 | yfjZ | b2645 | orf, hypothetical protein |
| MDS04 | CDS | 2775475 ... 2775804 | ypjF | b2646 | orf, hypothetical protein |
| MDS04 | CDS | complement(2776168 ... 2780877) | ypjA | b2647 | putative ATP-binding companent of a transport system |
| MDS04 | CDS | complement(2781087 ... 2781230) | | b2648 | orf, hypothetical protein |
| MDS04 | CDS | complement(2781660 ... 2782451) | | b2649 | orf, hypothetical protein |
| MDS04 | CDS | complement(2782551 ... 2783033) | | b2650 | orf, hypothetical protein |
| MDS04 | CDS | 2783243 ... 2783374 | | b2651 | orf, hypothetical protein |
| MDS04 | tRNA | complement(2783784 ... 2783859) | ileY | b2652 | tRNA-Ile |
| | | | | | |
| MDS04 | CDS | camplement(2783822 ... 2783995) | | b2653 | orf, hypothetical protein |
| MDS04 | CDS | 2784419 ... 2784751 | | b2654 | orf, hypothetical protein |
| MDS04 | CDS | 2785628 ... 2786260 | | b2657 | putative enzyme |
| MDS04 | CDS | 2786399...2786671 | | b2658 | orf, hypothetical protein |
| MDS04 | CDS | 2786902 ... 2787984 | | b2659 | orf, hypothetical protein |
| MDS04 | CDS | 2787938 ... 2789272 | ygaF | b2660 | orf, hypothetical protein |
| MDS04 | CDS | 2784770 ... 2785456 | ygaR | b4462 | orf, hypothetical protein |
| MDS05 | CDS | complement(2064329 ... 2065345) | trs5_6 | b1994 | IS5 transposase |
| MDS05 | CDS | 2066632 ... 2067051 | | b1995 | orf, hypothetical protein |
| MDS05 | CDS | complement(2066976...2067881) | yi22_3 | b1996 | IS2 hypothetical protein |
| MDS05 | CDS | complement(2067839...2068249) | yi21_3 | b1997 | IS2 hypothetical protein |
| MDS05 | CDS | 2068268 ... 2068528 | | b1998 | orf, hypothetical protein |
| MDS05 | CDS | 2068525 ... 2069235 | yeeP | b1999 | putative histone |
| MDS05 | CDS | 2069563 ... 2072682 | flu | b2000 | antigen 43, phase-variable bipartite outer membrane fluffing protein |
| MDS05 | CDS | 2072797 ... 2074335 | | b2001 | orf, hypothetical protein |
| MDS05 | CDS | 2074332 ... 2074778 | yeeS | b2002 | putative DNA repair protein, RADC family |
| MDS05 | CDS | 2074841 ... 2075062 | yeeT | b2003 | orf, hypothetical protein |
| MDS05 | CDS | 2075136 ... 2075504 | yeeU | b2004 | putative structural protein |
| MDS05 | CDS | 2075593 ... 2075967 | yeeV | b2005 | orf, hypothetical protein |
| MDS05 | CDS | 2075964...2076158 | yeeW | b2006 | orf, hypothetical protein |
| MDS05 | CDS | complement(2077056 ... 2077451) | yeeX | b2007 | putative alpha helix protein |
| MDS05 | CDS | complement(2077557 ... 2078615) | yeeA | b2008 | orf, hypothetical protein |
| MDS05 | misc_RNA | 2069339...2069542 | | b4435 | IS102 |
| MDS06 | CDS | complement(3451530...3451949) | | b3322 | calcium-binding protein required for initiation of chromosome replication |
| MDS06 | CDS | complement(3451951...3453420) | yheD | b3323 | putative export protein A for general secretion pathway (GSP) |
| MDS06 | CDS | 3453600 ... 3454415 | yheE | b3324 | |
| MDS06 | CDS | 3454387...3456351 | yheF | b3325 | |
| MDS06 | CDS | 3456361 ... 3457942 | yheG | b3326 | |
| MDS06 | CDS | 3457839...3459035 | hofF | b3327 | |
| MDS06 | CDS | 3459045 ... 3459492 | hofG | b3328 | |
| MDS06 | CDS | 3459490... 3459999 | hofH | b3329 | |
| MDS06 | CDS | 3459957 ... 3460373 | yheH | b3330 | |
| MDS06 | CDS | 3460366... 3460953 | yheI | b3331 | |
| MDS06 | CDS | 3460946... 3461929 | yheJ | b3332 | |
| MDS06 | CDS | 3461941 ... 3463107 | yheK | b3333 | |
| MDS06 | CDS | 3463080 ... 3463565 | pshM | b3334 | |
| MDS06 | CDS | 3463565 ... 3464242 | hofD | b3335 | |
| MDS06 | CDS | camplement(3464271 ... 3464747) | bfr | b3336 | bacterioferrin, an iron storage homoprotein |
| MDS06 | CDS | complement(3464819...3465013) | yheA | b3337 | |
| MDS06 | CDS | complament(3465182...3467875) | yheB | b3338 | |
| MDS07 | CDS | 2464567... 2465724 | intC | b2349 | |
| MDS07 | CDS | 2465877... 2466239 | | b2350 | |
| MDS07 | CDS | 2471542... 2471988 | | b2350 | orf, hypothetical protein |
| MDS07 | CDS | 2466236... 2467156 | | b2351 | |
| MDS07 | CDS | 2467153 ... 2468484 | | b2352 | putative ligase |
| MDS07 | CDS | 2468783...2469127 | | b2353 | |
| MDS07 | CDS | complement(2469099...2469539) | | b2354 | orf, hypothetical protein |
| MDS07 | CDS | complement(2469566...2470084) | yidL | b2355 | putative RNA polymerase beta |
| MDS07 | CDS | complement(2470134...2470442) | yfdM | b2356 | orf, hypothetical protein |
| MDS07 | CDS | complement(2470409...2470903) | yfdN | b2357 | orf, hypothetical protein |
| MDS07 | CDS | complement(2470900...2471268) | yfdO | b2358 | orf, hypothetical protein |
| MDS07 | CDS | 2472054... 2472878 | | b2360 | orf, hypothetical protein |
| MDS07 | CDS | 2472979 ... 2473542 | | b2361 | orf, hypothetical protein |
| MDS07 | CDS | 2473533 ... 2473895 | | b2362 | orf, hypothetical protein |
| MDS07 | CDS | 2473895...2474200 | | b2363 | orf, hypothetical protein |
| MDS08 | CDS | 1625541 ... 1626287 | ydfG | b1539 | putative oxidoreductase |
| MDS08 | CDS | 1626376... 1627062 | ydfH | b1540 | orf, hypothetical protein |
| MDS0B | CDS | 1627239 ... 1627442 | | b1541 | orf, hypothetical protein |
| MDS08 | CDS | complement(1627477...1628937) | ydfl | b1542 | putative oxidoreductase |
| MDS08 | CDS | complement(1629026...1630309) | | b1543 | putative transport protein |
| MDS08 | CDS | 1631063 ... 1631329 | ydfK | b1544 | orf, hypothetical protein |
| MDS08 | CDS | 1631646... 1632236 | | b1545 | |
| MDS08 | CDS | complement(1632334...1632909) | ydfM | b1546 | tail fiber assembly protein homolog from lambdoid praphage Qin |
| MDS08 | CDS | complement(1632909...1633871) | | b1547 | |
| MDS08 | CDS | complement(1633822...1634391) | nohA | b1548 | DNA packaging protein NU1 homolog from lambdoid prophages |
| MDS08 | CDS | 1635056...1635481 | ydfO | b1549 | orf, hypothetical protein |
| MD60B | CDS | complement(1635633...1635809) | | b1550 | |
| MDS08 | CDS | complement(1635978...1636169) | | b1551 | orf, hypothetical protein |
| MDS08 | CDS | complement(1636479...1636691) | cspI | b1552 | cold shock- like protein |
| MDS08 | CDS | complement(1637054...1637551) | | b1553 | orf, hypothetical protein |
| MDS08 | CDS | complement(1637548...1638081) | | b1554 | |
| | | | | | |
| MDS08 | CDS | complement(1638078...1638389) | | b1555 | orf, hypothetical protein |
| MDS08 | CDS | complement(1638394...1638684) | | b1556 | |
| MDS08 | CDS | complement(1639363...1639578) | cspB | b1557 | |
| MDS08 | CDS | 1639879...1640091 | cspF | b1558 | |
| MDS08 | CDS | complement(1640513...1641295) | | b1559 | |
| MDS08 | CDS | complement(1641279...1642367) | | b1560 | orf, hypothetical protein |
| MDS08 | CDS | complement(1642675...1642926) | rem | b1561 | orf, hypothetical protein |
| MDS08 | CDS | complement(1643143...1643298) | horD | b1562 | polypeptide destructive to membrane potential |
| MDS08 | CDS | complement(1643370...1643657) | relE | b1563 | orf, hypothetical protein |
| MDS08 | CDS | complement(1643657...1643896) | rclB | b1564 | negative regulator of translation |
| MDS08 | CDS | 1643921...1644226 | | b1565 | orf, hypothetical protein |
| MDS08 | CDS | 1644429...1644761 | flxA | b1566 | orf, hypothetical protein |
| MDS08 | CDS | complement(1645198...1645347) | | b1567 | orf, hypothetical protein |
| MDS08 | CDS | complement(1645370...1645660) | | b1568 | orf, hypothetical protein |
| MDS08 | CDS | complement(1645644...1645874) | dicC | b1569 | regulator of dicB |
| MDS08 | CDS | 1645958...1646365 | dicA | b1570 | regulator of dicB |
| MDS08 | CDS | 1646532... 1646697 | ydfA | b1571 | orf, hypothetical protein |
| MDS08 | CDS | 1646647...1646817 | ydfB | b1572 | orf, hypothetical protein |
| MDS08 | CDS | 1646847... 1647065 | ydfC | b1573 | orf, hypothetical protein |
| MDS08 | misc_RNA | 1647406 ... 1647458 | dicF | b1574 | DicF antisene RNA, inhibits ftsZ translation |
| MDS08 | CDS | 1647633 ... 1647821 | diaB | b1575 | inhibition of cell division |
| MDS08 | CDS | 1647818...1648009 | ydfD | b1576 | orf, hypothetical protein |
| MDS08 | CDS | 1648102 ... 1649022 | ydfE | b1577 | orf, hypothetical protein |
| MDS08 | CDS | 1648905... 1649561 | | b1578 | orf, hypothetical protein |
| MDS08 | CDS | 1649536 ... 1650732 | | b1579 | |
| MDS09 | CDS | complement(4493213...4494274) | xjgB | b4269 | putative oxidoreductae |
| MDS09 | tRNA | 4494428...4494512 | leuX | b4270 | tRNA-Leu |
| MDS09 | CDS | 4494773 ... 4495963 | intB | b4271 | praphage P4 integrase |
| MDS09 | CDS | 4496250 ... 4496660 | yi21_6 | b4272 | IS2 hypothetical protein |
| MDS09 | CDS | 4496618 ... 4497523 | yi22_6 | b4273 | IS2 hypothetical protein |
| MDS09 | CDS | 4497622...4497957 | yjgW | b4274 | orf, hypothetical protein |
| MDS09 | CDS | complement(4498066...4498512) | yjgX | b4275 | orf, hypothetical protein |
| MDS09 | CDS | complement(4498455 ... 4498904) | yjgY | b4276 | orf, hypothetical protein |
| MDS09 | CDS | 4499283...4499612 | yjgZ | b4277 | orf, hypothetical protein |
| MDS09 | CDS | complement(4500126...4501454) | yi41 | b4278 | IS4 hypothetical protein |
| MDS09 | CDS | 4502021 ... 4503298 | yjhB | b4279 | putative transport protein |
| MDS09 | CDS | 4503295 ... 4504428 | yjhC | b4280 | putative dehydrogenase |
| MDS09 | CDS | complement(4504649...4505023) | yjhD | b4281 | orf, hypothetical protein |
| MDS09 | CDS | 4504929 ... 4505132 | yjhE | b4282 | orf, hypothetical protein |
| MDS09 | CDS | 4505184...4505486 | yi91b | b4283 | |
| MDS09 | CDS | complement(4505469 ... 4506640) | tra8_3 | b4284 | IS30 transposase |
| MDS09 | CDS | 4506981 ... 4507577 | | b4285 | putative transposase |
| MDS09 | CDS | 4507743 ... 4508156 | | b4286 | orf, hypothetical protein |
| MDS09 | CDS | camplement(4308713 ... 4509480) | fecE | b4287 | ATP-binding component of citrate-dependent |
| MDS09 | CDS | complament(4509481 ... 4510437) | feed | b4288 | citrate-dependent iron transport, membrane-bound protein |
| MDS09 | CDS | complement(4510434...4511432) | fecC | b4289 | citrate-dependent iron(III) transport protein, cytosolic |
| MDS09 | CDS | complement(11429...4512337) | fecB | b4290 | citrate-dependent iron transport, periplasmic protein |
| MDS09 | CDS | complement(4512376...4514700) | fecA | b4291 | outer membrane receptor; citrate-dependent iron |
| MDS09 | CDS | complement(4514787...4515740) | fecR | b4292 | outer membrane receptor; citrate-dependent iron tranrport, outer membrane receptor |
| MDS09 | CDS | complement(4515737...4516258) | fecI | b4293 | probable RNA polymerase sigma factor |
| MDS09 | CDS | 4516550 ... 4516825 | insA_7 | b4294 | IS1 protein InsA |
| MDS09 | CDS | complement(4517361... 4518161) | yjhU | b4295 | orf, hypothetical protein |
| MDS09 | CDS | complement(4518694...4520043) | yjhF | b4296 | putative transport system permease |
| MDS09 | CDS | complement(4520150...4522117) | yjhG | b4297 | putative dehydratase |
| MDS09 | CDS | complement(4522128...4523087) | yjhH | b4298 | putative lyase/synthase |
| MDS09 | CDS | complement(4523038...4523826) | yjhI | b4299 | putative regulator |
| MDS09 | CDS | complement(4524129...4524911) | sgcR | b4300 | putative DEOR-type transcriptional regulator |
| MDS09 | CDS | complement(4524928...4525560) | sgcE | b4301 | putative epimerase |
| MDS09 | CDS | complement(4525572...452δ003) | sgcA | b4302 | putative PTS system enzyme II A component |
| MDS09 | CDS | complement(4526134...4526940) | sgcQ | b4303 | putative nucleoside triphosphatase |
| MDS09 | CDS | complement(4526953...4528266) | sgcC | b4304 | putative PTS system enzyme IIC component |
| MDS09 | CDS | complement(4528553...4529704) | sgcX | b4305 | putative lyase/synthase |
| MDS09 | CDS | complement(4530460...4531206) | yjhP | b4306 | putative methyltransferase |
| MDS09 | CDS | complement(4531262...4531807) | yjhQ | b4307 | orf, hypothetical protein |
| MDS09 | CDS | 4533038 ... 4534054 | yjhR | b4308 | putative frameahift suppressor" |
| MDS09 | CDS | complement(4534637...4535617) | yjhS | b4309 | orf, hypothetical protein |
| MDS09 | CDS | complement(4535682...4536896) | yjht | b4310 | orf, hypothetical protein |
| MDS09 | CDS | complement(4536808...4537533) | yjhA | b4311 | orf, hypothetical protein |
| MDS09 | CDS | 4538980...4539582 | fimB | b4312 | recombinase involved in phase variation; regulator for fimA" |
| MDS09 | CDS | 4540060...4540656 | fimE | b4313 | recombinase involved in phase variation; regulator for fimA" |
| MDS09 | CDS | 4541138...4541686 | fimA | b4314 | major type 1 subunit fimbrin (pilin) |
| MDS09 | CDS | 4541643...4542290 | fimI | b4315 | fimbrial protein |
| MDS09 | CDS | 4542327...4543052 | fimC | b4316 | periplasmic chaperone, required for type 1 fimbriae |
| MDS09 | CDS | 4543119...4545755 | fimD | b4317 | outer membrane protein; export and assembly of type 1 fimbriae, inteu opted |
| MDS09 | CDS | 4545765 ... 4546295 | fimF | b4318 | fimbrial morphology |
| MDS09 | CDS | 4546308... 4546811 | fimG | b4319 | fimbrial morphology |
| MDS09 | CDS | 4546831...4547733 | fimH | b4320 | minor fimbrial subunit, D-mannose specific adhesin |
| MDS10 | CDS | complement(3108612...3109148) | yghD | b2968 | putative secretion pathway protein |
| MDS10 | CDS | complement(3109150...3110010) | yghE | b2969 | putative general secretion pathway for protein export (GSP) |
| MDS10 | CDS | complement(3110076...3110942) | | b2970 | putative general secretion pathway for protein export (GSP) |
| MDS10 | CDS | complement(3111089...3111499) | | b2971 | orf, hypothetical protein |
| MDSI0 | CDS | complement(3111565...3112497) | | b2972 | |
| MDS10 | CDS | complement(3117619...3119301) | yghK | b2975 | putative permease |
| MDS10 | CDS | complement(3119656...3121827) | glcB | b2976 | malate synthase G |
| MDS10 | CDS | complement(3121849...3122253) | glcG | b2977 | orf, hypothetical protein |
| MDS10 | CDS | complement(3124544...3126043) | glcD | b2979 | glycolate oxidase subunit D |
| MDS10 | CDS | 3126294...3127058 | gloC | b2980 | transcriptional activator for glc operon |
| MDS10 | CDS | complement(3127065...3128237) | | b2981 | orf, hypothetical protein |
| MDS10 | CDS | 3128200...3129216 | trs5_9 | b2982 | IS5 transposase |
| MDS10 | CDS | complement(3129363...3130430) | yghQ | b2983 | orf, hypothetical protein |
| MDS10 | CDS | complement(3130476...3131234) | yghR | b2984 | orf, hypothetical protein |
| MDS10 | CDS | complement(3131266...3131979) | yghS | b2985 | orf, hypothetical protein |
| MDS10 | CDS | 3132153... 3132845 | yghT | b2986 | orf, hypothetical protein |
| MDS10 | CDS | complement(3132894...3134393) | pitB | b2987 | low-affinity phosphate transport |
| MDS10 | CDS | complement(3112572...3117134) | yghJ | b4466 | putative lipoprotein |
| MDS10 | CDS | complement(3122258...3123481) | glcF | b4467 | glycolate oxidase iron-sulfur subunit |
| MDS10 | CDS | complement(3173492...3124544) | gleE | b4468 | glycolate oxidase iron-sulfur subunit |
| MDS11 | CDS | complement(1196090...1196755) | ymfD | b1137 | orf, hypothetical protein |
| MDS11 | CDS | complement(1196756...1197460) | ymfE | b1138 | orf, hypothetical protein |
| MDS11 | CDS | 1197918...1198811 | lit | b1139 | phage T4 late gene expression; at locus of e14 element |
| MDS11 | CDS | complement(1198902...1200029) | intE | b1140 | prophage e14 integrase |
| MDS11 | CDS | complement(1200010...1200255) | | b1141 | |
| MDS11 | CDS | complement(1200292...1200603) | ymfH | b1142 | |
| MDS11 | CDS | 1200675...1201061 | ymfI | b1143 | |
| MDS11 | CDS | complement(1200999...1201283) | ymfJ | b1144 | |
| MDS11 | CDS | complement(1201482...1202156) | | b1145 | |
| MDS11 | CDS | 1201944...1202447 | | b1146 | |
| MDS11 | CDS | 1202479... 1203048 | ymfL | b1147 | |
| MDS11 | CDS | 1203045...1203383 | ymfM | b1148 | |
| MDS11 | CDS | 1201393...1204760 | ymfN | b1149 | |
| MDS11 | CDS | 1204772...1204954 | ymfR | b1150 | |
| MDS11 | CDS | 1204954...1205427 | ymfO | b1151 | |
| MDS11 | CDS | 1205354...1206145 | | b1152 | |
| MDS11 | CDS | 1206136...1206720 | | b1153 | |
| MDS11 | CDS | 1206724...1207353 | ycfK | b1154 | |
| MDS11 | CDS | 1207355...1207768 | | b1155 | |
| MDS11 | CDS | complement(1207740...1208342) | ycfA | b1156 | |
| MDS11 | CDS | complement(1208342...1208881) | | b1157 | |
| MDS11 | CDS | 1208908...1209462 | pin | b1158 | inversion of adjacent DNA; at locus of e14 element |
| MDS11 | CDS | 1209589...1210402 | mcrA | b1159 | restriction of DNA at 5-methylcytosine residues; at locus of e14 element |
| MDS11 | CDS | complement(1210903...1211226) | ycgW | b1160 | orf, hypothetical protein |
| MDS11 | CDS | complement(1211926...1212330) | ycgX | b1161 | orf, hypothetical protein |
| MDS11 | CDS | complement(1212551...1213282) | ycgB | b1162 | putative transcriptional regulator |
| MDS11 | CDS | complement(1213487...1214698) | | b1163 | orf, hypothetical protein |
| MDS11 | CDS | 1215012...1215248 | ycgZ | b1164 | orf, hypothetical protein |
| MDS11 | CDS | 1215291...1215563 | ymgA | b1166 | orf, hypothetical protein |
| MDS11 | CDS | 1215592...1215858 | ymgB | b1166 | orf, hypothetical protein |
| MDS11 | CDS | 1215971...1216219 | ymgC | b1167 | orf, hypothetical protein |
| MDS11 | CDS | 1216509...1218074 | | b1168 | putative proteases |
| MDS11 | CDS | 1218824...1220344 | | b1169 | putative ATP-binding component of a transport system |
| | | | | | |
| MDS11 | CDS | 1220429...1221445 | | b1170 | |
| MDS11 | CDS | complement(1221528...1221863) | | b1171 | orf, hypothetical protein |
| MDS11 | CDS | complement(1221867...1222151) | | b1172 | orf, hypothetical protein |
| MDS12 | CDS | complement(564038...565201) | intD | b0537 | prophage DLP12 integrase |
| MDS12 | CDS | 565195...565755 | | b0538 | putative sensory transduction regulator |
| MDS12 | CDS | complement(563321...565584) | | b0539 | |
| MDS12 | CDS | 566056...566364 | | b0540 | |
| MDS12 | CDS | 566361...567227 | tra5_2 | b0541 | |
| MDS12 | CDS | 567333...567470 | | b0542 | orf, hypothetical protein |
| MDS12 | CDS | 567538...567870 | emrE | b0543 | methylviologen resistance |
| MDS12 | CDS | 568125...569651 | ybcK | b0544 | orf hypothetical protein |
| MDS12 | CDS | 570116...570667 | ybcL | b0545 | orf, hypothetical protein |
| MDS12 | CDS | 570677...571474 | ybcM | b0546 | putative ARAC-type regulatory protein |
| MDS12 | CDS | 571689...572144 | ybcw | b0547 | orf, hypothetical protein |
| MDS12 | CDS | 572144...572314 | ninE | b0548 | similar to phage 82 and lambda proteins |
| MDS12 | CDS | 572307...572597 | ybeo | b0549 | orf, hypothetical protein |
| MDS12 | CDS | 512594... 572956 | rus | b0550 | endodeoxyribonuclease RUS (Hollidty junction resolvase) |
| MDS12 | CDS | 573179...573562 | ybcQ | b0551 | orf, hypothetical protein |
| MDS12 | CDS | complement(573960...574976) | trs5_2 | b0552 | IS5 transposase |
| MDS12 | CDS | complement(574981...576108) | nmpC | b0553 | outer membrane porin protein; locus of qsr prophage |
| MDS12 | CDS | 576621...576836 | ybcR | b0554 | orf, hypothetical protein |
| MDS12 | CDS | 576836...577333 | ybcS | b0555 | bacteriophage lambda lysozyme homolog |
| MDS12 | CDS | 577330...577791 | ybcT | b0556 | bacteriophage lambda endopeptidase homolog |
| MDS12 | CDS | complement(577823...578116) | ybcU | b0557 | bacteriophage lambda Bor protein homolog |
| MDS12 | CDS | complement(578407...578859) | ybcV | b0558 | putative an envelop protein |
| MDS12 | CDS | 579103...579309 | ybcw | b0559 | orf, hypothetical protein |
| MDS12 | CDS | 580057...580602 | nohB | b0560 | bacteriophage DNA packaging protein |
| MDS12 | CDS | 580577...581320 | ybcX | b0561 | orf, hypothetical protein |
| MDS12 | CDS | complement(581375...581959) | ybcY | b0562 | orf, hypothetical protein |
| MDS12 | CDS | 582098...582283 | ylcE | b0563 | orf, hypothetical protein |
| MDS12 | CDS | 582904...583653 | appY | b05δ4 | regulatory protein affecting appA and other genes |
| MDS12 | CDS | complement(583903...584856) | ompT | b0565 | outer membrane protein 3b (a), protease VII |
| MDS13 | CDS | 15445...16557 | yi81_ | b0016 | IS186 hypothetical protein 1 |
| MDS13 | CDS | complement(15869...16177) | yi82-1 | b0017 | |
| MDS13 | CDS | complement(15751...16960) | mokC | b0018 | regulatory peptide whose translation enables hake (gef) expression |
| MDS13 | CDS | 17489...18655 | nhaA | b0019 | Na+/H antiporter, pH dependent |
| MDS13 | CDS | 18715 ... 19620 | nhaR | b0020 | transcriptional activator of nhaA |
| MDS13 | CDS | complement(19811...20314) | insB_1 | b0021 | IS1 protein InsB |
| MDS13 | CDS | complement(20233...20508) | insA | b0022 | IS1 protein InsA_1 |
| MDS13 | CDS | complement(16751...16903) | hokC | b4412 | small toxic membrane polypeptide |
| MDS13 | misc_RNA | 16952...17006 | sokC | b4413 | antisense RNA blocking mokC (orf69) and hokC (gef) translation |
| MDS14 | CDS | complement(602639...603886) | ybdG | b0577 | putative transport |
| MDS14 | CDS | complement(603994...604647) | nfnB | b0578 | oxygen-insensitive NAD(P)H nitroreductase |
| MDS14 | CDS | complement(604741...605109) | ybdF | b0579 | orf, hypothetical protein |
| MDS14 | CDS | complement(605174...605422) | ybdJ | b0580 | orf, hypothetical protein |
| MDS14 | CDS | complement(605488...606606) | ybdβ | b0581 | orf, hypothetical protein |
| MDS14 | CDS | 607288...608400 | yi81_2 | b0582 | IS186 hypothetical protein |
| MDS14 | CDS | 607059...607211 | hoke | b4415 | small toxic membrane polypeptide |
| MDS15 | CDS | 2507652...2508908 | | b2389 | orf, hypothetical protein |
| MDS15 | CDS | 2509023...2509349 | ypeC | b2390 | orf, hypothetical protein |
| MDS15 | CDS | complement(2509490...2510728) | | b2392 | |
| MDS15 | CDS | 2511064... 2512266 | nupC | b2393 | permease of transport system for 3 nucleosides |
| MDS15 | CDS | 2512347...2513465 | yi81_3 | b2394 | |
| MDS15 | CDS | complement(2513665...2515971) | yfeA | b2395 | orf, hypothetical protein |
| MDS16 | CDS | complement(379293...380066) | yaiO | b0358 | orf, hypothetical protein |
| MDS16 | CDS | complement(380068 ... 380511) | | b0359 | putative transferase |
| MDS16 | CDS | 380530...380940 | yi21_1 | b0360 | IS2 hypothetical protein |
| MDS16 | CDS | 380898...381803 | yi22_1 | b0361 | IS2 hypothetical protein |
| MDS16 | CDS | complement(381728...382114) | | b0362 | orf, hypothetical protein |
| MDS16 | CDS | complement(381963...383159) | yaiP | b0363 | potysaccharide metabolism |
| MDS16 | CDS | complement(383283...383693) | yaiS | b0364 | orf, hypothetical protein |
| MDS16 | CDS | 384399 ... 385418 | tauA | b0365 | taurine transport system periplasmic protein |
| MDS16 | CDS | 385431...386198 | tauB | 60366 | taurine ATP-binding component of a transport system |
| MDS16 | CDS | 386195...387022 | tauC | b0367 | taurine transport system permease protein |
| MDS16 | CDS | 387019...387870 | tauD | b0368 | taurine dioxygenase, 2-oxoglutarate-dependent |
| MDS17 | CDS | complement(389121...389390) | | b0370 | orf, hypothetical protein |
| MDS17 | CDS | 389475...390935 | yaiT | b0371 | orf, hypothetical protein |
| MDS17 | CDS | complement(390963...391829) | tra5_1 | b0372 | |
| MDS17 | CDS | complement(391826...392134) | | b0373 | putative flagellin structural protein |
| MDS17 | CDS | 392239...393642 | yaiU | b0374 | putative flagellin structural protein |
| MDS17 | CDS | 393685...394353 | yaiV | b0375 | orf, hypothetical protein |
| MDS17 | CDS | complement(394354...395511) | yaiH | b0376 | |
| MDS17 | CDS | 395863...397083 | sbmA | b0377 | sensitivity to microcin B17, possibly mwdapa protein |
| MDS17 | CDS | 397096...398190 | yaiW | b0378 | orf, hypothetical protein |
| MDS17 | CDS | complement(398249...398557) | yaiY | b0379 | orf, hypothetical protein |
| MDS17 | CDS | 398685...399029 | | b0390 | orf, hypothetical protein |
| MDS18 | CDS | complement(2992959...2993114) | | b2856 | orf, hypothetical protein |
| MDS18 | CDS | complement(2993336...2993767) | | b2857 | orf, hypothetical protein |
| MDS18 | CDS | complement(2993770...2993991) | | b2858 | orf, hypothetical protein |
| MDS18 | CDS | complement(2993984...2994409) | | b2859 | orf, hypothetical protein |
| MDS18 | CDS | complement(2994394...2995299) | yi22_4 | b2860 | IS2 hypothetical protein |
| MDS18 | CDS | complement(2995257...2995622) | γi21_4 | b2861 | IS2 hypothetical protein |
| MDS18 | CDS | complement(2995711...2996(10) | | b2862 | orf, hypothetical protein |
| MDS18 | CDS | complement(2996056...2996892) | | b2863 | orf, hypothetical protein |
| MDS19 | CDS | 3182802...3183152 | | b3042 | orf, hypothetical protein |
| MDS19 | CDS | 3183436...3183987 | ygiL | b3043 | putative fimbrial-like protein |
| MDS19 | CDS | 3184209...3184574 | yi21_5 | b3044 | IS2 hypothetical protein |
| MDS19 | CDS | 3184532...3185437 | yi22_5 | b3045 | IS2 hypothetical protein |
| MDS19 | CDS | 3185422...3157887 | yqiG | b3046 | putative membrane protein |
| MDS19 | CDS | 3197894...3188652 | yqiH | b3047 | putative membrane protein |
| MDS19 | CDS | 3188654...3189718 | yqiI | b3048 | orf, hypothetical protein |
| MDS20 | CDS | complement(687220...688236) | trs5_3 | b0656 | IS5 transposase |
| MDS21 | CDS | 1386912...1387919 | ycjG | b1325 | putative muconate cycloisomerase I (EC 5.5 |
| MDS21 | CDS | complement(1387894...1388682) | ycjI | b1326 | putative carboxypeptidase |
| MDS21 | CDS | complement(1388957...1389889) | | bl327 | orf, hypothetical protein |
| MDS21 | CDS | 1390015...1390914 | ycjZ | bl328 | putative transcriptional regulator LYSR-type |
| MDS21 | CDS | 1391230...1392864 | | b1329 | |
| MDS21 | CDS | complement(1392915...1393946) | | b1330 | orf, hypothetical protein |
| MDS21 | CDS | 1394100...1395116 | trs5_4 | b1331 | IS5 transposase |
| MDS21 | CDS | 1395389...1395646 | ynaJ | b1332 | orf, hypothetical protein |
| MDS21 | CDS | complement(1395696...1396646) | ydaA | b1333 | orf, hypothetical protein |
| MDS22 | CDS | complement(2099919...2100935) | trs5_7 | b2030 | |
| MDS22 | CDS | complement(2100940...2101413) | yefJ | b2031 | |
| MDS22 | CDS | complement(2101415...2102533) | wbbK | b2032 | putative glucose transferase |
| MDS22 | CDS | complement(2102519...2103108) | wbbJ | b2033 | putative O-acetyl transferase |
| MDS22 | CDS | complement(2103089...2104081) | wbbI | b2034 | putative Galf transferase |
| MDS22 | CDS | complement(2104084...2105250) | wbbH | b2035 | O-antigen polymerase |
| MDS22 | CDS | complement(2105250...2106353) | glf | b203δ | UDP-galactopyranose mutase |
| MDS22 | CDS | complement(2106361...2107608) | rfbX | b2037 | putative O-antigen transporter |
| MDS22 | CDS | complement(2107605...2108162) | rfbC | b2038 | dTDP-6-deoxy-D-glucase-3,5 epimerase |
| MDS22 | CDS | complement(2108162...2109043) | rfbA | b2039 | glucose-1-phosphate thymidylyltransferase |
| MDS22 | CDS | complement(2109101...2110000) | rfbD | b2040 | dTDP-6-deoxy-L-mannose-dehydrogenase |
| MDS22 | CDS | complement(2110000...2111085) | rfbB | b2041 | dTDP-glucose 4,6 dehydratase |
| MDS22 | CDS | complement(2111458...2112351) | galF | b2042 | homolog of Salmonella UTP-glucose-1-P uridyltransferase, probably a UDP-gal transferase |
| MDS22 | CDS | complement(2112526...2113920) | wcaM | b2043 | orf, hypothetical protein |
| MDS22 | CDS | complement(2113931...2115151) | wcaL | b2044 | putative colanic acid biosynthesis glycosyl transferase |
| MDS22 | CDS | complement(2115148...2116428) | wcaK | b2045 | putative galactokinase (EC 2.7.1.6 |
| MDS22 | CDS | complement(2116704...2118182) | wzxC | b2046 | probable export protein |
| MDS22 | CDS | complement(2118184...2119578) | wcaJ | b2047 | putative colanic acid biosynthsis UDP-glucose lipid carrier transferase |
| MDS22 | CDS | complement(2119633...2121003) | cpsG | b2048 | phosphomannomutase |
| MDS22 | CDS | complement(2121108...2122544) | cpsB | b2049 | mannose-1-phosphate guanyltransferase |
| MDS22 | CDS | complement(2122547...2123770) | wcaI | b2050 | putative aalanio biosynthesis glycosyl transferase |
| MDS22 | CDS | complement(2123767...2124249) | wcaR | b2051 | GDP-mannose mannosyl hydrolase |
| MDS22 | CDS | complement(2124249...2125214) | wcaG | b2052 | putative nucleotide di-P-sugar epimerase or dehydratase |
| MDS22 | CDS | complement(2125217...2126338) | gmd | b2053 | GDP-D-mannose dehydratase |
| MDS22 | CDS | complement(2126364...2126912) | wcaF | b2054 | putative transferase |
| | | | | | |
| MDS22 | CDS | complement(2126928...2127674) | wcaE | b2055 | putative colanic acid biosynthesis glycosyl transferase |
| MDS22 | CDS | complement(2127685...2128902) | wcaD | b2056 | putative colanic acid polymerase |
| MDS22 | CDS | complement(2128977...2130094) | wcaC | b2057 | putative glycosyl transferase |
| MDS22 | CDS | complement(2130091...2130579) | wca8 | b2058 | putative transferase |
| MDS22 | CDS | complement(2130582...2131421) | wcaA | b2059 | putative regulator |
| MDS22 | CDS | complement(2131514...2133712) | | b2060 | |
| MDS22 | CDS | complement(2133679...2134122) | wzb | b2061 | low molecular weight protein-tyrosine-phosphatase |
| MDS22 | CDS | complement(2134128...2135267) | wza | b2062 | putative polysaccharide export protein |
| MDS23 | CDS | complement(2284412...2286922) | yejO | b2190 | putative ATP- binding component of a transport system |
| MDS23 | CDS | 2286927...2287049 | | b2191 | orf, hypothetical protein |
| MDS23 | CDS | complement(2287087...2288103) | trs5_8 | b2192 | IS5 transposase |
| MDS24 | CDS | 3360134...3360808 | yhcA | b3215 | putative chaperone |
| MDS24 | CDS | 3360829...3363210 | yhcD | b3216 | putative outer membrane protein |
| MDS24 | CDS | 3363207...336368δ | yhcE | b3217 | orf, hypothetical protein |
| MDS24 | CDS | complement(3363724...3364740) | trs5_10 | b3218 | IS5 transposase |
| MDS24 | CDS | 3364848...3365664 | yhcF | b3219 | putative transcriptional regulator |
| MDS25 | CDS | 3649314...3650096 | yhiS | b3504 | orf, hypothetical protein |
| MDS25 | CDS | complement(3650205...3651221) | trs5_11 | b3505 | IS5 transposase |
| MDS26 | CDS | complement(1128637...1129053) | flgN | b1070 | protein of flagellar biosynthesis |
| MDS26 | CDS | complement(1129058...1129351) | flgM | b1071 | anti-FliA (anti-sigma) factor; also known as RflB protein |
| MDS26 | CDS | complement(1129427...1130086) | flgA | b1072 | flagellar biosynthesis; assembly of basal-body periplasmic P ring |
| MDS26 | CDS | 1130241...1130657 | flgB | b1073 | flagellar biosynthesis, cell-proximal portion of basal-body rod |
| MDS26 | CDS | 1130661...1131065 | agC | b1074 | flagellar biosynthesis, cell-proximal portion of basal-body rod |
| MDS26 | CDS | 1131077...1131772 | ftgD | b1075 | flagellar biosynthesis, initiation of hook assembly |
| MDS26 | CDS | 1131797...1133005 | flgE | b1076 | flagellar biosynthesis, hook protein |
| MDS26 | CDS | 1133025...1133780 | flgF | b1077 | flagellar biosynthesis, cell-proximal portion of basal-body rod |
| MDS26 | CDS | 1133952...1134734 | flgG | b1078 | flagellar biosynthesis, cell-distal portion of basal-body rod |
| MDS26 | CDS | 1134787...1135485 | flgH | b1079 | ffagellar biosynthesis, basal-body outer-membrane L (lipopolysaccharide layer) ring protein |
| MDS26 | CDS | 1135497...1136594 | flgI | b1080 | homolog of Salmonella P-ring of flagella basal body |
| MDS26 | CDS | 1136594... 1137535 | flgJ | b10B1 | flagellar biosynthesis |
| MDS26 | CDS | 1137601 ... 1139244 | flgK | b10B2 | flagellar biosynthesis, hook-filament junction protein 1 |
| MDS26 | CDS | 1139256...1140209 | flgL | b1083 | flagellar biosynthesis hook-filament junction protein |
| MDS27 | CDS | complement(1960604...1960996) | flhE | b1878 | flagellar protein |
| MDS27 | CDS | complement(1960996...1963074) | flhA | b1879 | flagellar biosynthesis; possible export of flagellar proteins |
| MDS27 | CDS | complement(1963067...1964215) | flhB | b1880 | putative part of export apparatus for flagellar proteins |
| MDS27 | CDS | complement(1964417...1965061) | cheZ | b1881 | chemotactic response; CheY protein phophatase; antagonist of CheY as switch regulator |
| MDS27 | CDS | complement(1965072... 1965461) | cheY | b1882 | chemotaxis regulator transmits chemoreceptor signals to flagelllar motor components |
| MDS27 | CDS | complement(1965476. . . 1966525) | cheB | b1883 | response regulator for chemotaxis (cheA sensor); protein methylesterase |
| MDS27 | CDS | complement(1966528...1967388) | cheR | b1884 | response regulator far chemotaxis protein glutamate methyltransferase |
| MDS27 | CDS | complement(1967407...1969008) | tap | b18B5 | methyl-accepting chemotaxis protein IV peptide sensor receptor |
| MDS27 | CDS | complement(1969054...1970715) | tar | b1886 | methyl-accepting chemotaxis protein II aspartate sensor receptor |
| MDS27 | CDS | complement(1970860...1971363) | cheW | b1887 | positive regulator of CheA protein activity |
| MDS27 | CDS | complement(1971384...1973348) | cheA | b1888 | sensory transducer kinase between chemo-signal receptors and CheB and CheY |
| MDS27 | CDS | complement(1973353...1974219) | motB | b1889 | enables flagellar motor rotation linking torque machinery to cell wall |
| MDS27 | CDS | complement(1974276...1975163) | motA | b1890 | proton conductor component of motor; no effect on switching |
| MDS27 | CDS | complement(1975290...1975868) | flhC | b1891 | regulator of flagellar biosynthesis acting on class 2 operons; transcription initiation factor |
| MDS27 | CDS | complement(1975871...1976230) | flhD | b1892 | regulator of flagellar biosynthesis acting on class 2 operons; transcription initiation factor |
| MDS27 | CDS | complement(1976542...1977045) | insB_5 | b1893 | IS1 protein InsB |
| MDS27 | CDS | complement(1976964...1977239) | insA_5 | b1894 | IS1 protein InsA |
| MDS28 | CDS | complement(1995086...1995838) | yecC | b1917 | putative ATB-binding component of a transport system |
| MDS28 | CDS | complament(1995935...1996503) | yecS | b1918 | putative transport system permease protein (former yecC) |
| MDS28 | CDS | complement(1996518...1997600) | yedO | bl919 | putative 1-aminocyclopropane-1-carbaxylate deaminase |
| MDS28 | CDS | complement(1997609...1998409) | fliY | b1920 | putative periplasmic binding transport protein |
| MDS28 | CDS | complement(1998497... 1999084) | fliZ | b1921 | orf, hypothetical protein |
| MDS28 | CDS | complement(1999094...1999813) | fliA | b1922 | flagellar biosynthesis; alternative sigma factor 28; regulation of flagellar **o**perons |
| MDS28 | CDS | complement(2000134...2001630) | fliC | b1923 | flagellar biosynthesis; flagellin filament structural protein |
| MDS28 | CDS | 2001896... 2003302 | fliD | b1924 | flagellar biosynthesis; filament capping protein; enables filament assembly |
| MDS28 | CDS | 2003327 ... 2003737 | fliS | b1925 | flagellar biosynthesis; repressor of class 3a and 3b operons (RflA activity) |
| MDS28 | CDS | 2003737 ... 2004102 | orf fliT | b1926 | flagellar biosynthesis; repressor of class 3a and 3b operons (RflA activity) |
| MDS28 | CDS | 2004180 ... 2005667 | amyA | b1927 | cytoplasmic alpha-amylase |
| MDS28 | CDS | complement(2005701... 2006114) | yedD | b1928 | orf, hypothetical protein |
| MDS28 | CDS | 2006301...2007506 | yedE | b1929 | putative transport system permease protein |
| MDS28 | CDS | 2007503 ... 2007736 | yedF | b1930 | orf hypothetical protein |
| MDS28 | CDS | 2007845...2008513 | yedK | b1931 | orf, hypothetical protein |
| MDS28 | CDS | 2008624... 2009103 | yodL | b1932 | orf, hypothetical protein |
| MDS28 | CDS | complement(2009372... 2009563) | | b1933 | orf, hypothetical protein |
| MDS28 | CDS | complement(2009573... 2009893) | yedN | b1934 | orf, hypothetical protein |
| MDS28 | CDS | complement(2010025... 2010375) | yedM | b1935 | orf, hypothetical protein |
| MDS28 | CDS | 2010526... 2010804 | | b1936 | orf, hypothetical protein |
| MDS28 | CDS | complement(2010724... 2011038) | fliE | b1937 | flagellar biosynthesis; basal-body component, possibly at (MS-ring)-rod junction |
| MDS28 | CDS | 2011253 ... 2012911 | fliF | b1938 | flagellar biosynthesis; basal-body MS(membrane and supramembrane)-ring and collar protein |
| MDS28 | CDS | 2012904... 2013899 | fliG | b1939 | flagellar biosynthesis, component of motor switching and energizing, enabling rotation and determinin its direction |
| MDS28 | CDS | 2013871... 2014578 | fliH | b1940 | flagellar biosynthesis; export of |
| MDS28 | CDS | 2014578 .. .2015951 | fliI | b1941 | flagellum-specific ATP synthase |
| MDS28 | CDS | 2015970... 2016413 | fliJ | b1942 | flagellar fliJ protein |
| MDS28 | CDS | 2016410 ... 2017537 | flik | b1943 | flagellar hook-length control protein |
| MDS28 | CDS | 2017642 ... 2018106 | fliL | b1944 | flaggelar bioxynsthesis |
| MDS28 | CDS | 2018111 ... 2019115 | fliM | b1945 | flagellar biosynthesis, component of motor switch and energizing, enabling rotation and determining its direction |
| MDS28 | CDS | 2019112 ... 2019525 | fliN | b1946 | flagellar biosynthesis, component of motor switch and energizing, enabling rotation and determining its direction |
| MDS28 | CDS | 2019388...2019893 | fliO | b1947 | flagellar biosynthesis |
| MDS28 | CDS | 2019893 ... 2020630 | fliP | b1948 | flaggellar biosynthesis |
| MDS28 | CDS | 2020640 ... 2020909 | fliQ | b1949 | flaggellar biosynthesis |
| MDS28 | CDS | 2020917... 2021702 | fliR | b1950 | flagellar biosynthesis |
| MDS29 | CDS | 4552599... 4553372 | uxuR | b4324 | regulator for oxu operon |
| MDS29 | CDS | complement(4553513...4554343) | yjiC | b4325 | orf, hypothetical protein |
| MDS29 | CDS | 4555007... 4555448 | yjiD | b4326 | orf, hypothetical protein |
| MDS29 | CDS | comptement(4555401... 4556312) | yjiE | b4327 | putative transcriptional regulator LYSR-type |
| MDS29 | CDS | comptement(4536377... 4557549) | iadA | b4328 | isoaspartyl dipeptidase |
| MDS29 | CDS | complement(4557562...4558023) | yjiG | b4329 | orf hypothetical protein |
| MDS29 | CDS | complement(4558020...4558715) | yjiH | b4330 | orf, hypothetical protein |
| MDS29 | CDS | 4558851... 4559507 | yjiI | b4331 | orf, hypothetical protein |
| MDS29 | CDS | complement(4559520...4560698) | yjiJ | b4332 | putative transport protein |
| MDS29 | CDS | complement(4560766...4561737) | yjiK | b4333 | orf, hypothetical protein |
| MDS29 | CDS | complement(4561945... 4562718) | yjiL | b4334 | putative enzyme |
| MDS29 | CDS | complement(4562722... 4563894) | yjiM | b4335 | orf, hypothetical protein |
| MDS29 | CDS | complement(4563989...4565269) | yjin | b4336 | orf, hypothetical protein |
| MDS29 | CDS | complement(4565310...4566542) | yjiO | b4337 | putative transport protein |
| MDS29 | CDS | 4567021...4567332 | yjiP | b4338 | orf, hypothetical protein |
| MDS29 | CDS | 4567381 ... 4567941 | yjiQ | b4339 | orf, hypothetical protein |
| MDS29 | CDS | complement(4568185... 4569597) | yjiR | b4340 | putative regulator |
| MDS29 | CDS | 4569774...4569938 | yjiS | b4341 | orf, hypothetical protein |
| MDS29 | CDS | 4570389... 4571954 | yjiT | b4342 | orf, hypothetical protein |
| MDS29 | CDS | complement(4574935... 4575981) | mcrC | b4345 | component of McrBC 5-methylcytosine restriction system |
| MDS29 | CDS | complement(4575981... 4577378) | mcrB | b4346 | complement of McrBC 5-methilcytosine restriction system |
| MDS29 | CDS | complement(4571522... 4577920) | yjiW | b4347 | orf, hypothetical protein |
| MDS29 | CDS | complement(4578091... 4579485) | hsdS | b4348 | specificity determinant for hsdM and hsdR |
| MDS29 | CDS | complement(4579482...4581071) | hsdM | b4349 | host modification; DNA methylase M |
| MDS29 | CDS | complement(4581272... 4584838) | hsdR | b4350 | host restriction; endonuclease R |
| MDS29 | CDS | 4584972... 4585886 | mrr | b4351 | restriction of methylated adenine |
| MDS29 | CDS | complement(4585932... 4586786) | yjiA | b4352 | orf, hypothetical protein |
| MDS29 | CDS | complement(4586899... 4587102) | yjiX | b4353 | orf, hypothetical protein |
| MDS29 | CDS | complement(4587152... 4589317) | yjiY | b4354 | putative carbon starvation protein |
| MDS29 | CDS | 4589680... 4591335 | tsr | b4355 | methyl-accepting chemotaxis protein I serine sensor receptor |
| MDS29 | CDS | complement(4591384...4592745) | yjiZ | b4356 | putative transport protein, cryptic orf, joins former yjiZ and yjjL |
| MDS29 | CDS | complement(4592960...4593874) | yjjM | b4357 | orf, hypothetical protein |
| MDS29 | CDS | 4593998 ... 4595035 | yjjN | b4358 | putative oxidoreductase |
| MDS29 | CDS | 4572158 ... 4574878 | yjiV | b4486 | conserved hypothetical protein |
| MDS30 | CDS | 522485 ... 526765 | rhsD | b0497 | rhsD protein in rhs clement |
| MDS30 | CDS | 526805 ...527173 | | b0498 | orf, hypothetical protein |
| MDS30 | CDS | 527173 ... 527883 | | b0499 | orf, hypothetical protein |
| MDS30 | CDS | 527864 ... 528124 | ybbD | b0500 | orf, hypothetical protein |
| MDS30 | CDS | 528163 ... 528354 | | b0501 | orf, hypothetical protein |
| MDS30 | CDS | complement(528869... 529276) | | b0502 | orf, hypothetical protein |
| MDS31 | CDS | 728806 ... 732999 | rhsC | b0700 | |
| MDS31 | CDS | 732593 ... 732814 | | b0701 | |
| MDS31 | CDS | 732999 ... 733325 | ybfB | b0702 | orf, hypothetical protein |
| MDS31 | CDS | 733443 ... 734876 | | b0703 | orf, hypothetical protein |
| MDS31 | CDS | 734873 ... 735442 | ybfC | b0704 | orf, hypothetical protein |
| MDS31 | CDS | 136327 ... 737184 | ybfL | b0705 | putative receptor protein |
| MDS31 | CDS | 737315 ... 738076 | ybfD | b0706 | putative DNA ligase |
| MDS32 | CDS | 1525914... 1527962 | rhsE | bl456 | |
| MDS32 | CDS | 1527946 ... 1528428 | ydcD | b1457 | orf, hypothetical protein |
| MDS32 | CDS | 1528610 ... 1529356 | | b1458 | orf, hypothetical protein |
| MDS32 | CDS | 1529400... 1529600 | | b1459 | orf, hypothetical protein |
| MDS32 | CDS | 1529840...1530976 | ydcC | b1460 | H repeat- associated protein (ORF-H) |
| MDS32 | CDS | 1531076... 1531309 | ydcE | b1461 | orf, hypothetical protein |
| MDS32 | CDS | complement(1531306...1531923) | | b1462 | orf, hypothetical protein |
| MDS33 | CDS | 3616611...3617012 | yhhG | b3481 | |
| MDS33 | CDS | 3617215 ... 3621450 | thsB | b3482 | rhsB protein in rhs element |
| MDS33 | CDS | 3621437 ... 3621805 | yhhH | b3483 | orf, hypothetical protein |
| MDS33 | CDS | 3622401 ... 3623537 | yhhI | b3484 | putative receptor |
| MDS34 | CDS | complement(3759370... 3759978) | yibF | b3592 | putative S-transferase |
| MDS34 | CDS | 3760206 ... 3764339 | rhsA | b3593 | rhsA protein in rhs element |
| MDS34 | CDS | 3764360... 3765202 | yibA | b3594 | orf, hypothetical protein |
| MDS34 | CDS | 3765244... 3765945 | yibJ | b3595 | orf, hypothetical protein |
| MDS34 | CDS | 3766200... 3766661 | yibG | b3596 | orf, hypothetical protein |
| MDS35 | CDS | complement(1041253...1043433) | yccC | b0981 | orf, hypothetical protein |
| MDS35 | CDS | complement(1043453...1043911) | yccY | b0982 | yccY putative phosphatase |
| MDS35 | CDS | complement(1043987... 1045026) | yccZ | b0983 | putative function in exopolysaccharide production |
| MDS35 | CDS | complement(1045072... 1047168) | ymcA | b0984 | orf, hypothetical protein |
| MDS35 | CDS | complement(1047168...1047914) | ymcB | b0985 | orf, hypothetical protein |
| MDS35 | CDS | complammd(1047911...1048555) | ymcC | b0986 | putative regulator |
| MDS35 | CDS | complcmcnt(1048662 ...1048985) | ymcD | b0987 | orf, hypothetical protein |
| MDS35 | CDS | 1049250 ... 1049753 | insB_4 | b0988 | IS1 protein ImB |
| MDS36 | CDS | complement(1085329...1085742) | ycdP | b1021 | orf, hypothetical protein |
| MDS36 | CDS | complement(1095744...1087069) | yodQ | b1022 | orf, hypothetical protein |
| MDS36 | CDS | complement(1087062...1089080) | ycdR | b1023 | orf, hypothetical protein |
| MDS36 | CDS | complement(1089089...1091512) | ycdS | b1024 | putative outer membrane protein |
| MDS36 | CDS | 1092099 ... 1093457 | ycdT | b1025 | orf, hypothetical protein |
| MDS36 | CDS | complement(1093498...1(94364) | tra5_3 | b1026 | |
| MDS36 | CDS | complement(1094361... 1094669) | | b1027 | |
| MDS36 | CDS | 1094746...1095069 | | b1028 | orf, hypothetical protein |
| MDS36 | CDS | 1095066... 1096052 | ycdU | b1029 | orf, hypothetical protein |
| MDS37 | CDS | 2163174... 2163545 | | b2080 | orf, hypothetical protein |
| MDS37 | CDS | 2163692... 2165053 | yegQ | b2081 | orf, hypothetical protein |
| MDS37 | CDS | complement(2165326... 2165544) | ogrK | b2082 | prophage P2 ogr protein |
| MDS37 | CDS | complement(2165626... 2165772) | | b2083 | orf, hypothetical protein |
| MDS37 | CDS | complement(2165759... 2166025) | | b2084 | orf, hypothetical protein |
| MDS37 | CDS | complement(2166013...2166390) | yegR | b2085 | orf, hypothetical protein |
| MDS37 | CDS | 2166736 ... 2167635 | | b2086 | orf, hypothetical protein |
| MDS37 | CDS | complement(2167717...2168163) | gatR_1 | b2087 | split galactitol utilization operon repressor |
| MDS37 | CDS | 2168251 ... 2168559 | | b2088 | |
| MDS37 | CDS | 2168556...2169422 | tra5_4 | b2089 | |
| MDS37 | CDS | complement(2169419...2169757) | gatR_2 | b2090 | |
| MDS37 | CDS | complement(2169857... 2170897) | gatD | b2091 | galactitol-1-phosphate dehydrogenase |
| MDS37 | CDS | complement(2170945... 2172300) | gatC | b2092 | PTS system galactitol-specific enzyme IIC |
| MDS37 | CDS | complement(2172304...2172588) | gatB | b2093 | galactitol-specific enzyme IIB of phosphotransferase system |
| MDS37 | CDS | complement(2172619... 2173071) | gatA | b2094 | galactitol-specific enzyme IIA of phosphotransferase system |
| MDS37 | CDS | complement(2173081... 2174343) | gatZ | b2095 | putative tagatose 6-phosphate kinase 1 |
| MDS37 | CDS | complement(2174372... 2175232) | gatY | b2096 | tagatose-biphosphate aldolase 1 |
| MDS37 | misc_RNA | 2165136 ... 2165221 | ryeE | b4438 | |
| MDS38 | CDS | complement(3577791 ... 3578828) | yhhX | b3440 | putative regulator |
| MDS38 | CDS | 3379161 ... 3379649 | yhhY | b3441 | orf, hypothetical protein |
| MDS38 | CDS | 3579886 ... 3581064 | yhhZ | b3442 | orf, hypothetical protein |
| MDS38 | CDS | 3581061 ... 3581477 | yrhA | b3443 | orf, hypothetical protein |
| MDS38 | CDS | 3581506 ... 3581781 | insA_6 | b3444 | IS1 protein Im A |
| MDS38 | CDS | 3581700 ... 3582203 | insB_6 | b3445 | IS1 protein InsB |
| MDS38 | misc_RNA | complement(3578946... 3579039) | ryhB | b4451 | regulatory RNA mediating Fur regulon |
| MDS39 | CDS | 3718072...3718284 | cspA | b3556 | cold shock protein 7.4, transcriptional activator of hns |
| MDS39 | CDS | 3718703 ...3719224 | yi5A | b3557 | IS150 hypothetical protein |
| MDS39 | CDS | 3719221 ... 3720072 | t150 | b3558 | IS150 putative transposase |
| MDS39 | CDS | complement(3718471... 3718623) | hoka | b4455 | small toxic membrane polypeptide |
| MDS40 | CDS | 1869885... 1871555 | yeaJ | b1786 | orf, hypothetical protein |
| MDS41 | CDS | 167494 ...169727 | fhuA | b0150 | cuter membrane protein receptor for ferrichrome, colicin M, and phages T1, T5, and phi80 |
| MDS41 | CDS | 169778...170575 | fhuC | b0151 | ATP-binding component of hydroxymate-dependent iron transport |
| MDS41 | CDS | 170575 ...171465 | fhuD | b0152 | hydroxamate-dependent iron uptake cytoplasmic membrane component |
| MDS41 | CDS | 171462 ...173444 | fhuB | b0153 | hydroxamate-dependent iron uptake cytoplasmic membrane component |

A reduced genome *E*. *coli* K-12 strain lacks at least the following genes (identified by "b" numbers based on the designations set out in Blattner et al., Science, 277:1453-74 and in GenBank Accession No. 400096): b0245-b0301, b0303-b0310, b1336-b1411, b4426-b4427, b2441-b2450, b2622-b2654, b2657-b2660, b4462, b1994-b2008, b4435, b3322-b3338, b2349-b2363, b1539-b1579, b4269-b4320, b2968-b2972, b2975-b2977, b2979-b2987, b4466-b4468, b1137-b1172, b0537-b0565, b0016-b0022, b4412-b4413, b0577-b0582, b4415, b2389-b2390, b2392-b2395, b0358-b0368, b0370-b0380, b2856-b2863, b3042-b3048, b0656, b1325-b1333, b2030-b2062, b2190-b2192, b3215-b3219, b3504-b3505, b1070-b1083, b1878-b1894, b1917-b1950, b4324-b4342, b4345-b4358, b4486, b0497-b0502, b0700-b0706, b1456-b1462, b3481-b3484, b3592-b3596, b0981-b0988, b1021-b1029, b2080-b2096, b4438, b3440-b3445, b4451, b3556-b3558, and b4455, which are the genes deleted from *E*. *coli* K-12 MG1655 to create reduced genome (or multiple deletion) strain MDS39. The reduced genome *E. coli* K-12 strain further lacks the following gene: b1786, which is the gene deleted from MDS39 to create reduced genome strain MDS40. The reduced genome *E*. *coli* K-12 strain further lacks the following genes: b0150-b01530, which are the genes deleted from MDS40 to create MDS41. The reduced genome *E*. *coli* K-12 strain further lacks the following gene: b2945 (*endA*) which is the gene deleted from MDS41 to create reduced genome strain MDS42. In still another embodiment, the reduced genome *E*. *coli* K-12 strain further lacks any of the following genes: b0315-b0331, b0333-b0341 and b0346-b0354, which are the genes deleted from MDS42 to create reduced genome strain MDS43. In yet another embodiment, the reduced genome *E*. *coli* K-12 strain further lacks any of the following genes : b2481-b2492, b2219-b2230, b4500, b3707-b3723, b0644-b0650, b4079-4090, b4487, b4092-b4106, b0730-b0732, b3572-b3587, b1653, b2735-b2740, b2405-b2407, b3896-b3900, b1202, b4263-b4268, b0611, b2364-b2366, b0839, b0488-b0500, b0502, which are the genes deleted from MDS43 to create MDS60. In yet another preferred embodiment, the reduced genome *E*. *coli* K-12 strain further lacks any of the following genes: b0566-b0575, b2209, b0160-b0161, b1431-b1444, b3643, b1037-b1043, b0383, b0226-b0234, b2115-b2132, which are the genes deleted from MDS60 to create MDS69. In certain embodiments, the reduced genome *E. coli* K-12 strain for use in the methods described herein is MDS41, MDS42, MDS60 or MDS69.

*E. coli* host cells for use in the present invention preferably comprise a functional *recA* gene (b2699), although *E*. *coli* lacking a functional *recA* gene (b2699) can also be used as a host cell for producing CRM197. For example, a reduced genome *E*. *coli* strain such as e.g. strain MDS40, MDS41, MDS42 or MDS69 can be modified by inactivation of b2699 by complete or partial deletion of the gene from the modified *E*. *coli* K-12 strain. In one embodiment, CRM197 fused to an OmpA signal sequence is expressed in a reduced genome *E*. *coli* host lacking a functional *recA* gene.

In another aspect, the reduced genome *E*. *coli* comprises one or more non-functional genes selected from the group consisting of the genes encoding Pol II, Pol IV and Pol V, as described in WIPO Publication No. 2013/059595. In one embodiment, the reduced genome *E*. *coli* has non-functional PolB (encoded by b0060, coordinates 63429-65780 on the *E*. *coli* K12 MG1655 genome) and DinB (encoded by b0231, coordinates 250898-251953 on the MG1655 genome) genes. In another embodiment, the reduced genome *E*. *coli* has non-functional PoIB, DinB and UmuDC (encoded by b1183-b1184, coordinates 1229990-1231667 on the MG1655 genome) genes. Preferably, the gene(s) are rendered inactive by complete or partial deletion. For example, the *polB, dinB* and *umuDC* genes may be rendered nonfunctional in a reduced genome *E. coli* strain such as strain MDS40, MDS41, MDS42 or MDS69.

In another aspect, the reduced genome *E*. *coli* (e.g. strain MDS40, MDS41, MDS42 or MDS69) has been genetically modified so as to (a) enhance orotate phosphoribosyltransferase activity (b) produce active acetohydroxy acid synthase II and (c) reduce expression of the *iclR* and *arpA* gene products.

*E. coli* orotate phosphoribosyltransferase, an enzyme that catalyzes synthesis of pyrimidine nucleotides, is encoded by the *pyrE* gene, b-number b3642. The *pyrE* gene is present in an operon with the upstream *rph* gene (b3643). The *pyrE* gene is expressed at sub-optimal levels in *E. coli* K-12 strains such as MG1655 and W3310 due to a -1 frame shift mutation in the coding region of the *rph* gene. Orotate phosphoribosyltransferase activity can be enhanced by a deletion that entirely removes the *rph* coding sequence to bring the promoter of the *rph-pyrE* operon closer to the translation initiation site of *pyrE.* Alternatively, any of the methods described in U.S. Patent No. 8,293,505 can be used to enhance orotate phosphoribosyltransferase activity.

*E. coli* acetohydroxy acid synthase II normally consists of a large subunit, encoded by the *ilvG* gene and a small subunit, encoded by the *ilvM* gene (b3769). The *ilvG* sequence of *E*. *coli* K-12 strain MG1655 is corrupted and is actually a pseudo gene (b-number b4488), as set forth in GenBank Accession No. AAC77488.1. The *ilvG* pseudo gene is comprised of two separate coding sequences, *ilvG_1* (b3767) and *ilvG_2* (b3768). The *ilvG* pseudo gene sequence in K-12 strains such as MG1655 comprises a deletion of nucleotides GT at positions 983 and 984 relative to the intact *ilvG* genes found in other *E*. *coli* strains (e.g. B strain, O strain, etc.). The deletion of these nucleotides results in a frameshift mutation and nucleotides TGA at positions 982-984 of the K-12 *ilvG* pseudo gene sequence serve as a premature termination codon resulting in a truncated form of *ilvG* corresponding to *ilvG_1.* Thus, the normal gene product of *ilvG* is not expressed and acetohydroxy acid synthase II is not present in *E. coli* K-12 strains. The reduced genome *E. coli* can be modified to produce active acetohydroxy acid synthase II by the introduction of a mutation which complements a native -2 frameshift mutation in the *ilvG* gene. Alternatively, the reduced genome *E. coli* can be modified to produce active acetohydroxy acid synthase II by any of the methods of U.S. Patent No. 7,300,776.

The *iclR* and *arpA* genes of *E. coli* K strain are adjacent genes encoding regulatory proteins that modulate expression of the glyoxylate shunt enzymes and of acetyl-CoA synthetase, respectively. The *iclR* (isocitrate lyase regulator) gene, b-number b4018, is described at NCBI Entrez GeneID No. 948524. The *arpA* (ankyrin-like regulator protein) gene, b-number b4017, is described at NCBI Entrez GeneID No. 944933. The *arpA* gene was found to be partially deleted in the genome sequence of B strains such as BL21DE3 and REL606 relative to the K-12 strain sequence. The *iclR* and *arpA* genes can be inactivated (i.e. rendered non-functional) in the reduced genome *E. coli* by deletion of all or part of the *iclR* and *arpA* gene sequences for example by the "scarless" deletion methods described at column 8, line 45 to column 14, line 41 of U.S. Patent No. 6,989,265.

In other embodiments, the reduced genome *E. coli* comprises a *relA* gene containing any of the mutations described in U.S. Patent No. 8,367,380. For example, a reduced genome *E*. *coli* strain such as strain MDS40, MDS41, MDS42 or MDS69 may be modified to incorporate any of these mutations.

Reduced genome *E. coli* for use according to the disclosure may comprise any combination of the modifications described above. In some preferred embodiments, a reduced genome *E. coli* comprising at least the deletions of MDS42 or comprises at least the deletions of MDS69 and has been genetically modified so as to (a) enhance orotate phosphoribosyltransferase activity (b) produce active acetohydroxy acid synthase II and (c) reduce expression of the *iclR* and *arpA* gene products is employed as a host for periplasmic production of CRM197. The reduced genome *E. coli* preferably comprises a functional *recA* gene.

Various protein coding genes can be deleted to form reduced genome bacteria as claimed. In *E*. *coli* and other bacteria, a type of DNA sequence that can be deleted includes those that in general will adversely affect the stability of the organism or of the gene products of that organism. Such elements that give rise to instability include without limitation transposable elements, insertion sequences, and other "selfish DNA" elements which may play a role in genome instability. For example, insertion sequence (IS) elements and their associated transposes are often found in bacterial genomes, and thus are targets for deletion. IS sequences are common in *E. coli,* and all of them may be deleted. For purposes of clarity in this document, we use the term IS element and transposable element generically to refer to DNA elements, whether intact or defective, that can move from one point to another in the genome. An example of the detrimental effects of IS elements in science and technology is the fact that they can hop from the genome of the host *E*. *coli* into a plasmid during propagation for sequencing. This artifact can be prevented by deletion from the host cells of all IS elements. For a specific application, other specific genes associated with genomic instability, such as active and inactive prophages may also be deleted. In particularly preferred embodiments, the reduced genome *E*. *coli* host according to the invention has deleted therefrom all insertion sequences (i.e. does not comprise insertion sequences). In a related aspect, the reduced genome *E*. *coli* host lacks all IS1, IS2, IS3, IS5, IS150 and IS186 insertion sequences.

Reduced genome bacteria of the invention may also be engineered to lack, for example, without limitation, certain genes unnecessary for growth and metabolism of the bacteria, pseudo genes, prophage, undesirable endogenous restriction-modification genes, pathogenicity genes, toxin genes, fimbrial genes, periplasmic protein genes, invasin genes, lipopolysaccharide genes, class III secretion systems, phage virulence determinants, phage receptors, pathogenicity islands, RHS elements, sequences of unknown function and sequences not found in common between two strains of the same native parental species of bacterium. Other DNA sequences that are not required for cell survival can also be deleted or omitted.

In a particularly preferred embodiment of the disclosure, a reduced genome *E*. *coli* is provided having a genome between five percent (5%) and thirty percent (30%) smaller than the genome of a native parent *E*. *coli* K strain and lacking all insertion sequence (IS) elements. Positions of the IS elements on a genome map of *E. coli* MG1655 are shown in Fig. 1 and Table 2 of U.S. Patent No. 8,178,339 (Table 6). Insertion sequence elements which commonly occur in *E. coli* and which may be removed, include without limitation, IS1, IS2, IS3, IS4, IS5, IS30, IS150, IS186, IS600, IS911 and IS10. Preferably, the native parent *E*. *coli* strain is *E*. *coli* K-12 strain MG1655.

A reduced genome *E. coli* described herein may comprise deletion(s) of one or more periplasmic protein genes, including without limitation, the following genes alone or in any combination: b0018, b0150, b0152-b0153, b0161, b0227, b0250, b0291-b0293, b0297, b0316, b0329, b0365, b0371, b0376, b0383-b0384, b0494, b0497-b0498, b0545, b0553, b0559, b0562, b0565, b0567, b0569, b0572-b0574, b0611, b0700, b0704, b0839, b0983-b0986, b1023-b1024, b1072, b1079-b1080, b1083, b1038-b1039, b1041-b1043, b1329, b1357, b1369, b1377, b1383, b1386, b1435-b1436, b1440, b1562, b1878, b1889, b1920, b1995, b2000, b2062, b2123, b2126, b2131-b2132, b2190, b2209, b2487, b2637, b2647, b2945, b3043, b3046-b3048, b3215-b3216, b3219, b3325, b3329, b3338, b3482, b3579, b3584, b3586, b3593, b3596, b4080, b4088, b4105, b4280, b4290-b4292, b4309-b4311, b4314, b4316-b4320, b4412, b4415, b4455, and b4487.

A parent strain as defined in the claims such as K-12 MG1655 is used to produce recombinant CRM197 according to the methods herein described.

The recombinant protein may be co-expressed with chaperones/disulfide-bond forming enzymes, which may provide proper folding of the recombinant protein, including but not limited to Skp, DnaK, DnaJ, CaflM, CaflA, DsbA, DsbB, DsbC, DsbD, PpiA, PpiD, FkpA, SurA, MBP, GST, YebF, MalE, HlyA, Hirudin, OmpF, Spy, YccA; and PspA. Nucleic acid sequences of such proteins useful for periplasmic expression of recombinant protein include, without limitation, those described in U.S. Pat. Nos. 5,747,662; 5,578,464 and 6,022,952.

*E. coli* host cells as claimed transformed with an expression vector encoding CRM197 can be cultured in any fermentation format. For example, shake flask cultures, batch, fed-batch, semi-continuous and continuous fermentation modes may be used herein. As used herein "fermentation" includes both embodiments in which literal fermentation is employed and embodiments in which other non-fermentative culture modes are employed. Further, any scale of fermentation may be employed including 1 liter scale and larger fermentation volumes. In one embodiment, the fermentation volume is or is at least 1 Liter. In other embodiments, the fermentation volume is or is at least 5 Liters, 10 Liters, 15 Liters, 20 Liters, 25 Liters, 50 Liters, 75 Liters, 100 Liters, 200 Liters, 500 Liters, 1,000 Liters, 5,000 Liters, 10,000 Liters, 50,000 Liters, or more.

In various embodiments, fermentation medium may be selected from among rich media, minimal media and mineral salts media. In preferred embodiments, a minimal medium or mineral salts medium is selected. The media is preferably free or substantially free of serum and animal-derived products. A mineral salts medium typically consists of mineral salts and a carbon source (e.g. glucose, sucrose, or glycerol). The mineral salts used to make mineral salts media include those selected from among, e.g., potassium phosphates, ammonium sulfate or chloride, magnesium sulfate or chloride, and trace minerals such as calcium chloride, borate, and sulfates of iron, copper, manganese, and zinc. No organic nitrogen source, such as peptone, tryptone, amino acids, or a yeast extract, is included in a mineral salts medium. Instead, an inorganic nitrogen source is used and this may be selected from among, e.g., ammonium salts, aqueous ammonia, and gaseous ammonia. A preferred mineral salts medium will contain glucose as the carbon source. In comparison to mineral salts media, minimal media can also contain mineral salts and a carbon source, but can be supplemented with, e.g., low levels of amino acids, vitamins, peptones, or other ingredients, though these are added at very minimal levels.

In embodiments, a target culture cell density is reached at which time an inducer, preferably IPTG, is added to initiate protein production. It is understood that the cell density at induction, the concentration of inducer, pH and temperature can be varied to determine optimal conditions for expression

In preferred embodiments, the pH of the culture is from about 6.5 to 7.5.

Growth, culturing and/or fermentation of the transformed reduced genome *E*. *coli* is performed within a temperature range permitting survival but is preferably from about 20 °C to about 30 °C, more preferably is about 25 °C. In another preferred embodiment, the culturing comprises a relatively short initial incubation at 37 °C (e.g. 1 to 3 hours) and is followed by growth at about 20 °C to about 30 °C, preferably about 25 °C prior to and subsequent to induction. In other embodiments, culturing comprises growth at about 25 °C prior to and subsequent to induction.

In embodiments, under shake flask conditions, inducer is added at an optical density (OD) at 600 nm of about 0.1 to about 1.5, more preferably about 0.2 to about 0.9, even more preferably about 0.3 to about 0.6) at an incubation temperature of 20-30 °C, preferably 25 °C. At 600 nm, 1 OD unit corresponds to about 0.8 x 10⁹ cells/ml. In other embodiments, under fermentation conditions, inducer is added at an OD600 of about 100 to 400, more preferably about 150 to 300, most preferably between 230 and 250.

The present methods provide for an increase in the level of properly processed CRM197 in comparison with conventional expression systems, such as in wild type *E*. *coli* B strains. In certain embodiments, the methods provide for an increase in soluble CRM197. In this context, the term "soluble" means that the protein is not precipitated at centrifugation between approximately 5,000 and 20,000 x gravity when spun for 10-30 minutes in a buffer under physiological conditions. Conversely, "insoluble" means that the protein can be precipitated by centrifugation at between 5,000 and 20,000 x gravity when spun for 10-30 minutes in a buffer under physiological conditions.

The methods of the present invention can comprise recovery of recombinant CRM197 produced from the (e.g. reduced genome) *E*. *coli* host cells. When produced in the periplasm as a soluble protein, the recovery of recombinant CRM197 in soluble form is preferably accomplished by mechanically lysing the *E. coli* host cells in the absence of detergents and solubilizers. Mechanical disruption typically involves sonication (Neppiras and Hughes, Biotechnology and Bioengineering, 6:247-270 (1964)), microfluidization (Sauer et al., Biotechnology and Bioengineering, 33:1330-1342 (1989)), or bead milling (Limon-Lason et al., Biotechnology and Bioengineering, 21(5):745-774 (1979)). Other mechanical methods known in the art may also be employed.

Recombinant CRM197 may be purified by standard techniques known in the art including, but not limited to, ammonium sulfate or ethanol precipitation, acid extraction, anion or cation exchange chromatography, phosphocellulose chromatography, hydrophobic interaction chromatography, affinity chromatography, hydroxyapatite chromatography, immunopurification methods and the like. In a preferred embodiment, purification of recombinant CRM197 comprises hydrophobic interaction chromatography and/or anion exchange chromatography.

The yield of CRM197 can be determined by methods known to those skilled in the art such as capillary gel electrophoresis and Western blot analysis. Activity assays can also provide information regarding protein yield. Useful measures of protein yield include the amount of recombinant protein per culture volume (e.g. grams of protein /liter of culture), percent or fraction of active protein (e.g. amount of active protein/amount of protein used in the assay), percent or fraction of total cell protein, amount of protein/cell and percent or proportion of dry biomass.

Activity assays for evaluating CRM197 are known in the art and described in the literature and may include immunological assays, e.g. Western Blot analysis and ELISA, as well as receptor binding assays, e.g. Diphtheria toxin receptor (proHB-EGF) binding. In one embodiment, activity is represented by the % active recombinant CRM197 protein in the extract supernatant as compared with the total amount assayed (i.e. based on the amount of CRM197 determined to be active by the assay relative to the total amount of CRM197 used in the assay). In another embodiment, activity is represented by the % active recombinant CRM197 protein in the extract supernatant compared to a standard e.g. native protein (i.e. based on the amount of active CRM197 protein in the supernatant extract sample relative to the amount of active protein in a standard sample where the same amount of protein from each sample is used in the assay). In embodiments, about 60% to about 100%, about 70% to about 100%, about 80% to about 100%, about 90% to about 100%, about 95% to about 100%, or about 99% to 100% of the recombinant CRM197 protein is determined to be active.

Means of confirming the identity of CRM197 are also known in the art, e.g. a protein can be analyzed by peptide mass fingerprint using MALDI-TOF mass spectrometry, N-terminal sequencing analysis or peptide mapping.

The following are among preferred embodiments of the invention

A method for producing a recombinant CRM197 of claim 1
whereby a yield of at least 1 gram, preferably at least 2 grams per liter of soluble CRM197 is obtained and wherein the incubation conditions comprise culturing the *E. coli* host cell in a minimal medium free of animal serum or other animal by-products.

A method for producing a recombinant CRM197 of claim 1
wherein the incubation conditions comprise culturing the *E. coli* host cell in a minimal medium free of animal serum or other animal by-products.

A method for producing a recombinant CRM197 of claim 1
whereby a yield of at least 1 gram, preferably at least 2 grams per liter of soluble CRM197 is obtained,
wherein the incubation conditions comprise culturing the *E*. *coli* host cell in a minimal medium free of animal serum or other animal by-products.

### Example 1

### Cytoplasmic Expression of Insoluble CRM197 in Reduced Genome E. coli Hosts

CRM197 is currently manufactured by fermentation of *Corynebacterium diphtheriae C7,* where it is expressed from multiple lysogens of the β phage, or from a recombinant plasmid system in *Pseudomonas fluorescens.* The yield of CRM197 in *C*. *diphtheriae* is low (at most ~200 mg/L) and requires biosafety level 2 (BSL2) facilities. Production in *P. flurescens* results in a higher yield (about 2 g/L); however, both hosts retain numerous mobile elements, cyrptic prophages and gene remnants with pathogenic functions. In bacterial fermentations, mobility of insertion sequence (IS) elements can lead to insertions that inactivate the gene of interest. The end result can be fermentation failure or the unwanted expression of a truncated product, both of which are economically problematic and potentially dangerous. In addition, reversion of CRM197 into its toxic parent could have disastrous consequences. Reversion of CRM197 may have contributed to toxic activity that was detected in tissue culture cells (Qiao et al., 2008). Thus, expressions systems with reduced mutation rates may provide the highest reliability and productivity coupled with the lowest level of risk for reversion.

The single greatest factor contributing to the high price and short supply of CRM197 is the historical inability to generate high amounts of CRM197 in the production workhorse *E*. *coli.* CRM197 is insoluble when expressed in the cytoplasm of bacteria and requires re-folding prior to use when made in standard commercial *E*. *coli* strains. Since relatively low amounts of CRM197 are produced in conventional strains, a reduced genome *E*. *coli* strain, MDS42, was tested as a production host for insoluble CRM197 in shake flask culture.

Reduced genome strain MDS42 was produced by the methods described in International Patent Publication No. WO 2003/070880. Briefly, a series of reduced genome strains (MDS01-MDS39), were produced by making a series of 39 cumulative deletions (approximately 14.1% of the genome) of nucleic acid sequences from the parental strain *E*. *coli* MG1655. Hybridization to genome scanning chips (NimbleGen Systems, Madison, WI) containing the K-12 sequence and all sequences in the IS database revealed that MDS39, the first strain designed to lack all IS elements, unexpectedly contained additional copies of an IS element that had translocated to new locations during its production. These IS elements were deleted to produce MDS40. The *fhuACDB* (the *tonA* locus) was deleted from MDS40 to produce MDS41. The location and function of each cumulative deletion made to produce MDS01-MDS41 can be found at Table 2 of U.S. Patent No. 8,178,339 (Table 6). The *endA* gene was then deleted from MDS41 to produce MDS42. Twenty-seven additional nucleic acid deletions were made in MDS42 to create MDS69. MDS42 and all strains based on MDS42 (MDS43, MDS44...MDS69 etc.) are free of insertion sequences.

For production of insoluble CRM197, a modified CRM197 sequence was employed comprising DNA sequence changes that result in a release of hairpin structures in the CRM197 sequence. The optimized CRM197 sequence removes secondary structure that inhibits translation initiation and enhances recognition of both the start site (ATG) and ribosomal binding site (RBS). See Figure 1.

The native CRM197 signal sequence was removed and the optimized CRM197 sequence (cyto-CRM197, SEQ ID NO: 3) amplified by PCR andsubcloned into expression vector pSX2, which contains a Kanamycin resistance cassette and uses a lactose-inducible promoter to drive expression of the cloned sequences. Plasmid pSX2 containing CRM197 (lacking its native signal sequence) was transformed into reduced genome *E. coli* strain MDS42 and examined in shake flask culture. Briefly, 3 ml cultures were grown to saturation in Korz minimal medium (Korz DJ et al., J. Biotechnol., 39(1):59-65 (1995)) supplemented with 0.2% glucose and 50 µg/ml Kanamycin and used to inoculate 20 ml cultures to an initial OD₆₀₀ = 0.075. The growth temperature and inducer (IPTG) concentration that produced optimal levels of insoluble cytoplasmic CRM197 were then determined (in minimal media supplemented with the plasmid-selectable antibiotic kanamycin) using shake flasks. The optimal IPTG concentration was determined to be 250 µM. Figure 2 is an example of a protein gel analyzing total cell protein (T) and the soluble fraction (S) and insoluble fraction (I) following high speed centrifugation of total cell protein from three separate cultures grown to an OD₆₀₀ of 0.5 (late induction) prior to the addition of IPTG. Surprisingly high amounts of cyto-CRM197 were present in the insoluble fractions (see Figure 2, arrows). When quantified against protein standards, the shake flask results predict 10 to 12 g/L of cyto-CRM197 in a modest fermentation of OD₆₀₀ of 200. Production of insoluble CRM197 in the reduced genome *E*. *coli* host cell was 10 times higher than in conventional *E*. *coli* strains.

### Example 2

### Periplasmic Expression of Soluble CRM197 in Reduced Genome E. coli Hosts

Next, production of soluble CRM197 in reduced genome *E*. *coli* strains was tested by directing expression of CRM197 to the periplasmic space. CRM197 has proved notoriously difficult to produce in a soluble form in *E*. *coli.* Export of highly expressed proteins to the periplasmic space aids stability by providing an optimal non-reducing environment for correct protein folding and formation of disulfide bridges. To this end, six signal sequences, in combination with a number of co-expressed chaperone proteins were examined to identify the signal sequence and chaperone protein that conferred the highest levels of periplasmic delivery of CRM197. Figure 3 illustrates the signal sequences examined and the co-expressed chaperone proteins. The signal sequences examined included representative signal sequences from each of the three *E*. *coli* secretion pathways

The CRM197 open reading frame (ORF), codon-optimized for *E. coli* (SEQ ID NO: 1), was ordered from DNA 2.0 (Menlo Park, CA). The CRM197 ORF was preceded by a sequence encoding a PelB signal sequence. The *pelB* and CRM197 ORF were flanked by sequences designed to facilitate cloning into the pSX2 expression vector. The nucleotide sequence of the 5' flanking sequence-PelB signal sequence-CRM197 ORF (including stop codon)-3' flanking sequence is provided at Table 1 below, with the flanking sequences underlined, the nucleotide sequence encoding the PelB signal sequence in bold, and the CRM197 ORF in plain text.

**Table 1: pelB (bold)-CRM197 nucleotide sequence (plain text) + flanking sequences (underlined):**

| |
|---|
| CCTCTAGAAATAATTTTGTTTAACTTTGAAGGAGATATACATATGAAATA CTTGCTGCCAACCGCCGCCGCCGGCCTGCTGCTGCTCGCAGCACAGCCGGCTA TGGCAGGTGCGGATGATGTTGTGGACAGCTCTAAGTCTTTTGTGATGGAAAACTTTA GCTCGTACCACGGTACGAAGCCAGGTTATGTCGACAGCATTCAAAAAGGTATCCAGA AACCGAAGTCCGGCACGCAGGGTAACTACGACGATTGGAAAGAGTTCTACAGC ACCGACAACAAGTATGACGCAGCGGGTTACAGCGTTGACAATGAGAATCCGTTGAGC GGCAAAGCGGGTGGTGTTGTCAAAGTGACGTATCCGGGTCTGACCAAGGTCCTGGCG TTGAAAGTTGATAACGCGGAAACCATTAAGAAAGAGCTGGGTCTGAGCCTGACCGAG CCGTTGATGGAGCAAGTTGGTACCGAAGAGTTTATCAAACGTTTCGGCGATGGTGCG AGCCGCGTTGTCCTGTCCCTGCCTTTCGCGGAGGGCAGCTCCAGCGTTGAGTATATC AATAACTGGGAGCAAGCAAAAGCGCTGTCCGTCGAACTGGAAATCAATTTTGAAACG CGCGGTAAACGTGGTCAAGATGCAATGTACGAGTATATGGCCCAGGCCTGCGCTGGT AATCGTGTTCGTCGCAGCGTTGGTAGCAGCTTGTCTTGTATCAACCTGGATTGGGAT GTGATCCGTGATAAGACCAAGACTAAGATCGAGAGCCTGAAAGAACATGGCCCGATT AAGAACAAGATGTCGGAGAGCCCGAATAAGACCGTGAGCGAAGAAAAGGCCAAGCA GTATCTGGAAGAGTTCCACCAAACGGCTCTGGAGCATCCGGAGCTGAGCGAGCTGAA AACGGTTACGGGCACCAACCCGGTGTTCGCAGGTGCGAATTACGCGGCGTGGGCAGT GAATGTGGCGCAGGTCATCGACTCCGAAACGGCGGACAATTTGGAGAAAACCACCG CAGCGCTGAGCATTCTGCCGGGCATCGGCAGCGTTATGGGCATTGCAGATGGTGCTG TGCACCATAACACTGAAGAAATCGTAGCGCAAAGCATTGCCCTGTCTAGCTTGATGG TGGCGCAGGCTATTCCGCTGGTCGGCGAACTGGTTGATATCGGCTTTGCTGCCTACA ACTTCGTTGAAAGCATCATTAACCTGTTTCAGGTGGTCCACAACAGCTATAATCGCCC AGCGTACAGCCCGGGTCACAAGACCCAACCGTTCCTGCACGATGGCTATGCGGTGTC TTGGAACACGGTCGAAGATAGCATCATTCGTACCGGTTTCCAGGGCGAGAGCGGCCA TGACATCAAGATTACTGCAGAAAATACCCCGCTGCCGATCGCAGGTGTCCTGCTGCC |
| TACGATTCCGGGTAAGCTGGACGTTAACAAAAGCAAAACCCACATTTCTGTGAACGG TCGTAAGATTCGCATGCGTTGTCGTGCGATTGACGGCGACGTCACCTTCTGCCGTCC GAAGAGCCCGGTCTACGTTGGTAATGGTGTGCACGCGAACCTGCACGTGGCGTTTCA CCGCAGCAGCTCGGAGAAAATCCATAGCAATGAGATTTCTAGCGACAGCATTGGCGT TCTGGGTTACCAAAAGACGGTTGACCATACCAAAGTCAATTCCAAACTGAGCCTGTT CTTTGAGATCAAAAGCTAACTCGAGCCCAAGGGCGACACCCCCT |

The nucleotide sequence encoding the PelB-CRM197 ORF was PCR amplified from the DNA 2.0 clone with a sense primer (GGAGATATACATATGAAATACTTGCTGCCAACC) and antisense primer (CTTTGTTAGCAGCCGATTAGCTTTTGATCTCAAAGAACA) to generate the flanking regions needed for cloning into the pSX2 vector.

Alternative signal sequences were fused to the CRM197 ORF using a 2 or 3 step PCR process. In the first step, a sense primer covering both the C-terminal coding region of the signal sequence and the N-terminal coding region of CRM197 was used together with an anti-sense primer covering the C-terminal coding region of CRM197. In the second step, a primer completing the ORF of the signal sequence was used with the same primer covering the C-terminal coding region of CRM197. In the case of the OmpA-CRM197 construct, a third step was used that included a shorter primer covering the N-terminal region of the signal sequence and the same primer covering the C-terminal coding region of CRM197. Primers used to fuse the *E*. *coli* ompA and OmpF signal sequence to the CRM197 ORF are described below.

The following primers used to fuse the *E. coli ompA* encoded signal sequence to the CRM197 ORF. For Step 1, the sense primer = 5'-GCTACCGTAGCGCAGGCCGGTGCGGATGATGTTGTGGA-3' and the antisense primer = 5'-CTTTGTTAGCAGCCGATTAGCTTTTGATCTCAAAGAACA-3'. For Step 2, the sense primer = 5'-GGAGATATACATATGAAAAAGACAGCTATCGCGATTGCAGTGGCAC TGGCTGGTTTCGCTACCGTAGCGCAGGCC-3' and the antisense primer = 5'-CTTTGTTAGCAGCCGATTAGCTTTTGATCTCAAAGAACA-3'. For step 3, the sense primer = 5'-GGAGATATACATATGAAAAAGACAGCTATCG-3' and the antisense primer = 5'-CTTTGTTAGCAGCCGATTAGCTTTTGATCTCAAAGAACA-3'.

The following primers were used to fuse the *ompF*encoded signal sequence to the CRM197 ORF. For Step 1, the sense primer = 5'-GTTAGTAGCAGGTACTGCAAACGCTGGTGCGGATGATGTTGTGGA-3' and the antisense primer = 5'-CTTTGTTAGCAGCCGATTAGCTTTTGATCTCAAAGAACA-3'. For Step 2, the sense primer = 5'-GGAGATATACATATGATGAAGCGCAATATTCTGGCAGTGATCGTCCCTGCTCTGTTA GTAGCAGGTACTGCAAACGCT-3' and the antisense primer = 5'-CTTTGTTAGCAGCCGATTAGCTTTTGATCTCAAAGAACA-3'.

Completed signal sequence-CRM197 PCR products were cloned into the pSX2 expression vector. The termini of the signal sequence-CRM197 PCR products possessed 15 bp of sequence that overlaps the sequence of the pSX2 vector. The pSX2 vector was linearized with the restriction enzymes Kpn I and Sac I to facilitate the cloning reaction. Cloning reactions were transformed into MDS42, MDS42recA or MDS42recA with a further deletion of IS609, to generate recombinant pSX2 expression vectors. The signal sequence-CRM197 region and flanking vector sequences were verified by sequence analysis.

Plasmid pSX2 containing the combinations of signal sequence and CRM197 sequence (lacking its native signal sequence) illustrated at Figure 3 (B) were transformed into reduced genome *E*. *coli* strain MDS42 or MDS42recA (MDS42 strain with a deletion of the *recA* gene (the *recA1819* allele)) and examined in shake flask culture. In addition to signal sequence and chaperone protein, culture variables examined included temperature, inducer (IPTG) concentration and time point at which the inducer was added (either early [OD₆₀₀ₙₘ of 0.01] or late [OD₆₀₀ₘₙ of about 0.4]). The following conditions were determined to be optimal for periplasmic secretion of CRM197 and these conditions were used in subsequent experiments: (i) a growth temperature of about 25 °C preceded by a brief 37 °C incubation (e.g. 2 hours) (ii) late induction (addition of IPTG at an OD₆₀₀ of about 0.4) and (iii) an inducer (IPTG) concentration between 15 and 35 µM (about 1/10 that required for optimal expression of cyto-CRM197).

Briefly, 3 ml cultures were grown to saturation in Korz minimal medium supplemented with 0.2% glucose and 50 µg/ml Kanamycin and used to inoculate 20 ml cultures to an initial OD₆₀₀ = 0.075. The 20 ml cultures (in 125 ml baffled Erlenmeyer flasks) were placed into a 37 °C shaking incubator (250 rpm) for 2 hours. The cultures were then shifted to a 25 °C shaking incubator and monitored until OD₆₀₀ was between 0.3-0.4. At that time, IPTG was added at the indicated concentrations. The induced cultures were incubated overnight in the 25 C shaking incubator. Total induction time was between 18-22 hours. After induction, the OD₆₀₀ of the cultures was determined. Aliquots of the culture representing 2 OD units were processed to create periplasmic samples. The periplasmic samples were prepared with the aid of Periplasting Buffer (Epicentre, Madison, WI). The 2 OD sample was harvested by centrifugation at 7500xg for 10 minutes in a 1.5 ml Eppendorf tube. The supernatant was removed and the cell pellet gently resuspended in 50 µl of Periplasting Buffer (200 mM Tris-HCl [pH 7.5], 20% sucrose, 1 mM EDTA, and 30 U/µl Ready-Lyse Lysozyme). After 5 minutes at room temperature, 50 µl of ice cold water was rapidly added to the resuspended pellet. The mixture was incubated on ice for 5 minutes prior to fractionating the periplasmic fraction from the spheroplasts by centrifuging at 4000xg for 15 minutes. The supernatant representing the periplasmic fraction was prepared for SDS-PAGE analysis. An amount equivalent to 0.12 OD units was loaded per lane.

The most successful signal sequences and induction characteristics that resulted in the highest secretion of CRM197 into the periplasm are shown in Figure 4. The periplasmic signals OmpA and OmpF were found to facilitate the greatest movement of CRM197 into the periplasm. None of the three co-expressed chaperone proteins influenced periplasmic delivery differently (as an example expression is compared with and without, YccA in Figure 4). Since OmpA and OmpF appeared to result in roughly similar amounts of periplasmic CRM197, OmpA-CRM197 was used in subsequent experiments.

Since expression of components of the sec-dependent pathway that include *omp*A and *omp*F can be subject to catabolite repression, the influence of glycerol as a carbon source for production of ompA-CRM197 was compared to glucose in reduced genome *E*. *coli* strain MDS42 in shake flask cultures under the conditions described above. As illustrated at Figure 5, minimal media supplemented with glycerol resulted in dramatically lower levels of CRM197 expression compared to glucose. Glucose was therefore used as a carbon source in all subsequent experiments.

Next, the production of periplasmic CRM197 in several different reduced genome *E*. *coli* host cells was compared. Thus, a series of deletions within either the MDS42 or MDS69 strain background were examined for their effect on production of periplasmic (soluble) CRM197 in shake flask cultures based on the optimal conditions described above for MDS42 that contained either (i) deletions that optimized cell metabolism or (ii) deletions that remove or reduce the level of proteases (e.g. Blon) that could adversely influence CRM197 expression. The following reduced genome *E. coli* strains based on MDS42 were tested: MDS42recA, MDS42metab, and MDS42Blon/metab. MDS42metab was created by (i) deleting the *iclR* (b-number b4018, described at NCBI Entrez GeneID No. 948524) and *arpA* genes (b-number b4017, described at NCBI Entrez GeneID No. 944933) (ii) deleting the *rph* gene (b3643) (thereby increasing transcriptional levels of the downstream *pyrE* gene), and (iii) correcting the *ilvG* frameshift mutation by insertion of an AT dinucleotide at position 982 (resulting in expression of active acetohydroxy acid synthase II). MDS42Blon/metab contains the modifications described for MDS42metab as well as a modification of the lon protease (b0439) promoter region to mimic the sequence of the lon promoter region of B strain *E*. *coli,* in which an IS insertion separates the -35 region from the -10 region of the ancestral E. *coli lon* promoter. The following reduced genome *E. coli* strains based on MDS69 were tested: MDS69metab (MDS69 strain modified as described above for MDS42metab), MDS69Blon/metab (MDS69metab further altered to include the Blon protease modification, MDS691pp/metab (MDS69metab further modified to delete lipoprotein lpp (b1677), and MDS69Blon/lpp/metab (MDS69metab further modified to include both the Blon protease modification and lipoprotein *lpp* gene deletion).

Figure 6 compares OmpA-CRM197 expression in these strains. Of the eight strains examined, the highest levels of CRM197 expression were evident in those strains (on either MDS42 or MDS69 backgrounds) that contained deletions aimed at enhancing metabolic activity (metabolism strains). However, all strains tested contained a surprisingly large amount of periplasmic CRM197 (Fig. 6 panels B and D) that was also evident on protein gels in total cellular protein preparations (Fig. 6 panels A and C). Importantly, these results indicate that the protease deletion Blon did not influence CRM197 levels in the metabolism strains. In addition, removal of the highly abundant lipoprotein protein Lpp, thought, by its absence, to "free up" the sec-dependent periplasmic transport system was also not found to influence periplasmic CRM197 levels. The media from these experiments was isolated post-induction and examined for CRM197 release. CRM197 was not identified in media from any of the strains examined. Table 2 is a summary of yield results for the MDS strains that generated the highest periplasmic CRM197 expression levels. These results were obtained by comparing stain intensities of CRM197 from the four strains indicated with protein standards run on the same gel. The shake flask values were extrapolated to predict quantities of CRM197 in fermentations that reach either 100 or 200 OD₆₀₀. The four strains shown in Table 2 typically reach ODs of 300 in fed-batch fermentation suggesting that these strains have the capacity for generating far more CRM197 than is currently possible in conventional strains.

**Table 2**

| **Expression Strain with pSX2-ompA CRM197** | **Periplasmic Samples** | **#ODs loaded** | **Calibrated Volume ngs/lane** | **% Target Protein in Periplasm** | **AVG g/L at 100 ODs** | **AVG g/L at 200 ODs** |
|---|---|---|---|---|---|---|
| MDS42 Metabolism | Peri, Late 25 µM IPTG, 25°C o/n | 0.06 | 1102 | 48% | 1.75 | 3.5 |
| | | 0.03 | 499 | 53% | | |
| MDS69 Metabolism | Peri, Late 25 µM IPTG, 25°C o/n | 0.06 | 1047 | 43% | 1.62 | 3.24 |
| | | 0.03 | 449 | 48% | | |
| MDS42 Δprotease | Peri, Late 35 µM IPTG, 25°C o/n | 0.06 | 825 | 47% | 1.24 | 2.48 |
| | | 0.03 | 330 | 45% | | |
| MDS69 Δprotease | Peri, Late 25 µM IPTG, 25°C o/n | 0.06 | 1028 | 28% | 1.7 | 3.4 |
| | | 0.03 | 506 | 30% | | |

CRM197 is highly sensitive to proteolytic cleavage which has rendered production of high quality CRM197 challenging (Bishai et al., J. Bacteriol., 169:5140-51 (1987); Recombinant Production of Carrier Proteins, GEN News, Dec. 1, 2012). In a separate set of experiments, production of periplasmic CRM197 was examined in a series of protease deletion strains to determine whether the targeted removal of protease genes from the reduced genome E. *coli* strains would result in an increase in CRM197 in the periplasm. Thus, the following protease encoding genes were deleted separately in combination: *degP* (b0161), *prc* (b1830), *htpX* (b1829), as well as portions of the *lon* promoter region. Deletion of the protease genes, either individually or in combination, did not influence CRM197 expression levels. See Figure 7, illustrating that reduced genome *E*. *coli* strain MDS42, modified to delete the specified combination of protease genes, had no effect on periplasmic expression of CRM197. This data indicate that proteolytic cleavage of CRM197 does not occur when produced in reduced genome *E. coli* strains based on MDS42 or MDS69 presumably due to the low levels of protease activity in these strains.

### Example 3

### CRM197 Production in Fed-Batch Fermentation

Next, commercial scale-up of CRM197 in reduced genome E. *coli* strains was examined. Thus, OmpA-CRM197 in the MDS42 metabolism strain was subjected to fed-batch fermentation in defined minimal media at the 10 liter scale. Fermentation conditions included a batch phase at 37 °C that was inoculated to 0.18 OD and allowed to grow until the 1% glucose in the batch medium has been consumed (~7.5 hrs). The fed batch phase was triggered by the DO spike that occurs when the batch medium is depleted of glucose. The feed began with an exponential feed rate to produce a growth rate of 0.3 Mu (1/h) controlled gravimetrically (~12.5hrs). The induction point was determined to be the point at which the available phosphate was nearly depleted. At a point around 2 hours prior to the induction point, the temperature was shifted to 25 °C and the feed rate was lowered to a rate that produces a growth rate of 0.2 Mu (1/hr). Once the inducer is added (100uM) the feed was changed to a constant rate such that 80g of glucose is added per hour for about 7hrs. The fermentation OD₆₀₀ approached 300 and generated a very high level of periplasmic targeted CRM197 as illustrated at Figure 8. A second fermentation at the optimal conditions resulted in periplasmic CRM197 levels of about 2 g/L indicating a high level of consistency in test fermentations.

The results described above demonstrate the surprising yield of soluble CRM197 obtained in reduced genome *E*. *coli* production hosts such as MDS42 and MDS69 in both shake-flask and 10 L fed-batch fermentation.

One problem observed during preliminary fermentation analysis was a reduction in the soluble form of CRM197 in total cell protein isolations. Since periplasmic isolation methods are not applicable to large scale, a general method of soluble CRM197 isolation was developed. Initial experiments were performed to determine whether the CRM197 observed following conventional total cell protein (TCP) isolation that was insoluble could be isolated in a soluble form. Thus, OmpA-CRM197 in the MDS42*rec*4 strain was subjected to fed-batch fermentation in defined minimal media at the 10 liter scale as described above (including incubation at 37 °C followed by a short period of incubation at 25 °C prior to the addition of inducer). The cells, containing high amounts of periplasmic CRM197, were subjected to standard detergent digestion with a commercially available non-ionic detergent-based buffer to isolate total cell protein (TCP). Samples of total cell protein were centrifuged for 10 minutes at high speed (21k g) and the soluble fraction was isolated. Samples of TCP and the soluble fraction were analyzed. As illustrated at Fig. 9, Panel A, the soluble periplasmic form of CRM197 was rendered completely insoluble by detergent homogenization. Conversely, when periplasmic preparations (as described above) were subjected to high speed centrifugation, periplasmic CRM197 was retained in a soluble form as expected. See Fig. 9, Panel B.

In an attempt to recover the fraction of CRM197 that was insoluble, detergent-based bacterial cell lysis was compared with mechanical methods of cell lysis which would be more conducive to production-level platforms for generating CRM197 compared to detergent lysis and would eliminate the need to isolate periplasm in a commercial scale-up process. In addition, lysis was performed in the presence of chemical agents known to enhance protein solubilization as described at Table 3 below:

**Table 3: List of lysis method and solubilization agent.**

| **Agent to enhance solubilization** | **% soluble CRM197 after sonication** |
|---|---|
| Imidazole, 250 mM | 107% |
| Trehalose, 50 or 250 mM | 64%, 79% |
| Glutathione in 5:1 reduced to oxidized state | 104% |
| Glycerol, 10% | 68% |
| Sucrose, 10% | 75% |
| No agent | 88% |

Sonication and microfluidization were performed in a 50 mM TrisHCl buffer (pH 8) and all lysis methods were carried out in the presence of Lysonase^{™}, a commercial mixture of lysozyme and benzonase (Novagen, Darmstadt, Germany). Each of the agents listed in Table 3 were then tested in separate preparations. Figure 10 is an example of a series of isolations that were performed by detergent or mechanical lysis. Soluble CRM197 was not obtainable using detergent lysis and only small increases in soluble CRM197 were evident using detergent lysis that included solubilization agents. Glycerol and sucrose modestly enhanced the amount of soluble CRM197 found in the soluble fraction when compared to detergent alone (Fig. 10, Panel A). However, mechanical lysis dramatically increased the levels of CRM197 in the soluble fraction. In fact, a dramatic increase of CRM197 levels was evident in the soluble fraction from all samples that underwent mechanical lysis, whether sonication (Fig. 10, Panel B) or microfluidization was used. Further, the amounts of soluble CRM197 obtained following mechanical lysis did not differ markedly by solubilization agent (compare "no agent" with all other agents in Table 3). A compilation of results generated from the mechanical lysis method suggests that CRM197 in MDS42 (using culture conditions that include a short 37 °C incubation followed by growth at 25 °C and late stage induction with 25-35 µM IPTG) comprises 7.2-8.3% of the total cellular protein and between 6.3 and 7.7% of soluble protein. These results are intriguing because mechanical lysis is the standard method of cell disruption used in large scale commercial fermentations and imply the capability of generating high amounts of soluble CRM197.

Based on the aforementioned data, a suitable commercial protocol for generating soluble CRM197 comprises fermentation of reduced genome *E*. *coli* host carrying an expression vector encoding CRM197 coding sequence fused to a periplasmic signal sequence (e.g. encoded by *ompA* or *ompF*) at 25 °C in which the cells are collected by low speed centrifugation, lysed by mechanical means (e.g. sonication or microfluidization) in a suitable buffer (e.g. 50 mM Tris-HCl buffer at pH ~8). Following centrifugation to remove debris, soluble CRM197 is then isolated from the supernatant. In shake flask cultures incubated at 25 °C and 25-35 mM IPTG, between 95 and 100% of CRM197 was isolated in a soluble form.

A summary of the results of fermentations using reduced genome *E*. *coli* strain MDS69 metab (as described above) carrying an expression vector containing an ompA-CRM197 fusion is shown at Table 4 below. These fermentations occurred under fed-batch conditions using defined minimal media and the addition of inducer IPTG late in logarithmic growth. The fermentation scale was 10 liters. By altering the inducer concentration the amount of periplasmic CRM197 was increased from 0.5 to about 2 g/L.

| **Table 4:** Fed-batch fermentations of strain Ta9 metabolic using glucose feed | | **Max yield@ 29 hours** | |
|---|---|---|---|
| **Fermentation** | **Induction Level** | **OD** | **Soluble CRM₁₉₇ Yield** |
| **Ferm 167** | **25 uM** | 256 | 0.74 g/L |
| **Ferm 168** | 50 uM | 262 | 1.62 g/L |
| **Ferm 169** | 100 uM | 291 | 1.96 g/L |

Figure 11 illustrates specifics of the fermentation employing a 100 µM inducer concentration. Gels from this fermentation comparing total cell protein (TCP) isolates with soluble (Sol) and insoluble (Insol) fractions clearly indicated robust expression of soluble CRM197 during fermentation (see Fig. 12).

Optimal conditions for production of soluble CRM197 in fed-batch fermentation of reduced genome *E*. *coli* host strains were as follows. With respect to temperature, initiation of growth in the batch phase by incubating at 37 °C followed by a temperature shift to between 20 and 25 °C prior to addition of inducer (in this case IPTG) was optimal. Optimal pH range is between 6.5 and 7.5 (e.g. 6.5, 7.0 or 7.5). Optimal inducer concentration is between 100 and 250 µM IPTG (added during late log phase of growth). With respect to media conditions, minimal media conditions were determined to be adequate and have the advantage of reduced cost and defined conditions free from animal derived products. Importantly, conventional *E*. *coli* strains do not grow robustly in minimal media. Employing these optimal conditions, it is estimated that a target yield of at least 4 g/L of soluble CRM197 can be reliably produced in 10 L scale fermentations using reduced genome *E. coli* host strains (e.g. MDS42 or MDS69).

### Example 4

### Downstream Processing of CRM197

Following production of CRM197 in reduced genome *E*. *coli* and mechanical lysis, the CRM197 can be purified. To determine whether CRM197 produced from MDS69 metab under fermentation conditions is amenable to purification, a small scale purification was performed using a combination of hydrophobic interaction chromatography (phenyl sepharose) and anion exchange chromatography (DEAE-cellulose). 50 OD units of the 28 hr fermentation sample shown in Fig. 11 was subjected to homogenization using a microfluidizer (MF) in a 10 mM sodium phosphate buffer (pH 7.5) solution. The resulting homogenate was centrifuged at 21,000 g for 10 minutes and the soluble and insoluble (IS) fractions were isolated. Using Western blotting and polyclonal antibodies against diphtheria toxin (DPX), CRM197 was found to be highly enriched in the soluble fraction (Fig. 13, panel A compares the microfluidizer (MF) and the resuspended insoluble (IS) fraction with the pre-column soluble fraction at three concentrations (0.1, 0.07 and 0.04 OD)). The soluble fraction (25 OD equivalent) was filtered (0.45 µm), brought to 13% (wt/vol) ammonium sulfate and loaded onto a phenyl sepharose column (Phenyl sepharose HP HiTrap, General Electric) that was previously equilibrated in 10 mM sodium chloride, 10 mM sodium phosphate buffer, pH 7.5. The column was washed using 0.6 M ammonium sulfate, 6 mM sodium phosphate buffer, pH 7.5 and CRM197 was eluted under low salt conditions (10 mM sodium choloride, 10 mM sodium phosphate buffer, pH 7.5). The five 2.5 mL eluted fractions were then analyzed by anti-DPX Western blot and protein staining (Fig. 13, Panel A, lanes labeled 10 mM NaCl). A small amount of unprocessed CRM197 (Fig. 13, Panel A, arrows) was purified away from the main eluted sample with a final wash with distilled water. The fractions circled in Fig. 13 Panel A were then pooled, diluted 1:2 with distilled water and loaded onto a column containing 1 ml of DEAE sepharose fast flow (Pharmacia) that had been equilibrated in 10 mM sodium chloride, 10 mM sodium phosphate, pH 7.5. After loading the sample and collecting the flow through, the column was washed with 3 volumes of 50 mM sodium chloride, 0.5 mM sodium phosphate buffer, pH 7.5. CRM197 eluted using increasing sodium chloride concentrations: 100 mM NaCl (2 times 3 ml), 1 M NaCl (3 ml) and 1.5 M NaCl (3 ml). SDS-PAGE analysis revealed that the most highly pure soluble CRM197 was eluted using 1 M NaCl, although a significant amount still remained bound to the column.

These results indicate that CRM197 produced in reduced genome *E*. *coli* host strains is highly soluble and can be isolated to high purity using existing purification methods.

### Example 5

### CRM197 Production in Reduced Genome E. coli Hosts Compared to Wild Type Strains

Periplasmic production of CRM197 in reduced genome *E. coli* strains was compared to the production of CRM197 in wild type *E*. *coli* strains under similar conditions. Thus, CRM197 *E. coli* BLR(DE3) strain was transformed with pSX2 vector carrying an OmpA-CRM197 fusion and periplasmic production was assessed and compared to periplasmic production of CRM197 in reduced genome *E*. *coli* strain MDS42*recA*. Fermentation conditions were as described above. Following a brief growth initiation phase at 37 °C, cells were grown in Korz media supplemented with 0.2% glucose (and 31 µg/ml of Isoleucine for BLR(DE3) cultures) at 25 °C for 19 hours. Expression of CRM197 was induced at OD = 0.3 with 15 or 25 mM IPTG.

As illustrated at Figure 14, at least a ~5-fold increase in production of periplasmic CRM197 was observed in the reduced genome *E. coli* host compared to the wild type B strain.

Additional experimentation revealed that the OmpF-CRM197 fusion actually resulted in a higher amount of soluble periplasmic CRM197 in reduced genome *E. coli* hosts compared to the OmpA-CRM197 fusion. Reduced genome *E. coli* host strain MDS69 metab and MDS69 lowmut (MDS69 strain further comprising deletions of *polB* (b0060), *dinB* (b0231) and *umuDC* (b1183-b1184)) were transformed with an expression vector encoding an OmpF-CRM197 fusion and periplasmic expression of CRM197 was compared to that in a MDS69 lowmut host carrying an expression vector encoding an OmpA-CRM197 fusion under the same conditions. Following a brief growth initiation phase at 37 °C, cells were grown in Korz media supplemented with 0.2% glucose at 25 °C for 23 hours. Expression of CRM197 was induced at OD = 0.3 to 0.34 with 25 or 35 mM IPTG. Periplasmic proteins were isolated and the expression of soluble CRM197 in each strain was analyzed. As illustrated at Figure 15, a higher yield of CRM197 was obtained with the OmpF-CRM197 construct compared to the OmpA-CRM197 construct.

### Example 6

### Testing CRM197 Production with a Variety of Signal Sequences in a reduced genome E. coli strain

Signal sequences were selected based on their abundance in the periplasm of *E. coli* B and K strains as determined by 2D gel analysis of periplasmic fractions (Han, Mee-Jung et al., Journal of Bioscience and Bioengineering, 117(4):437-442 (2014)). Table 5 lists the signal sequences selected and their relative abundance in the periplasm of B and K strains:

**Table 5**

| Protein | Abundance in Periplasmic Fraction (B and/or K) | Gene/Protein Function |
|---|---|---|
| MglB | K | methyl galactose transporter |
| MalE | B + K | maltose transporter |
| OppA | B +_K | oligopeptide transporter |
| RbsB | B + K | subunit ribose transporter |
| Agp | B > K | glucose-1 phosphatase, 3-phytase |
| FkpA | B > K | peptidyl-prolyl cis-trans isomerase; in protein folding |
| YtfQ | B » K | galactofuranose binding protein, subunit ABC transporter |
| HdeA | K | Stress response induced by acidic conditions |
| HdeB | K | Stress response induced by acidic conditions |
| GlnH | B > K | subunit of glutamine ABC transporter |

Plasmid pSX2 containing the combinations of signal sequence and CRM197 sequence (lacking its native signal sequence) illustrated at Table 5 and Figure 17 (MglB, MalE, OppA, RbsB, Agp, FkpA, YtfQ, HdeA, HdeB or GlnH; OmpF and OmpC were tested as well) was transformed into reduced genome *E*. *coli* strain MDS69 metab (T69 Meta in Figure(s) 18-21) and examined in shake flask culture. As described above, MDS69 metab comprises the following modifications on an MDS69 background (i) deletion of the *iclR* (b-number b4018, described at NCBI Entrez GeneID No. 948524) and *arpA* genes (b-number b4017, described at NCBI Entrez GeneID No. 944933) (ii) deletion of the *rph* gene (b3643), and (iii) correction of the *ilvG* frameshift mutation by insertion of an AT dinucleotide at position 982. Briefly, colony forming units of the transformed bacteria from MOPS minimal medium-kanamycin (MMM/Kan)-glucose streak plates were resuspended in 3 ml Korz minimal medium supplemented with 0.2% glucose and 50 µg/ml Kanamycin and incubated at 37 °C overnight to generate the starter culture. Starter culture was used to inoculate 20 ml Korz/0.2% glucose/Kan in 125 ml Erlenmeyer-flasks to OD600 = 0.05 and grown at 37 °C for 1.5 hours and then shifted to 25 °C and grown until OD₆₀₀ ~0.3. At that point, inducer (IPTG) was added at 25 µM, 35 µM or 50 µM concentration (late induction). The late inductions were then grown at 25 °C for 20 hours and 2 ODs of culture were harvested. Total cell protein was prepared using BugBuster + Lysonase and periplasmic and spheroplast fractions were prepared using Epicentre Periplasting Method.

Figures _18-20 depict the periplasmic yield of (soluble) CRM197 at 25 µM, 35 µM or 50 µM inducer concentration respectively using the indicated signal sequences (Induction A - OmpF, MalE, HdeA, OppA, HdeB, GlnH; Induction B - OmpF, MglB, Agp, OmpC, RbsB, FkpA, YtfQ). Good yield was obtained with all signal sequences at the 25 µM inducer concentrations (Figure 18). Interestingly, at the 35 µM inducer concentration, yield of CRM197 with the YtfQ signal sequence significantly increased relative to the yield of CRM197 obtained at this inducer concentration with the other tested signal sequences (Figure 19). This effect became even more significant at the 50 µM inducer concentration, with the yield of CRM197 remaining high whereas the yield of CRM197 with the other tested signal sequences was significantly reduced at this inducer concentration (Figure 20). Thus, the combination of CRM197 and YtfQ signal sequence was determined to have a significantly broader induction range than the combination of CRM197 with the other signal sequences tested.

To further assess the induction range for CRM197 with the YtfQ signal sequence, two cultures each of 8 IPTG (inducer) levels were tested in MDS69 metab (0, 25, 35, 50, 75, 100, 150 and 250 pM) according to the method described above. As a control, 2 cultures each of 4 IPTG levels for MDS69 metab with CRM197 and the OmpF signal sequence were also tested (0, 25, 35, 50 pM). 2 OD samples were collected for total cell protein (TCP) and periplasmic analysis on Caliper.

The averaged results of the two cultures tested for each inducer level is illustrated at Figure 21. Yield of CRM197 in combination with the YtfQ signal sequence (YtfQ-CRM197) remained high across all inducer levels up to 100 µM. Yield of CRM197 in combination with OmpF, however, was high only at the 25 and 35 µM inducer concentrations. Figure 22 is a protein gel comparing the effect of OmpF and YtfQ signal sequence on CRM197 yield in periplasm (P) and media (M) at 50 µM IPTG (OmpF) and at 50, 75, 100, 150 and 250 µM IPTG (YtfQ). A surprisingly large amount of periplasmic CRM197 was evident at the 50, 75 and 100 µM inducer concentration for the YtfQ signal sequence whereas a much smaller amount of periplasmic CRM197 was present at the 50 µM inducer concentration for the OmpF sequence.

"Briefly, colony forming units of the transformed bacteria from MOPS minimal medium-kanamycin (MMM/Kan)-glucose streak plates were resuspended in 3 ml Korz minimal medium supplemented with 0.2% glucose and 50 µg/ml Kanamycin and incubated at 37 °C overnight to generate the starter culture. Starter culture was used to inoculate 20 ml Korz/0.2% glucose/Kan in 125 ml Erlenmeyer-flasks to OD₆₀₀ = 0.05 and grown at 37 °C for 1.5 hours and then shifted to 25 °C until OD₆₀₀ ~0.3. At that point, inducer (IPTG) was added at 25 µM, 35 µM or 50 µM concentration (late induction). The late inductions were then grown at 25 °C for 20 hours and 2 ODs of culture were harvested. Total cell protein was prepared using BugBuster + Lysonase and periplasmic and spheroplast fractions were prepared using Epicentre Periplasting Method"

Next, the effect of very late induction (OD₆₀₀ ~ 2) on CRM197 yield in combination with either OmpF or YtfQ signal sequence in MDS69 metab and in combination with OmpF in an *E. coli* B strain (BL21DE3 ) was assessed. Briefly, 3 ml Korz minimal medium supplemented with 0.2% glucose and 50 µg/ml Kanamycinin was inoculated with colony forming units of transformed MDS69 metab or BL21DE3, incubated at 37 °C overnight and used to inoculate 15 ml of Korz minimal medium supplemented with 0.2% glucose and 50 µg/ml Kanamycinin in 125 ml Erlenmeyer Flasks which was grown overnight at 25 °C to generate the 25 °C starter culture. The starter culture was used to inoculate 90 ml Korz/0.2% glucose/kanamycin in 500 ml Erlenmeyer flasks to OD₆₀₀ = 0.1 followed by growth at 25 °C until the OD₆₀₀ > 2 (saturated or near saturated) and then split into 4 x 20 aliquots in 125 ml Erlenmeyer flasks for induction at various IPTG concentrations (very late induction). The inductions were grown at 25 °C for 20 hours, and 2 ODs of culture harvested for analysis. Total cell protein (TCP) was prepared using BugBuster + Lysonase. Periplasmic and spheroplast fractions were prepared using the Epicentre Periplasting Method. As shown in Figure 23, good periplasmic expression up to 100 µM IPTG was observed for the combination of CRM197 and OmpF signal sequence in MDS69 metab which decreased at 200 mM inducer concentration, presumably due to insolubility. Good periplasmic expression was observed for the combination of CRM197 and YtfQ signal sequence in MDS69 metab up to 400 µM IPTG (the highest concentration tested) with no insoluble CRM197 observed. Weak expression was observeved for the combination of CRM197 and OmpF in BL21(DE3) strain at all inducer concentrations tested (25-200 pM). CRM197 was not observed in spheroplasts.

Summary - The data presented demonstrates that production of soluble CRM197 in reduced genome *E*. *coli* hosts delivered yields that were 10 times that obtained by conventional methods. Production in the reduced genome *E*. *coli* hosts is expected to increase the efficiency and reduce manufacturing costs. Moreover, production in reduced genome *E*. *coli* hosts will also be cleaner and safer than that produced in conventional bacteria with non-reduced genomes. These improvements will have a wide impact on production of pharmaceutical protein products and ultimately broaden access to vaccines for at-risk populations who need them. Moreover, high yield of CRM197 was observed in combination with a broad range of signal sequences. The broad induction range observed for YtfQ signal sequence in combination with CRM197 was surprising since YtfQ is found in much larger quantities in B strain *E*. *coli* compared to K strain *E*. *coli* and the exemplified reduced genome strains are based on a K strain. The broad induction range of CRM197 in combination with YtfQ in reduced genome *E. coli* is a significant advantage because the concentration of inducer can vary during production of the protein and accordingly the use of the YtfQ signal sequence in combination with CRM197 in these hosts results in a further increase in yield of CRM197.

## Claims

1. A method for producing a recombinant CRM197 in a reduced genome *E. coli* host comprising incubating a reduced genome *E. coli* comprising an expression vector comprising a nucleotide sequence encoding a CRM197 protein fused to an ompA, ompF or ytfQ signal sequence that directs transfer of the CRM197 protein to the periplasm, wherein said nucleotide sequence is operably linked to an expression control sequence, under conditions suitable for the expression of the recombinant CRM197 protein, wherein the native parent *E*. *coli* strain is a K-12 strain comprising a native -2 frameshift mutation wherein the native -2 frameshift mutation comprises a deletion of nucleotides GT at positions 983 and 984 of *ilvG* relative to the intact *ilvG,* preferably K12 MG1655, and wherein the reduced genome *E. coli* host comprises the following modifications: (i) deletion of at least the following DNA segments: b0245-b0301, b0303-b0310, b1336-b1411, b4426-b4427, b2441-b2450, b2622-b2654, b2657-b2660, b4462, b1994-b2008, b4435, b3322-b3338, b2349-b2363, b1539-b1579, b4269-b4320, b2968-b2972, b2975-b2977, b2979-b2987, b4466-4468, b1137-b1172, b0537-b0565, b0016-b0022, b4412-b4413, b0577-b0582, b4415, b2389-b2390, b2392-b2395, b0358-b0368, b0370-b0380, b2856-b2863, b3042-b3048, b0656, b1325-b1333, b2030-b2062, b2190-b2192, b3215-b3219, b3504-b3505, b1070-b1083, bl878-bl894, b1917-b1950, b4324-b4342, b4345-b4358, b4486, b0497-b0502, b0700-b0706, b1456-b1462, b3481-b3484, b3592-b3596, b0981-b0988, b1021-b1029, b2080-b2096, b4438, b3440-b3445, b4451, b3556-b3558, b4455, b1786, b0150-b0153 and b2945 of the *E*. *coli K-*12 strain MG1655 (ii) deletion of the *rph* gene to enhance orotate phosphoribosyltransferase activity, (iii) correction of the native -2 frameshift mutation in the *ilvG* gene in order to restore the active acetohydroxy acid synthase II production, preferably wherein said correction is an insertion of two nucleotides between positions 982 and 983, and (iv) deletion of the *iclR* and *ankyrin-like regulator protein* (b4017) genes; whereby a yield of at least 1 gram per liter, preferably at least 2 grams per liter of soluble CRM197 is obtained.

2. The method of claim 1, wherein the reduced genome *E. coli* has additionally deleted therefrom at least the following DNA segments: : b0315-b0331, b0333-b0341, b0346-b0354, b2481-b2492, b2219-b2230, b4500, b3707-b3723, b0644-b0650, b4079-4090, b4487, b4092-b4106, b0730-b0732, b3572-b3587, b1653, b2735-b2740, b2405-b2407, b3896-b3900, b1202, b4263-b4268, b0611, b2364-b2366, b0839, b0488-b0500, and b0502 of the *E*. *coli* K-12 strain MG1655.

3. The method of claim 1 or 2, wherein the reduced genome *E. coli* host lacks one or more of (i) a functional polB (b0060) gene, (ii) a functional dinB (b0231) gene and optionally lacks (iii) functional umuD (b1183) and umuC (b1184) genes.

4. The method of claim 3, wherein the reduced genome *E. coli* host lacks one or more of (i) a functional polB (b0060) gene, (ii) a functional dinB (b0231) gene and (iii) functional umuD (b 1183) and umuC (b 1184) genes.

5. The method of any one of claims 1-4, wherein the signal sequence is YtfQ.

6. The method of claim 1, wherein the reduced genome *E. coli* comprises a functional *recA* (b2699) gene.

7. The method of any preceding claim, wherein the CRM197 nucleotide sequence is optimized for expression in the *E. coli* host cell.

8. The method of any preceding claim comprising
(a) growing the reduced genome *E. coli* and
(b) inducing expression of CRM197; whereby the method is preferably as follows:
(1) step (a) comprises growing the reduced genome *E. coli* at 37 °C for up to 19 hours followed by growth at about 20- 30 °C, preferably about 25 °C prior to and subsequent to step (b);
(2) steps (a) and (b) are performed in growth medium that does not comprise serum, yeast extract or other animal by-products;
(3) the pH ranges between 6.5 and 7.5 in step (a);
(4) the pH is maintained using a phosphate buffer, a Tris buffer or a histidine buffer, preferably using a phosphate buffer;
(5) expression is induced in step (b) by addition of a suitable amount of IPTG; and/or
(6) comprises a further step of (c) mechanically disrupting the cultured reduced genome *E*. *coli* cells in the absence of detergent and centrifuging the resulting cell lysate to obtain a soluble fraction, preferably the mechanical disruption comprises sonication or microfluidization.

9. The method of claim 8, wherein the method is carried out in a fermentor, preferably wherein:
(1) expression is induced at an OD₆₀₀ of between 100 to 400, preferably between 200 to 275, more preferably between 230 and 250; and or
(2) the fermentor contains 0.5-50,000 liters of culture.

10. The method of claim 8 or 9, wherein the CRM197 is purified from the soluble fraction by one or more purification steps, preferably wherein one or more purification steps comprises hydrophobic interaction chromatography and/or anion exchange chromatography and even more preferably wherein the purification steps comprise hydrophobic interaction chromatography followed by anion exchange chromatography.

11. A reduced genome *E. coli* host comprising an expression vector comprising a nucleotide sequence encoding a CRM197 protein fused to an ompA, ompF or ytfQ signal sequence that directs transfer of the CRM197 protein to the periplasm, wherein said nucleotide sequence is operably linked to an expression control sequence, wherein the native parent *E*. *coli* strain is a K-12 strain, comprising a native -2 frameshift mutation wherein the native -2 frameshift mutation comprises a deletion of nucleotides GT at positions 983 and 984 of *ilvG* relative to the intact *ilvG,* preferably K12 MG1655, and wherein the reduced genome *E*. *coli* host comprises the following modifications: (i) deletion of at least the following DNA segments: b0245-b0301, b0303-b0310, b1336-b1411, b4426-b4427, b2441-b2450, b2622-b2654, b2657-b2660, b4462, b1994-b2008, b4435, b3322-b3338, b2349-b2363, b1539-b1579, b4269-b4320, b2968-b2972, b2975-b2977, b2979-b2987, b4466-4468, b1137-b1172, b0537-b0565, b0016-b0022, b4412-b4413, b0577-b0582, b4415, b2389-b2390, b2392-b2395, b0358-b0368, b0370-b0380, b2856-b2863, b3042-b3048, b0656, b1325-b1333, b2030-b2062, b2190-b2192, b3215-b3219, b3504-b3505, b1070-b1083, b1878-b1894, b1917-bl950, b4324-b4342, b4345-b4358, b4486, b0497-b0502, b0700-b0706, bl456-bl462, b3481-b3484, b3592-b3596, b0981-b0988, bl021-bl029, b2080-b2096, b4438, b3440-b3445, b4451, b3556-b3558, b4455, b1786, b0150-b0153 and b2945 of the *E*. *coli* K-12 strain MG1655, (ii) deletion of the *rph* gene to enhance orotate phosphoribosyltransferase activity (iii) correction of the native -2 frameshift mutation in the *ilvG* gene in order to restore the active acetohydroxy acid synthase II production, preferably wherein said correction is an insertion of two nucleotides between positions 982 and 983 and (iv) deletion of the *iclR* and *ankyrin-like regulator protein* (b4017) genes.

## Patentansprüche

1. Ein Verfahren zur Herstellung eines rekombinanten CRM197 in einem *E.-coli*-Wirt mit reduziertem Genom, das das Inkubieren eines *E. coli* mit reduziertem Genom umfasst, das einen Expressionsvektor umfasst, der eine Nukleotidsequenz umfasst, die für ein CRM197-Protein kodiert, das mit einer ompA-, ompF- oder ytfQ-Signalsequenz fusioniert ist, die den Transfer des CRM197-Proteins zum Periplasma lenkt, wobei die Nukleotidsequenz betriebsfähig mit einer Expressionskontrollsequenz verknüpft ist, unter für die Expression des rekombinanten CRM197-Proteins geeigneten Bedingungen, wobei der native *E.-coli*-Elternstamm ein K-12-Stamm ist, der eine native -2-Frameshift-Mutation umfasst, wobei die native -2-Frameshift-Mutation eine Deletion von Nukleotiden GT an Positionen 983 und 984 von *ilvG* relativ zu dem intakten *ilvG* umfasst, bevorzugt K12 MG1655, und wobei der *E.-coli*-Wirt mit reduziertem Genom die folgenden Modifikationen umfasst: (i) Deletion mindestens der folgenden DNA-Segmente: b0245-b0301, b0303-b0310, b1336-b1411, b4426-b4427, b2441-b2450, b2622-b2654, b2657-b2660, b4462, b1994-b2008, b4435, b3322-b3338, b2349-b2363, b1539-b1579, b4269-b4320, b2968-b2972, b2975-b2977, b2979-b2987, b4466-4468, b1137-b1172, b0537-b0565, b0016-b0022, b4412-b4413, b0577-b0582, b4415, b2389-b2390, b2392-b2395, b0358-b0368, b0370-b0380, b2856-b2863, b3042-b3048, b0656, b1325-b1333, b2030-b2062, b2190-b2192, b3215-b3219, b3504-b3505, b1070-b1083, b1878-b1894, b1917-b1950, b4324-b4342, b4345-b4358, b4486, b0497-b0502, b0700-b0706, b1456-b1462, b3481-b3484, b3592-b3596, b0981-b0988, b1021-b1029, b2080-b2096, b4438, b3440-b3445, b4451, b3556-b3558, b4455, b1786, b0150-b0153 und b2945 des *E*.-*coli*-K-12-Stamms MG1655, (ii) Deletion des *rph*-Gens, um die Aktivität von Orotatphosphoribosyltransferase zu verstärken, (iii) Korrektur der nativen -2-Frameshift-Mutation in dem *ilvG-*Gen, um die aktive Acetohydroxysäuresynthase-II-Produktion wiederherzustellen, wobei die Korrektur bevorzugt eine Insertion der zwei Nukleotide zwischen Positionen 982 und 983 ist, und (iv) Deletion der Gene *iclR* und *ankyrin-like regulator protein* (b4017); wodurch eine Ausbeute von mindestens 1 Gramm pro Liter, bevorzugt mindestens 2 Gramm pro Liter von löslichem CRM197 erhalten wird.

2. Verfahren nach Anspruch 1, wobei aus dem *E. coli* mit reduziertem Genom zusätzlich mindestens die folgenden DNA-Segmente deletiert wurden: b0315-b0331, b0333-b0341, b0346-b0354, b2481-b2492, b2219-b2230, b4500, b3707-b3723, b0644-b0650, b4079-4090, b4487, b4092-b4106, b0730-b0732, b3572-b3587, b1653, b2735-b2740, b2405-b2407, b3896-b3900, b1202, b4263-b4268, b0611, b2364-b2366, b0839, b0488-b0500 und b0502 des *E.-coli*-K-12-Stamms MG1655.

3. Verfahren nach Anspruch 1 oder 2, wobei dem *E*.-*coli*-Wirt mit reduziertem Genom eines oder mehrere von (i) einem funktionellen polB(b0060)-Gen, (ii) einem funktionellen dinB(b0231)-Gen fehlen und optional (iii) funktionelle umuD(b1183)- und umuC(b1184)-Gene fehlen.

4. Verfahren nach Anspruch 3, wobei dem *E.-coli-*Wirt mit reduziertem Genom eines oder mehrere von (i) einem funktionellen polB(b0060)-Gen, (ii) einem funktionellen dinB(b0231)-Gen und (iii) funktionellen umuD(b1183)- und umuC(b1184)-Genen fehlen.

5. Verfahren nach einem der Ansprüche 1-4, wobei die Signalsequenz YtfQ ist.

6. Verfahren nach Anspruch 1, wobei das *E. coli* mit reduziertem Genom ein funktionelles *recA*(b2699)-Gen umfasst.

7. Verfahren nach einem der vorhergehenden Ansprüche, wobei die CRM197-Nukleotidsequenz zur Expression in der *E.-coli*-Wirtszelle optimiert ist.

8. Verfahren nach einem der vorhergehenden Ansprüche, das Folgendes umfasst:
(a) Anzucht des *E. coli* mit reduziertem Genom und
(b) Induzieren der Expression von CRM197; wonach das Verfahren bevorzugt wie folgt ist:
(1) Schritt (a) umfasst die Anzucht des *E. coli* mit reduziertem Genom bei 37 °C für bis zu 19 Stunden, gefolgt von Wachstum bei etwa 20-30 °C, bevorzugt etwa 25 °C vor und nach Schritt (b);
(2) Schritte (a) und (b) werden in Wachstumsmedium durchgeführt, das kein Serum, keinen Hefeextrakt oder keine anderen Tiernebenprodukte umfasst;
(3) der pH-Wert liegt in Schritt (a) im Bereich zwischen 6,5 und 7,5;
(4) der pH-Wert wird unter Verwendung eines Phosphatpuffers, eines Tris-Puffers oder eines Histidinpuffers aufrechterhalten, bevorzugt unter Verwendung eines Phosphatpuffers;
(5) die Expression wird in Schritt (b) durch Zugeben einer geeigneten Menge von IPTG induziert; und/oder
(6) umfasst einen weiteren Schritt des (c) mechanischen Aufschließens der kultivierten *E.-coli*-Zellen mit reduziertem Genom in Abwesenheit von Detergens und des Zentrifugierens des resultierenden Zelllysats, um eine lösliche Fraktion zu erhalten, wobei der mechanische Aufschluss bevorzugt Beschallung oder Mikrofluidisation umfasst.

9. Verfahren nach Anspruch 8, wobei das Verfahren in einem Fermenter durchgeführt wird, wobei bevorzugt:
(1) die Expression bei einem OD₆₀₀ zwischen 100 bis 400, bevorzugt zwischen 200 bis 275, bevorzugter zwischen 230 und 250 induziert wird; und/oder
(2) der Fermenter 0,5-50 000 Liter Kultur enthält.

10. Verfahren nach Anspruch 8 oder 9, wobei das CRM197 durch einen oder mehrere Reinigungsschritte aus der löslichen Fraktion gereinigt wird, wobei der eine oder die mehreren Reinigungsschritte bevorzugt hydrophobe Interaktionschromatographie und/oder Anionenaustauschchromatographie umfassen und wobei die Reinigungsschritte bevorzugter hydrophobe Interaktionschromatographie, gefolgt von Anionenaustauschchromatographie, umfassen.

11. Ein *E.-coli-*Wirt mit reduziertem Genom, der einen Expressionsvektor umfasst, der eine Nukleotidsequenz umfasst, die für ein CRM197-Protein kodiert, das mit einer ompA-, ompF- oder ytfQ-Signalsequenz fusioniert ist, die den Transfer des CRM197-Proteins zum Periplasma lenkt, wobei die Nukleotidsequenz betriebsfähig mit einer Expressionskontrollsequenz verknüpft ist, wobei der native *E.-coli*-Elternstamm ein K-12-Stamm ist, der eine native -2-Frameshift-Mutation umfasst, wobei die native -2-Frameshift-Mutation eine Deletion von Nukleotiden GT an Positionen 983 und 984 von *ilvG* relativ zu dem intakten *ilvG* umfasst, bevorzugt K12 MG1655, und wobei der *E.-coli-*Wirt mit reduziertem Genom die folgenden Modifikationen umfasst: (i) Deletion mindestens der folgenden DNA-Segmente: b0245-b0301, b0303-b0310, b1336-b1411, b4426-b4427, b2441-b2450, b2622-b2654, b2657-b2660, b4462, b1994-b2008, b4435, b3322-b3338, b2349-b2363, b1539-b1579, b4269-b4320, b2968-b2972, b2975-b2977, b2979-b2987, b4466-4468, b1137-b1172, b0537-b0565, b0016-b0022, b4412-b4413, b0577-b0582, b4415, b2389-b2390, b2392-b2395, b0358-b0368, b0370-b0380, b2856-b2863, b3042-b3048, b0656, b1325-b1333, b2030-b2062, b2190-b2192, b3215-b3219, b3504-b3505, b1070-b1083, b1878-b1894, b1917-b1950, b4324-b4342, b4345-b4358, b4486, b0497-b0502, b0700-b0706, b1456-b1462, b3481-b3484, b3592-b3596, b0981-b0988, b1021-b1029, b2080-b2096, b4438, b3440-b3445, b4451, b3556-b3558, b4455, b1786, b0150-b0153 und b2945 des *E.-coli*-K-12-Stamms MG1655, (ii) Deletion des *rph*-Gens, um die Aktivität von Orotatphosphoribosyltransferase zu verstärken, (iii) Korrektur der nativen -2-Frameshift-Mutation in dem *ilvG-*Gen, um die aktive Acetohydroxysäuresynthase-II-Produktion wiederherzustellen, wobei die Korrektur bevorzugt eine Insertion der zwei Nukleotide zwischen Positionen 982 und 983 ist, und (iv) Deletion der Gene *iclR* und *ankyrin-like regulator protein* (b4017).

## Revendications

1. Procédé de production d'une CRM197 recombinante dans un hôte *E. coli* à génome réduit, comprenant l'incubation d'un *E. coli* à génome réduit comprenant un vecteur d'expression comprenant une séquence nucléotidique codant une protéine CRM197 fusionnée à une séquence signal ompA, ompF ou ytfQ qui dirige un transfert de la protéine CRM197 au périplasme, dans lequel ladite séquence nucléotidique est fonctionnellement liée à une séquence de contrôle d'expression, dans des conditions propices à l'expression de la protéine CRM197 recombinante, dans lequel la souche parentale native *d'E. coli* est une souche K-12 comprenant une mutation native avec décalage -2 du cadre de lecture, dans lequel la mutation native avec décalage -2 du cadre de lecture comprend une délétion des nucléotides GT aux positions 983 et 984 du gène *ilvG* par rapport au gène *ilvG* intact, de préférence K12 MG1655, et dans lequel l'hôte *E. coli* à génome réduit comprend les modifications suivantes : (i) délétion d'au moins les segments d'ADN suivants : b0245-b0301, b0303-b0310, b1336-b1411, b4426-b4427, b2441-b2450, b2622-b2654, b2657-b2660, b4462, b1994-b2008, b4435, b3322-b3338, b2349-b2363, b1539-b1579, b4269-b4320, b2968-b2972, b2975-b2977, b2979-b2987, b4466-4468, b1137-b1172, b0537-b0565, b0016-b0022, b4412-b4413, b0577-b0582, b4415, b2389-b2390, b2392-b2395, b0358-b0368, b0370-b0380, b2856-b2863, b3042-b3048, b0656, b1325-b1333, b2030-b2062, b2190-b2192, b3215-b3219, b3504-b3505, b1070-b1083, b1878-b1894, b1917-b1950, b4324-b4342, b4345-b4358, b4486, b0497-b0502, b0700-b0706, b1456-b1462, b3481-b3484, b3592-b3596, b0981-b0988, b1021-b1029, b2080-b2096, b4438, b3440-b3445, b4451, b3556-b3558, b4455, b1786, b0150-b0153 et b2945 de la souche K-12 MG1655 d'*E*. *coli,* (ii) délétion du gène *rph* pour augmenter l'activité de l'orotate phosphoribosyltransférase, (iii) correction de la mutation native avec décalage -2 du cadre de lecture dans le gène *ilvG* afin de restaurer la production d'acétohydroxyacide synthase II active, de préférence dans lequel ladite correction est une insertion de deux nucléotides entre les positions 982 et 983, et (iv) délétion des gènes *iclR* et *ankyrin-like regulator protein* (b4017) ; en obtenant ainsi un rendement d'au moins 1 gramme par litre, de préférence d'au moins 2 grammes par litre de CRM197 soluble.

2. Procédé selon la revendication 1, dans lequel l'E. coli à génome réduit a en outre subi une délétion d'au moins les segments d'ADN suivants : b0315-b0331, b0333-b0341, b0346-b0354, b2481-b2492, b2219-b2230, b4500, b3707-b3723, b0644-b0650, b4079-4090, b4487, b4092-b4106, b0730-b0732, b3572-b3587, b1653, b2735-b2740, b2405-b2407, b3896-b3900, b1202, b4263-b4268, b0611, b2364-b2366, b0839, b0488-b0500 et b0502 de la souche K-12 MG1655 d'*E*. *coli.*

3. Procédé selon la revendication 1 ou 2, dans lequel l'hôte *E*. *coli* à génome réduit est dépourvu d'un ou plusieurs de (i) un gène fonctionnel polB (b0060), (ii) un gène fonctionnel dinB (b0231), et est optionnellement dépourvu (iii) des gènes fonctionnels umuD (b1183) et umuC (b1184).

4. Procédé selon la revendication 3, dans lequel l'hôte *E. coli* à génome réduit est dépourvu d'un ou plusieurs de (i) un gène fonctionnel polB (b0060), (ii) un gène fonctionnel dinB (b0231), et (iii) des gènes fonctionnels umuD (b1183) et umuC (b1184).

5. Procédé selon l'une quelconque des revendications 1 à 4, dans lequel la séquence signal est YtfQ.

6. Procédé selon la revendication 1, dans lequel l*'E. coli* à génome réduit comprend un gène fonctionnel *recA* (b2699) .

7. Procédé selon l'une quelconque des revendications précédentes, dans lequel la séquence nucléotidique de CRM197 est optimisée pour une expression dans la cellule hôte *d'E. coli.*

8. Procédé selon l'une quelconque des revendications précédentes, comprenant
(a) une culture de l*'E. coli* à génome réduit ;
(b) une induction de l'expression de CRM197 ; le procédé étant de préférence **caractérisé en ce que** :
(1) l'étape (a) comprend une culture de l*'E. coli* à génome réduit à 37 °C pendant une période de jusqu'à 19 heures suivie d'une culture à environ 20 à 30 °C, de préférence d'environ 25 °C avant et après l'étape (b) ;
(2) les étapes (a) et (b) sont réalisées dans un milieu de culture qui ne contient pas de sérum, d'extrait de levure ou d'autres sous-produits animaux ;
(3) le pH se situe entre 6,5 et 7,5 à l'étape (a) ;
(4) le pH est maintenu à l'aide d'un tampon phosphate, d'un tampon Tris ou d'un tampon histidine, de préférence à l'aide d'un tampon phosphate ;
(5) l'expression est induite à l'étape (b) par l'ajout d'une quantité adéquate d'IPTG ; et/ou
(6) il comprend une étape supplémentaire (c) consistant à perturber mécaniquement les cellules d'*E*. *coli* à génome réduit en culture en l'absence de détergent et à centrifuger le lysat cellulaire ainsi produit pour obtenir une fraction soluble, la perturbation mécanique comprenant de préférence une sonication ou une microfluidisation.

9. Procédé selon la revendication 8, le procédé étant mis en œuvre dans un fermenteur, de préférence dans lequel :
(1) l'expression est induite à une OD₆ₒₒ comprise entre 100 et 400, de préférence entre 200 et 275, plus préférablement entre 230 et 250 ; et/ou
(2) le fermenteur contient 0,5 à 50 000 litres de culture.

10. Procédé selon la revendication 8 ou 9, dans lequel la CRM197 est purifiée à partir de la fraction soluble par une ou plusieurs étapes de purification, de préférence dans lequel une ou plusieurs étapes de purification comprennent une chromatographie à interaction hydrophobe et/ou une chromatographie par échange d'anions, et encore plus préférablement dans lequel les étapes de purification comprennent une chromatographie à interaction hydrophobe suivie d'une chromatographie par échange d'anions.

11. Hôte *E. coli* à génome réduit comprenant un vecteur d'expression comprenant une séquence nucléotidique codant une protéine CRM197 fusionnée à une séquence signal ompA, ompF ou ytfQ qui dirige un transfert de la protéine CRM197 au périplasme, dans lequel ladite séquence nucléotidique est fonctionnellement liée à une séquence de contrôle d'expression, dans lequel la souche parentale native *d'E. coli* est une souche K-12, comprenant une mutation native avec décalage -2 du cadre de lecture, dans lequel la mutation native avec décalage -2 du cadre de lecture comprend une délétion des nucléotides GT aux positions 983 et 984 du gène *ilvG* par rapport au gène *ilvG* intact, de préférence K12 MG1655, et dans lequel l'hôte *E*. *coli* à génome réduit comprend les modifications suivantes : (i) délétion d'au moins les segments d'ADN suivants : b0245-b0301, b0303-b0310, b1336-b1411, b4426-b4427, b2441-b2450, b2622-b2654, b2657-b2660, b4462, b1994-b2008, b4435, b3322-b3338, b2349-b2363, b1539-b1579, b4269-b4320, b2968-b2972, b2975-b2977, b2979-b2987, b4466-4468, b1137-b1172, b0537-b0565, b0016-b0022, b4412-b4413, b0577-b0582, b4415, b2389-b2390, b2392-b2395, b0358-b0368, b0370-b0380, b2856-b2863, b3042-b3048, b0656, b1325-b1333, b2030-b2062, b2190-b2192, b3215-b3219, b3504-b3505, b1070-b1083, b1878-b1894, b1917-b1950, b4324-b4342, b4345-b4358, b4486, b0497-b0502, b0700-b0706, b1456-b1462, b3481-b3484, b3592-b3596, b0981-b0988, b1021-b1029, b2080-b2096, b4438, b3440-b3445, b4451, b3556-b3558, b4455, b1786, b0150-b0153 et b2945 de la souche K-12 MG1655 d'*E*. *coli,* (ii) délétion du gène *rph* pour augmenter l'activité de l'orotate phosphoribosyltransférase, (iii) correction de la mutation native avec décalage -2 du cadre de lecture dans le gène *ilvG* afin de restaurer la production d'acétohydroxyacide synthase II active, de préférence dans lequel ladite correction est une insertion de deux nucléotides entre les positions 982 et 983, et (iv) délétion des gènes *iclR* et *ankyrin-like regulator protein (protéine régulatrice ankyrin-like)* (b4017).
